(19) **Europäisches Patentamt / European Patent Office / Office européen des brevets**

(11) **EP 4 772 603 A2**

(12) # EUROPEAN PATENT APPLICATION

(43) Date of publication:
08.07.2026 Bulletin 2026/28

(21) Application number: 26174225.8

(22) Date of filing: 05.08.2021

(51) International Patent Classification (IPC):
**C11D 3/50** (2006.01)

(52) Cooperative Patent Classification (CPC):
**B01J 13/14; A01N 25/28; A23P 10/30; A23P 10/35; A61K 8/11; A61K 8/64; A61K 8/731; A61Q 13/00; A61Q 19/00; C11D 3/505;** A61K 9/5057

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 06.08.2020 PCT/EP2020/072202

(62) Document number(s) of the earlier application(s) in accordance with Art. 76 EPC:
**21762786.8 / 4 192 614**

(71) Applicant: **Symrise AG**
37603 Holzminden (DE)

(72) Inventors:
• **TECCHIO, Katherine**
Holzminden, 37603 (US)
• **RAABE, Britta**
37603 Holzminden (DE)
• **ROST, Benjamin**
37603 Holzminden (DE)

• **KOEPKE, Christina**
37603 Holzminden (DE)
• **GEORGI, Julian Alexander**
37603 Holzminden (DE)
• **BERTRAM, Ralf**
37603 Holzminden (DE)
• **GREGOR, Daniela**
37603 Holzminden (DE)
• **VOGEL, Andreas**
37603 Holzminden (DE)

(74) Representative: **Global IP Europe Patentanwaltskanzlei**
**Pfarrstraße 14**
**80538 München (DE)**

Remarks:
This application was filed on 23.04.2026 as a divisional application to the application mentioned under INID code 62.

(54) ## PROCESS FOR THE PREPARATION OF MICROCAPSULES

(57) The present invention relates to a process for the preparation of biodegradable microcapsules, in particular biodegradable protein- and/or polysaccharide-based microcapsules, as well as dispersions of such microcapsules (microcapsule slurry) that enclose at least one hydrophobic active ingredient. In addition, the present invention relates to biodegradable microcapsules obtainable by the process according to the invention. In another aspect, the present invention relates to the use of the microcapsules and dispersions as an ingredient in consumer products.

Figure 1 a:

**EP 4 772 603 A2**

## Description

## Field of the invention

The present invention relates to a process for the preparation of biodegradable microcapsules, in particular biodegradable protein- and/or polysaccharide-based microcapsules as well as dispersions of such microcapsules (microcapsule slurry) that enclose at least one hydrophobic active ingredient, preferably for perfume- or aroma-containing polysaccharide- and protein-based microcapsules, which have a well-balanced biodegradability, stability and performance compared to microcapsules according to the state of the art. In addition, the present invention relates to biodegradable microcapsules comprising at least one hydrophobic active agent obtainable by the process according to the invention. In another aspect, the present invention relates to the use of the microcapsules and dispersions as an ingredient in household products, textile care products, laundry detergents, fabric softeners, cleaning agents, scent boosters or fragrance enhancers in liquid or solid form, cosmetics, personal care products, perfume compositions, agricultural products, pharmaceutical products or print coating for paper. Finally, the present invention relates to consumer products comprising such microcapsules or microcapsule dispersions.

Microcapsules are particles comprising a core and a wall material surrounding the core, wherein the core may be a solid, liquid or gaseous substance surrounded by a polymeric dense, permeable or semi-permeable wall material. During manufacture, the polymers of the starting ingredients precipitate onto the substances to be encapsulated after emulsification and coacervation or interfacial polymerization. The core is also referred to as the inner phase. Other names used for the wall include outer phase, shell or coating. The diameter of the microcapsules typically varies in the range of 1 to 1 000 $\mu$m. The wall thickness is typically 0.5 to 150 $\mu$m but can be varied in the range of $5 \cdot 10^{-9}$ m to $5 \cdot 10^{-6}$ m. Typically loadings of 25 to 95 wt.-%, but also those of 1 to 99 wt.-% are possible.

Encapsulation of an active ingredient with a suitable wall material (coating material) can be done for several reasons:

- Conversion of liquids into a manageable powder form (e.g., coating of vegetable oils, fats);
- Time-controlled release of substances (dosage control, depot effect for pharmaceuticals, pesticides and fertilizers);
- Taste, odor and color lamination (e.g., bitter or pungent aroma substances);
- Protection against light, oxidation, heat, acids or bases (e.g., vitamins, aroma substances, etc.)
- Moisture protection (e.g., hygroscopic salts or minerals);
- Retardation of losses of volatile components (e.g., aroma substances);
- Prevention of premature chemical reactions with other mixture components;
- Better handling before or during processing (flow properties, dust formation);
- Protection of personnel from harmful or unpleasant materials (chemicals, aroma concentrates); or improved solubility/suspendability due to surface modification.

Hydrophobic active ingredients, such as fragrances or aroma substances, can be easily incorporated into numerous and diverse application formulations through encapsulation.

The contents of microcapsules can be released in a variety of ways and, in particular, is based on one of the mechanisms described below:

- The capsules are mechanically disrupted by crushing or shearing. This mechanism is used, for example, in reaction carbonless paper.
- The capsules are destroyed by melting the wall material. Based on this mechanism, ingredients such as raising agents or flavors are released, for example, in baking mixtures only during the baking process.
- The capsules are destroyed by dissolving the wall material. This mechanism is used, for example, in washing powder so that encapsulated ingredients such as enzymes are only released during the washing process.
- The capsules remain intact, and the capsule contents are gradually released by diffusion through the capsule wall. Based on this mechanism, e.g., a slow and uniform release of drug ingredients in the body can be achieved.

Based on their properties, microcapsules are used in the printing industry, food industry (vitamins, flavors, plant extracts, enzymes, microorganisms), agricultural chemistry (fertilizers, pesticides), feed industry (minerals, vitamins, enzymes, drugs, microorganisms), pharmaceutical industry, detergent industry, and cosmetic industry, among others.

Many articles of daily use, such as cleaning agents, fabric softeners, washing powders, liquid detergents, shower gels, shampoos, deodorants, body lotions, etc., are nowadays perfumed with fragrant substances or mixtures of fragrant substances. Very often, the fragrant substances interact with other components of the formulation, or the more volatile components of a perfumed product evaporate prematurely. This usually results in the fragrance impression of the perfumery changing over time or even disappearing completely.

Microencapsulation of such fragrance mixtures offers the possibility of reducing or completely preventing interactions in the perfumed product or the evaporation of the highly volatile fragrance components.

A variety of capsule wall or coating materials are known for the manufacture of microcapsules. The capsule wall can be made of either natural, semi-synthetic or synthetic materials. Natural shell materials include gum arabic, agar-agar, agarose, maltodextrins, alginic acid or its salts, e.g., sodium alginate or calcium alginate, fats and fatty acids, cetyl alcohol, collagen, chitosan, lecithins, gelatin, albumin, shellac, polysaccharides such as starch or dextran, polypeptides, protein hydrolysates, sucrose, and waxes. Semisynthetic capsule wall materials include chemically modified celluloses, especially cellulose esters, and cellulose ethers, e.g., cellulose acetate, ethyl cellulose, hydroxypropyl cellulose, hydroxypropyl methyl cellulose, and carboxymethyl cellulose, as well as starch derivatives, in particular starch ethers and starch esters. Synthetic shell materials are, for example, polymers such as polyacrylates, polyamides, polyvinyl alcohol or polyvinylpyrrolidone.

Depending on the type of capsule wall material and manufacturing process, microcapsules with different properties in terms of diameter, size distribution, and physical and/or chemical properties are formed in each case.

Polyurea microcapsules or polyurea/polyurethane microcapsules formed by polymerization between a polyisocyanate and a polyamine and/or a diol or polyol are well-known capsules used in a variety of technical fields, including perfumery.

Polyurea microcapsules obtained by reacting two polyisocyanates and a polyamine are described, for example, in WO 2011/161229 or WO 2011/160733. According to WO 2011/161229 or WO 2011/160733, the polyurea microcapsules are prepared in the presence of polyvinylpyrrolidone (PVP) as a protective colloid. WO 2012/107323 discloses polyurea microcapsules having a polyurea shell comprising the reaction product of a polyisocyanate with guanazole (3,5-diamino-1,2,4-triazole) and an amino acid in the presence of anionic stabilizers or surfactants such as anionic polyvinyl alcohol. EP 0 537 467 B describes microcapsules prepared from polyisocyanates containing polyethylene oxide groups in the presence of stabilizers such as polyvinyl alcohol. According to WO 2007/096592, microencapsulation can be carried out in an oil phase which is emulsified in a continuous aqueous phase that is in general stabilized by a surfactant system such as polyvinyl alcohols or carboxylated and sulfonated derivatives thereof.

The exemplary delivery systems of the state of the art described above exhibit both good stability, namely the ability to retain the active ingredient and thus the ability of the capsules to avoid loss of the volatile components, and good performance, for example fragrance release in the case of fragrance or odorant capsules.

However, the microcapsules according to the state of the art described above have the disadvantage that the polymeric capsule wall or capsule shell material requires a large polymer content to ensure sufficient stability and to avoid too much loss of active ingredients. In addition, the microencapsulation process introduces plastic into the environment, where it can cause problems as "microplastics," possibly causing environmental damage or adverse health effects.

Since plastic particles are increasingly under public criticism for their environmental impact, and the demand for biobased and biodegradable solutions is growing due to ever-increasing societal pressure with regard to environmental considerations, there is a need to develop new materials for microencapsulation to achieve a reduction of microplastics in the environment. Here, biobased and biodegradable materials are in focus.

In light of this, there is therefore a need for the provision of microcapsules that increasingly use biodegradable capsule wall materials in their preparation, while at the same time exhibiting outstanding stability and release properties for the applications in question. It is important that not only the macromolecular material of the capsule wall itself is biocompatible, but also each of the fragments formed during disintegration.

However, this task of using biodegradable materials to reduce the amount of microplastics in the environment is not trivial in the case of microencapsulation, as the desired functionality of the microcapsule, such as olfactory properties and positive secondary properties such as high stability, toxicological stability conflict with the requirements for rapid biodegradability in many applications.

It is particularly difficult to produce microcapsules that have both good stability and good release of the active ingredient. The ability to retain active ingredients, and thus the ability of the capsules to avoid losses of volatile components, depends in particular on the stability of the capsules in the product base. However, capsules with good stability in particular do not automatically exhibit good biodegradability.

As the degree of crosslinking increases, the stability of the microcapsules increases, but at the same time the ability to biodegrade the capsule shell decreases. In the case of very stable microcapsules, the performance, for example sensory performance, is lower because the number of microcapsules, which break open and release active ingredients due to pressure, friction, etc., decreases. If they are too unstable, they are already destroyed during storage and also do not perform.

The present invention is therefore based on the complex task of providing microcapsules that preferably meet one, several or preferably all of the following requirements:

- Improved biodegradability,
- No toxic effect towards humans and the environment;

- Maintenance of sufficient stability,
- Suitability for a wide variability with respect to the active ingredients to be encapsulated,
- Excellent release behavior of the encapsulated active ingredients, and
- Accessible by known microcapsule manufacturing processes, and
- Ingredients are readily available, i.e., can be produced from biobased or sustainably produced raw materials.

Surprisingly, it was found that this task can be solved by preparing the microcapsule from a polysaccharide and/or a protein and a crosslinking agent in an aqueous emulsion in the presence of a catalyst by interfacial polymerization. Crosslinking makes it possible to form a biodegradable and stable capsule shell or capsule wall that can be used to encapsulate a wide range of hydrophobic or lipophilic active ingredients, respectively.

**Summary of the Invention**

The present problem is solved by the objects of the independent patent claims. Preferred embodiments are apparent from the wording of the dependent patent claims as well as the following description.

Thus, a first object of the invention relates to a process for the preparation of a biodegradable protein- and/or polysaccharide-based microcapsule comprising the following steps in this order:

(i) Providing an internal non-aqueous phase comprising at least one first crosslinking agent and at least one hydrophobic active ingredient, and optionally at least one further crosslinking agent, and optionally at least one catalyst;

(ii) Providing an external aqueous phase comprising at least one protein and/or at least one polysaccharide and optionally at least one protective colloid, and optionally adjusting the pH-value of the aqueous phase to a pH-value below the isoelectric point of the protein;

(iii) Emulsifying or dispersing the internal non-aqueous phase in the external aqueous phase, optionally in the presence of at least one stabilizer and/or at least one emulsifier, to obtain an oil-in-water emulsion/dispersion;

(iv) Optionally adding at least one further polysaccharide and/or at least one further protein;

(v) First crosslinking by addition of at least one catalyst to obtain a microcapsule slurry;

(vi) Curing the microcapsule slurry at a temperature of at least 60 °C and optionally adding at least one further polysaccharide and/or at least one further protein;

(vii) Cooling and optionally second crosslinking by adding at least one second crosslinking agent; and

(viii) Optionally separating the microcapsules from the microcapsule slurry and optionally drying the microcapsules or adjusting the viscosity of the microcapsule slurry by adding at least one thickening agent.

In a second aspect, the present invention relates to a microcapsule comprising at least one lipophilic active ingredient or a microcapsule slurry prepared according to the process of the invention.

It is also an object of the present invention to provide a biodegradable microcapsule comprising or consisting of

(a) a core comprising or consisting of at least one hydrophobic active agent;

(b) a capsule shell comprising or consisting of a crosslinking matrix or units of at least one polysaccharide and/or at least one protein and at least one first crosslinking agent; and optionally at least one first protective colloid and/or optionally at least one further crosslinking agent.

Finally, in another aspect, the present invention relates to the use of the microcapsules according to the invention or of dispersions comprising the microcapsules according to the invention for the preparation of household products, textile care products, laundry detergents, fabric softeners, cleaning agents, scent boosters, scent lotions or scent enhancers in liquid or solid form, cosmetics, personal care products, perfume compositions, agricultural products, pharmaceutical products or print coating for paper.

Surprisingly, it was found in the context of the present invention that in the preparation of the microcapsules, a combination of polysaccharide and/or protein and subsequent crosslinking with a polyisocyanate having at least two or more isocyanate groups results in stable microcapsules, thus ensuring an efficient encapsulation of lipophilic active ingredients with subsequent targeted release of these active ingredients, while at the same time the microcapsules exhibit good biodegradability due to their biobased and biodegradable building blocks.

By using polysaccharide and/or protein, the polyisocyanate content of the capsule wall or capsule shell material can be reduced, i.e., replaced by biobased capsule wall components, and thus the proportion of biobased capsule wall components can be increased without sacrificing the stability of the microcapsule wall.

These and other aspects, features and advantages of the present invention will be apparent to those skilled in the art from a study of the following detailed description and claims. In this regard, any feature or variation from one aspect of the invention may be used or substituted in another aspect of the invention. Further, it is understood that the examples disclosed herein describe and illustrate the invention but are not intended to limit the invention and, in particular, that the present invention is not limited to these examples.

All percentages are by weight unless otherwise indicated. Numerical examples given in the form "from x to y" include said values. When multiple preferred numerical ranges are given in this format, it is understood that all ranges resulting from the combination of the various endpoints are also included.

The terms "at least one" or "one or more" as used herein refers to 1 or more, for example 2, 3, 4, 5, 6, 7, 8, 9 or more.

The term "and/or" expresses that a linkage exists, or an alternative is provided.

Numeric examples given in the form "x to y" include the values given. When multiple preferred numeric ranges are specified in this format, all ranges created by combining the various endpoints are also included.

**Figures**

Figures 1 a to 1 d show diagrams of the particle size distribution (d(0,5)-value) of microcapsules according to the invention with different compositions. Figure 1 e is a diagram showing the comparison of the particle size distribution of a microcapsule according to the state of the art and a microcapsule according to the invention. A MALVERN Mastersizer 3000 was used for the determination of the particle size distribution. The corresponding calculation is based on the Mie theory.

Figure 2 is a diagram showing the free oil content of microcapsules according to the present invention compared to microcapsules according to the state of the art.

Figure 3 is a diagram showing the free oil content of microcapsules according to the present invention compared to microcapsules according to the state of the art.

Figure 4 is a diagram showing the free oil content of microcapsules according to the present invention prepared without and with further crosslinking agent.

Figure 5 is a diagram showing the stability of microcapsules according to the invention in a fabric softener.

Figure 6 is a diagram showing the sensory evaluation of microcapsules according to the invention. In the y-axis numbers a comma is used for the decimal place.

Figure 7 is a diagram showing in general terms the correlation between microcapsule stability, performance and biodegradability as a function of the degree of crosslinking.

Figure 8 is a diagram showing the particle size distribution (d(0,5)-value) of microcapsules according to the invention based on chickpea protein.

Figure 9 is a diagram showing the free oil content of microcapsules according to the present invention based on chickpea protein.

Figures 10 a and 10 b are diagrams showing the sensory evaluation of microcapsules according to the invention based on chickpea protein and dextrin from pea starch in comparison to a pure fragrance oil reference in a fabric softener formulation, each aged for one week in the softener, after machine drying and after line drying, respectively.

Figures 11a to 11d are diagrams showing the sensory evaluation of microcapsules according to the invention based on chickpea protein and dextrin from pea starch in comparison to a pure fragrance oil reference in a fabric softener formulation, each aged for two or four weeks in the softener, after machine drying and after line drying, respectively.

Figures 12 a and 12 b are diagrams showing the sensory evaluation of freshly prepared and one year old microcapsules according to the invention based on chickpea protein and dextrin from pea starch which were prepared using a dual catalyst system in comparison to the corresponding microcapsules which were prepared using a single DABCO catalyst or Bi-catalyst, respectively.

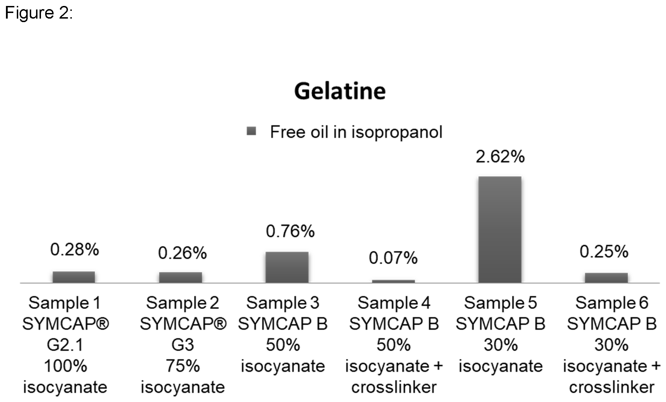

In Figures 2 to 6, the dot was used as a decimal separator.

**Detailed description of the invention**

In a first aspect, the present invention relates to a process for preparing a biodegradable protein- and/or polysaccharide-based microcapsule comprising the following steps in this order:

(i) Providing an internal non-aqueous phase comprising at least one first crosslinking agent and at least one hydrophobic active ingredient and optionally at least one further crosslinking agent, and optionally at least one catalyst;
(ii) Providing an external aqueous phase comprising at least one protein and/or at least one polysaccharide and optionally at least one protective colloid, and optionally adjusting the pH-value of the aqueous phase to a pH-value below the isoelectric point of the protein;
(iii) Emulsifying or dispersing the internal non-aqueous phase in the external aqueous phase, optionally in the presence of at least one stabilizer and/or at least one emulsifier, to obtain an oil-in-water emulsion/dispersion;
(iv) Optionally adding at least one further polysaccharide and/or at least one further protein;
(v) First crosslinking by addition of at least one catalyst to obtain a microcapsule slurry;
(vi) Curing the microcapsule slurry at a temperature of at least 60 °C and optionally adding at least one further polysaccharide and/or at least one further protein;
(vii) Cooling and optionally second crosslinking by adding at least one second crosslinking agent; and
(viii) Optionally separating the microcapsules from the microcapsule slurry and optionally drying the microcapsules or adjusting the viscosity of the microcapsule slurry by adding at least one thickening agent.

In another variant the process for preparing a biodegradable protein- and/or polysaccharide-based microcapsule comprising the following steps in this order:

(i) Providing an internal non-aqueous phase comprising at least one first crosslinking agent and at least one hydrophobic active ingredient and optionally at least one further crosslinking agent, and optionally at least one catalyst;
(ii) Providing an external aqueous phase comprising at least one protective colloid, and optionally at least one protein and/or at least one polysaccharide and optionally adjusting the pH-value of the aqueous phase to a pH-value below the isoelectric point of the protein;
(iii) Emulsifying or dispersing the internal non-aqueous phase in the external aqueous phase, optionally in the presence of at least one stabilizer and/or at least one emulsifier, to obtain an oil-in-water emulsion/dispersion;
(iv) Optionally adding at least one polysaccharide or at least one further polysaccharide and/or at least one protein or at least one further protein;
(v) First crosslinking by addition of at least one catalyst to obtain a microcapsule slurry;
(vi) Curing the microcapsule slurry at a temperature of at least 60 °C and optionally adding at least one further polysaccharide and/or at least one further protein;
(vii) Cooling and optionally second crosslinking by adding at least one second crosslinking agent; and
(viii) Optionally separating the microcapsules from the microcapsule slurry and optionally drying the microcapsules or adjusting the viscosity of the microcapsule slurry by adding at least one thickening agent.

In the context of the present invention, microcapsules are understood to be microparticles comprising at least one or more active ingredient(s) as core material inside the capsule and which are enclosed by a capsule shell or capsule wall. The active ingredients are preferably hydrophobic or lipophilic active ingredients. Such active ingredients are insoluble or poorly soluble in water, but readily soluble in fats and oils. The terms "microcapsule" and "capsule" or "hydrophobic" and "lipophilic" are used synonymously within the context of the present invention.
In the context of the present invention, the capsule shell or capsule wall is preferably composed of several crosslinking matrices or crosslinking units, which preferably have different compositions and are generated by several process steps or process sequences, in particular crosslinking steps, during the preparation of the microcapsule according to the invention. The crosslinking matrix comprises or consists of at least one polysaccharide and/or at least one protein. These capsule wall components are crosslinked together by means of a crosslinking agent and a catalyst by interfacial polymerization via selectively catalyzed mechanisms to form a three-dimensional network of polysaccharide, protein and crosslinking agent.
In a first step (i) of the process according to the invention, an internal non-aqueous phase is provided comprising at least one crosslinking agent and at least one hydrophobic active agent to be encapsulated and optionally a further crosslinking agent.

Moreover, optionally in this step (i) already a catalyst is added, in particular in case of dual catalyst systems. Preferably said catalyst is oil soluble. Therefore, the internal non-aqueous phase can additionally comprise a catalyst.

The first crosslinking agent according to the first and/or second aspect of the present invention for preparing the capsule shell or capsule wall is a crosslinking agent selected from the group consisting of polyisocyanate having two or more isocyanate groups, selected from the group consisting of aliphatic, cycloaliphatic, hydroaromatic, aromatic or heterocyclic polyisocyanates, their substitution products, and mixtures of two or more of the aforementioned first crosslinking agents.

The at least one isocyanate or polyisocyanate having two or more isocyanate groups, which is used in the process according to the invention for preparing the biodegradable protein- and/or polysaccharide-based microcapsule, comprises at least two isocyanate groups for forming polymeric networks by polymerization, which form a capsule shell or capsule wall.

Polyisocyanates are R-substituted organic derivatives (R-N=C=O) of isocyanic acid (HN=C=O). Organic isocyanates are compounds in which the isocyanate group (-N=C=O) is bonded to an organic radical. Polyfunctional isocyanates or polyisocyanates are those compounds which contain at least two or more, i.e., 3, 4, 5, 6, 7, 8, 9, 10, 11, 12, 13, 14, 15, 20, 50, 100, 200 or even more, isocyanate groups (-N=C=O) in the molecule. Polyisocyanates with two isocyanate groups are also referred to as diisocyanates.

Polyisocyanates can be classified as aliphatic, cycloaliphatic, hydroaromatic, aromatic or heterocyclic isocyanates or polyisocyanates. In addition, the polyisocyanates according to the invention can be linear or branched.

Polyisocyanates, in particular aromatic polyisocyanates, are highly reactive compounds. The polyaddition reactions of polyisocyanates with diols or polyols form the basis of polyurethane chemistry and the polyaddition reactions of polyisocyanates with amines form the basis of polyurea chemistry.

According to the invention, at least difunctional, preferably polyfunctional polyisocyanates are used, i.e., all aliphatic, alicyclic and aromatic isocyanates are suitable, provided they have at least two reactive isocyanate groups.

Aliphatic, cycloaliphatic, hydroaromatic, aromatic or heterocyclic polyisocyanates, their substitution products and mixtures of the aforementioned monomeric or oligomeric compounds are particularly preferred. Of the previously specified polyisocyanates, aliphatic and/or aromatic compounds are preferably used.

In preferred embodiments of the process according to the invention, the polyisocyanate contains on average 2 to 5 functional -N=C=O groups. These include, for example, aliphatic, cycloaliphatic and aromatic di-, tri- and higher polyisocyanates.

Among the abovementioned polyisocyanates, diisocyanates and polyisocyanates having three functional -N=C=O groups are particularly preferred and therefore are used primarily in the implementation of the present invention. Preferably, diisocyanates with the general structure O=C=N-R-N=C=O, wherein R represents aliphatic, alicyclic or aromatic radicals, are used. Preferably, the radicals have five or more carbon atoms.

In a preferred embodiment of the process according to the invention, the at least one polyisocyanate having two or more isocyanate groups is selected from the group consisting of aliphatic polyisocyanates and/or aromatic polyisocyanates. In an even more preferred variant of the process according to the invention, the at least one polyisocyanate is a combination of two different aliphatic polyisocyanates or a combination of an aliphatic and aromatic polyisocyanate.

Due to the number of functional groups, an optimal crosslinking or a network of the capsule wall is achieved, providing microcapsules that exhibit prolonged slow release of active ingredients as well as good stability in the consumer product.

In a preferred variant of the process according to the present invention, the polyisocyanate is an aliphatic polyisocyanate.

The term "aliphatic polyisocyanate" refers to any polyisocyanate molecule that is not aromatic. In addition, the molecule comprises at least two isocyanate groups, i.e., at least 2, 3, 4, 5, 6, 7, 8, 9, 10, 11, 12, 13, 14, 15, 20, 50, 100, 200 or more isocyanate groups directly attached to a corresponding number of different C atoms of the same aliphatic molecule, and derivatives of such compounds.

The aliphatic polyisocyanate molecule containing at least two isocyanate groups, i.e., at least 2, 3, 4, 5, 6, 7, 8, 9, 10, 11, 12, 13, 14, 15, 20, 50, 100, 200 or more isocyanate groups, may further be linear, branched or cyclic and may have any substitutions including, for example, aliphatic substituents, aromatic substituents, one or more heteroatoms such as nitrogen, oxygen, phosphorus and/or sulfur, halogens such as fluorine, chlorine, bromine and/or iodine and/or other functional groups such as alkoxy groups.

The linear aliphatic polyisocyanate molecule is preferably selected from C2- to C20-linear alkyl, preferably C3- to C15-linear alkyl, C4- to C12-linear alkyl, C5- to C10-linear alkyl, C6- to C9-linear alkyl, or C7- to C8-linear alkyl. Preferably, the linear aliphatic molecule does not comprise an aromatic structure.

The branched aliphatic polyisocyanate molecule is preferably selected from C2- to C20-branched alkyl, preferably C3- to C15-branched alkyl, C4- to C12-branched alkyl, C5- to C10-branched alkyl, C6- to C9-branched alkyl, C7- to C8-branched alkyl.

The shorter the carbon chain of the polyisocyanate molecule, the higher the reaction rate compared to longer chained analogs.

The cyclic aliphatic polyisocyanate molecule comprises at least 1, i.e., 1, 2, 3, 4 or more non-aromatic ring structures, wherein the ring structure itself preferably consists only of C-atoms. Of course, the C-atoms of the ring structure may bear suitable substituents. The at least 1-ring structures preferably consist independently of 3-, 4-, 5-, 6-, 7- or 8-membered rings. Preferably, the cyclic aliphatic molecule comprises 2 to 20 C-atoms, such as 3 to 15 C-atoms, 4 to 12 C-atoms, 5 to 10 C-atoms, 6 to 9 C-atoms or 7 to 8 C-atoms.

In another variant of the process according to the invention, the polyisocyanate is an aromatic polyisocyanate. The term "aromatic polyisocyanate" refers to any polyisocyanate compound in which two or more isocyanate groups are directly attached to aromatic C-atoms and comprises, for example, a phenyl, tolyl, xylyl, naphthyl or diphenyl moiety as an aromatic component, as well as derivatives of such polyisocyanate compounds.

Aromatic polyisocyanates react significantly faster than aliphatic polyisocyanates and are therefore preferably used in the process according to the invention.

The linear, branched or cyclic aliphatic or aromatic polyisocyanate may be present as a monomer or polymer, respectively. A monomeric polyisocyanate is a molecule that is not attached to another molecule, in particular not via one or more crosslinking agents. A polymeric polyisocyanate comprises at least two monomers linked by one or more crosslinking agents. The at least two monomers need not necessarily be the same monomers, but may be different. A polymeric polyisocyanate preferably comprises at least 2 or more monomers, i.e., at least 2, 3, 4, 5, 10, 20, 30, 40, 50, 100 or more monomers attached to each other via at least one crosslinking agent.

The linear, branched, or cyclic aliphatic or aromatic polyisocyanate preferably has a limited size/ a limited molecular weight, which allows reactivity with the one or more crosslinking agents. Examples of suitable molecular weights preferably include ca. 100 g/mol to $5 \cdot 10^4$ g/mol, preferably 120 g/mol to $2 \cdot 10^4$ g/mol, 140 g/mol to $10^4$ g/mol, 160 g/mol to $5 \cdot 10^3$ g/mol, 180 g/mol to $2 \cdot 10^3$ g/mol, 200 g/mol to $10^3$ g/mol, 220 g/mol to 900 g/mol, 240 g/mol to 800 g/mol, 260 g/mol to 700 g/mol, 280 g/mol to 600 g/mol, 300 g/mol to 500 g/mol, 320 g/mol to 450 g/mol, or 340 g/mol to 400 g/mol.

Any number of different linear, branched and/or cyclic aliphatic and/or aromatic polyisocyanates may be used. For example, at least one, i.e., at least 1, 2, 3, 4, 5, 6, 7, 8, 9 or 10 different linear aliphatic polyisocyanates is/are used. For example, at least one, i.e., at least 1, 2, 3, 4, 5, 6, 7, 8, 9 or 10 different branched aliphatic polyisocyanates is/are used. For example, at least one, i.e., at least 1, 2, 3, 4, 5, 6, 7, 8, 9 or 10 different branched cyclic polyisocyanates is/are used.

Preferably, derivatives of linear, branched and/or cyclic aliphatic polyisocyanates are used. A derivative, as used herein, is understood in its broadest sense as a compound derived from a compound by a chemical reaction. Examples of derivatives include oligomers and/or adducts of the abovementioned linear or branched aliphatic polyisocyanates. Preferred oligomers are biurets, isocyanurates, uretdiones, iminooxadiazinediones and preferred adducts are trimethylolpropane adducts. These oligomers/adducts are well known in the state of the art and are disclosed for example in US 4855490 A or US 4144268 A.

Preferably, the aliphatic polyisocyanate is present only in monomeric form and/or dimerized form (as isocyanate) or in oligomeric form.

The derivatives of the linear, branched or cyclic polyisocyanates and/or mixtures thereof can also be obtained by reacting the polyisocyanates with polyalcohols (e.g., glycerol), polyamines, polythiols (e.g., dimercaprol).

The isocyanate compounds as defined above specifically comprise the various isomers, if present, alone or in combination. For example, methylene bis(cyclohexyl isocyanate) (H12MDI) comprises 4,4'-methylene bis(cyclohexyl isocyanate), 2,4'-methylene bis(cyclohexyl isocyanate) and/or 2,2'-methylene bis(cyclohexyl isocyanate).

Exemplary aliphatic polyisocyanates comprise those commercially available such as BAYHYDUR N304 and BAYHYDUR N3Q5, which are aliphatic water dispersible polyisocyanates based on hexamethylene diisocyanate, DESMODUR N3400, DESMODUR N3600, DESMODUR N3700 and DESMODUR N3900, which are low viscosity, polyfunctional hexamethylene diisocyanate-based aliphatic polyisocyanates, and DESMODUR 3600 and DESMODUR N100, which are hexamethylene diisocyanate-based aliphatic polyisocyanates, each of which is available from Bayer Corporation, Pittsburgh, PA.

According to another preferred variant of the present invention, the linear or branched aliphatic polyisocyanates is or are selected from the group consisting of pentamethylene diisocyanate (PDI, such as Stabio D-370N or D-376N from Mitsui Chemicals Inc., Japan), hexamethylene diisocyanate (HDI), ethyl ester lysine triisocyanate, lysine diisocyanate ethyl ester, and derivatives thereof, preferably wherein each of said derivatives comprises more than one isocyanate group and optionally further comprises one or more groups selected from the group consisting of biuret, isocyanurate, uretdione-, iminooxadiazinedione- and trimethylolpropane-adduct and/or wherein the cyclic aliphatic polyisocyanate(s) is or are selected from the group consisting of isophorone diisocyanate (IPDI), 1,3-bis (isocyanatomethyl) cyclohexane (H6XDI, such as Takenate 600 from Mitsui Chemicals Inc., Japan), 1,2-bis (isocyanatomethyl) cyclohexane, 1,4-bis (isocyanato-methyl) cyclohexane, methylenebis (cyclohexyl isocyanate) (H12MDI) and derivatives thereof, preferably wherein each of the derivatives comprises more than one isocyanate group and optionally further comprises one or more groups selected from the group consisting of biuret, isocyanurate, uretdione, iminooxadiazinione and trimethylolpropane adduct (such as TMP adduct) of H6XDI, in particular Takenate D-120N from Mitsui Chemicals Inc., Japan.

Aliphatic polyisocyanates obtained from renewable raw materials such as PDI (Stabio D-370N or D-376N from Mitsui Chemicals Inc., Japan) are particularly preferred. It was found that such aliphatic polyisocyanates obtained from renewable raw materials do not affect the quality/properties of the core-shell capsules.

Other suitable commercially available polyisocyanates include LUPRANAT M20 (BASF), where the average n is 0.7; PA PI 27 (Dow Chemical), where the average n is 0.7; MONDUR MR (Bayer), where the average n is 0.8; MONDUR MR Light (Bayer) with an average n of 0.8; MONDUR 489 (Bayer) where the average n is 1.0; poly- [(phenyl isocyanate)-co-formaldehyde (Aldrich Chemical, Milwaukee, WI), other isocyanate monomers such as DESMODUR N3200 (Bayer) and TAKENATE D110-N (Mitsui Chemicals Corporation, Rye Brook, NY). Other representative polyisocyanates include polyisocyanates designated as TAKENATE D-110N (Mitsui), DESMODUR L75 (Bayer), and DESMODUR IL (Bayer).

In a preferred variant, the polyisocyanate used in the preparation of the polyurea/polyurethane microcapsules according to the present invention is used as the sole polyisocyanate component, i.e., without the admixture of any other polyisocyanate component different therefrom.

Examples of the monomeric polyisocyanates which can be used according to the present invention, and which contain at least two polyisocyanate groups are: Ethylene diisocyanate, trimethylene diisocyanate, 1,4-tetramethylene diisocyanate, 1,6-hexamethyldiisocyanate, ethylene diisothiocyanate, tetramethylene diisothiocyanate, hexamethylene diisothiocyanate, cyclobutane-1,3-diisocyanate, cyclohexane-1,3-diisocyanate, cyclohexane-1,4-diisocyanate, 1,3-phenylene diisocyanate, 1,4-phenylene diisocyanate, mixtures of 1,3-phenylene diisocyanate and 1,4-phenylene diisocyanate, p-phenylene diisothiocyanate, xylylene-1,4-diisothiocyanate, 2,4-toluylene diisocyanate, 2,6-toluylene diisocyanate, mixtures of 2,4-toluylene diisocyanate and 2,6-toluylene diisocyanate, xylylene-1,4-diisocyanate, xylylene-1,3-diisocyanate and mixtures of xylylene-1,4-diisocyanate and xylylene-1,3-diisocyanate, 2,4-hexahydrotoluylene diisocyanate, 2,6-hexahydrotoluylene diisocyanate, mixtures of 2,4-hexahydrotoluylene diisocyanate and 2,6-hexahydrotoluylene diisocyanate, hexahydro-1,3-phenylene diisocyanate, hexahydro-1,4-phenylene diisocyanate, mixtures of hexahydro-1,4-phenylene diisocyanate and hexahydro-1,4-phenylene diisocyanate, 1,3-diisocyanatobenzene, 1,3,5-trimethylbenzene-2,4-diisocyanate, 1,3,5-triisopropylbenzene-2,4-diisocyanate, diphenylmethane-4,4'-diisocyanate, 3,3'-dimethyldiphenylmethane-4,4'-diisocyanate, 4,4'-diphenylpropane diisocyanate, naphthylene-1,4-diisocyanate, naphthylene-1,5-diisocyanate, triphenylmethane-4,4',4"-triisocyanate, toluylene-2,4,6-triisocyanate, dimethyldiphenylmethane-2,2',5,5'-tetraisocyanate, or mixtures of the foregoing compounds.

As polymerizable compounds containing at least two polyisocyanate groups, preference is preferably given to di- and polyisocyanates produced on a large scale, for example TDI: Toluylene diisocyanate (isomer mixture of 2,4- and 2,6-toluylene diisocyanate in a ratio of 80 : 20), HDI: Hexamethylene diisocyanate-(1,6), IPDI: Isophorone diisocyanate or DMDI: Diphenylmethane-4,4'-diisocyanate.

Other particularly preferred monomeric polyisocyanate compounds are: Diisocyanates such as 1,4-diisocyanatobutane, 1,6-diisocyanatohexane, 1,5-diisocyanato-2,2-dimethylpentane, 2,2,4- and 2,4,4-trimethyl-1,6-diisocyanatohexane, 1,10-diisocyanatodecane, 1,3- and 1,4-diisocyanatocyclohexane, 1-polyisocyanato-3,3,5-trimethyl-5-polyisocyanatomethylcyclohexane (isophorone diisocyanate), 4,4'-diisocyanatodicyclohexylmethane, 2,4- and 2,6-diisocyanatomethylcyclohexane and mixtures thereof. In principle, aromatic polyisocyanates, e.g., toluylene diisocyanates or 4,4'-diisocyanatodiphenylmetha can also be used.

Other specific examples of diisocyanates include, for example, 1,5-naphthylene diisocyanate, 4,4'-diphenylmethane diisocyanate (MDI), hydrogenated MDI (H12MDI), xylylene diisocyanate (XDI), tetramethylxylene diisocyanate (TMXD1) 4,4'-diphenyl dimethyl methane diisocyanate, di- and tetraalkyldiphenylmethane diisocyanate, 4,4'-dibenzyldiisocyanate, 1,3-phenylene diisocyanate, 1,4-phenylene diisocyanate, the isomers of toluylene diisocyanate (TDI), optionally in a mixture, 1-methyl-2,4-diisocyanatocyclohexane, 1,6-diisocyanato-2,2,4-trimethylhexane, 1,6-diisocyanato-2,4,4-trimethylhexane, 1-isocyanatomethyl-3-isocyanato-1,5,5-trimethylcyclohexane, chlorinated and brominated diisocyanates, phosphorus-containing diisocyanates, 4,4'-diisocyanatophenylperfluoroethane, tetramethoxybutane-1,4-diisocyanate, butane-1,4-diisocyanate, (HDI), dicyclohexylmethane diisocyanate, cyclohexane-1,4-diisocyanate, ethylene diisocyanate, phthalic acid bisisocyanatoethyl ester, also polyisocyanates with reactive halogen atoms such as 1-chloromethylphenyl-2,4-diisocyanate, 1-bromomethyl-phenyl-2,6-diisocyanate, 3,3-bischloromethyl ether-4,4'-diphenyl diisocyanate.

It was surprisingly found that especially the use of longer chained aliphatic diisocyanates with six, seven, eight, nine, ten or even more carbon atoms leads to the formation of more stable capsule shells or capsule walls.

In a particularly preferred embodiment, the internal non-aqueous phase comprises a mixture of two or more different polymerizable polyisocyanates, for example polyisocyanates with different chain lengths, which can form mixed polymerizates.

Proportionally, derivatives of polyisocyanates which can be prepared by modifying the abovementioned diisocyanates or mixtures thereof by known methods and which contain, for example, uretdione, urethane, isocyanurate, biuret and/or allophanate groups can also be used in the process according to the invention.

Very particularly preferred is a combination of at least two different, preferably aliphatic, polyisocyanates or a combination of at least one aliphatic and at least one aromatic polyisocyanate.

In such a combination, the different reaction rates of the polyisocyanates are exploited: Aromatic polyisocyanates react significantly faster than aliphatic polyisocyanates and the reaction rate is higher for the short-chained aliphatic polyisocyanates, i.e., aliphatic polyisocyanates with one to five carbon atoms, preferably three to five carbon atoms, compared to longer-chained analogs.

In a further preferred further embodiment of the invention, therefore, the different aliphatic and/or aromatic polyisocyanates also have different chain lengths. In this context, longer chained polyisocyanates preferably have six, seven, eight, nine, ten, eleven, twelve, thirteen, fourteen, twenty, twenty-five or more carbon atoms, but even more preferred they have six to twelve carbon atoms and particularly preferred six to eight carbon atoms. Shorter chained polyisocyanates are understood to be polyisocyanates having from one to five carbon atoms and preferably polyisocyanates having from three to five carbon atoms.

Preferred according to the invention is a combination of a short-chained aliphatic polyisocyanate (C1, C2, C3, C4, C5) and a long-chained aliphatic polyisocyanate (C6, C7, C8, C9, C10, C11, C12, C13, C14, C15, C20, C25 or more) or a combination of a short-chained aliphatic polyisocyanate (C1, C2, C3, C4, C5) with a long-chained aromatic polyisocyanate (C6, C7, C8, C9, C10, C11, C12, C13, C14, C15, C20, C25 or more) or a combination of a long-chained aliphatic polyisocyanate (C6, C7, C8, C9, C10, C11, C12, C13, C14, C15, C20, C25 or more) with a short-chained aromatic polyisocyanate.

Particularly preferred in this context is the use of a mixture of different aliphatic polyisocyanates having two or more isocyanate groups with chain lengths of one to twelve carbon atoms in the chain, preferably three to eight carbon atoms and particularly preferably four to seven carbon atoms, for the preparation of the biodegradable microcapsules according to the invention.

Aliphatic polyisocyanates are particularly preferred in this context due to their chemical relationship to biobased systems. For example, both lysine and 1,5-diisocyanatopentane have the same degradation product, 1,5-diaminopentane, and are therefore particularly suitable for use in the preparation of biobased and biodegradable microcapsules, taking into account environmental considerations.

Primary embodiments comprise mixtures of longer chained and shorter chained diisocyanates in any mixing ratio. More preferably, the mixing ratio of longer chained diisocyanates to shorter chained diisocyanates is in the range of from 4 : 1 to 1 : 4, and particularly preferred from 2 : 1 to 1 : 2.

Examples of preferred specific mixtures of at least one aliphatic polyisocyanate and of at least one aromatic polyisocyanate are a mixture of a biuret of hexamethylene diisocyanate with a trimethylol adduct of xylylene diisocyanate, a mixture of a biuret of hexamethylene diisocyanate with a polyisocyanurate of diisocyanate, or a mixture of a biuret of hexamethylene diisocyanate with a trimethylolpropane adduct of toluene diisocyanate.

According to the invention, it is even more preferred if, in the aforementioned combination of a short-chained aliphatic polyisocyanate and a long-chained aliphatic polyisocyanate, or in the combination of a short-chained aliphatic polyisocyanate and a long-chained aromatic polyisocyanate, or in the combination of a long-chained aliphatic polyisocyanate with a short-chained aromatic polyisocyanate, the polyisocyanates are present in a mixture of monomeric or oligomeric or polymeric forms, respectively.

Preferably, this results in the following combinations for use in the process according to the invention:

- short-chained aliphatic polyisocyanate (monomer or oligomer or polymer) and short-chained aliphatic polyisocyanate (monomer or oligomer or polymer);
- short-chained aliphatic polyisocyanate (monomer or oligomer or polymer) and long-chained aliphatic polyisocyanate (monomer or oligomer or polymer);
- short-chained aliphatic polyisocyanate (monomer or oligomer or polymer) and short-chained aromatic polyisocyanate (monomer or oligomer or polymer);
- short-chained aliphatic polyisocyanate (monomer or oligomer or polymer) and long-chained aromatic polyisocyanate (monomer or oligomer or polymer);
- long-chained aliphatic polyisocyanate (monomer or oligomer or polymer) and short-chained aliphatic polyisocyanate (monomer or oligomer or polymer);
- long-chained aliphatic polyisocyanate (monomer or oligomer or polymer) and long-chained aliphatic polyisocyanate (monomer or oligomer or polymer);
- long-chained aliphatic polyisocyanate (monomer or oligomer or polymer) and short-chain aromatic polyisocyanate (monomer or oligomer or polymer);
- long-chained aliphatic polyisocyanate (monomer) and long-chain aromatic polyisocyanate (oligomer or polymer);

with the previously described definitions for short-chained and long-chained.

It has been observed that the choice of at least two aliphatic polyisocyanates of different chain length and degree of polymerization, or the choice of mixtures of aliphatic and aromatic polyisocyanates, lead to a significant gain in stability and performance (fragrance release in the case of fragrance or odorant capsules) due to the different reaction rates of the polyisocyanate components, dissociations, and crosslinking structures.

With the previously mentioned polyisocyanate combinations or polyisocyanate mixtures of two different aliphatic or one aliphatic and one aromatic polyisocyanate, particularly stable and better, i.e., more densely branched crosslinks can be formed within the capsule shell.

Thus, performant (fragrance release) microcapsules made from either a mixture of an aliphatic and aromatic polyisocyanate or a mixture of two different aliphatic polyisocyanates can be prepared based on the process described herein. Such microcapsules are very stable and are characterized by outstanding fragrance storage properties, which in turn is reflected in a better performance (fragrance release) of the capsules, for example in the field of odorant or fragrance encapsulation.

The use of two different polyisocyanates results in microcapsules that further exceed the stability of microcapsules made from single polyisocyanate systems, as illustrated in the following embodiments.

The microcapsule made of an aliphatic-aliphatic polyisocyanate mixture is as good as a microcapsule made of an aliphatic-aromatic polyisocyanate mixture, as illustrated in the following embodiments. Accordingly, in principle, the combination of at least two different polymerizable (preferably aliphatic and/or aromatic) polyisocyanates is preferred in the present invention.

The proportion of the first crosslinking agent in the internal non-aqueous phase is in a range from 0.1 to 5 wt.-%, preferably in a range from 0.15 to 2.5 wt.-%, based on the total weight of the non-aqueous phase. Most preferably, the first crosslinking agent is provided in the internal non-aqueous phase in a range of 0.5 to 1 wt.-%, based on the total weight of the non-aqueous phase.

The first crosslinking agent is added to the internal non-aqueous phase either as such, for example as a solid, or in the form of an aqueous solution.

The first crosslinking agent is present in the aqueous solution in a concentration of 0.01 to 2 mol/l, preferably in a concentration of 0.1 to 1.5 mol/l, most preferred in a concentration of 0.5 to 1.0 mol/l. The solution has a pH-value of 7 to 14, preferably a pH-value of 12.

To improve the crosslinking of the at least one polysaccharide and/or the at least one protein, at least one further crosslinking agent is optionally added to the internal non-aqueous phase. The further crosslinking agent is different from the first crosslinking agent.

However, for example, in the case of chickpea-based microcapsules preferably no further crosslinking agent is used as the resulting microcapsules already show excellent properties, thus allowing for a reduction of overall components required for preparing efficient microcapsules. Nevertheless, further crosslinking agents could be added in order to further improve the capsule properties.

The further crosslinking agent is selected from the group consisting of transglutaminase, peroxidase, secondary plant substances selected from the group consisting of polyphenols, in particular tannin, gallic acid, ferulic acid, hesperidin, cinnamaldehyde, vanillin, carvacrol, and mixtures of two or more of the aforementioned crosslinking agents.

Transglutaminase, as an enzyme, catalyzes crosslinking via isopeptide bonds of two amino acids, glutamine, and lysine. The phenolic groups of the secondary plant compounds crosslink the peptides via hydrogen bonds. The aldehydes, cinnamaldehyde and vanillin, react covalently with the free amino groups of the proteins via the reactive aldehyde groups.

Particularly preferred of the aforementioned further crosslinking agents are cinnamaldehyde, tannin and gallic acid.

Particularly advantageous combinations of first crosslinking agent and further crosslinking agent are:

Polyisocyanate plus transglutaminase;
Polyisocyanate plus peroxidase;
Polyisocyanate plus polyphenyol;
Polyisocyanate plus tannin;
Polyisocyanate plus gallic acid;
Polyisocyanate plus ferulic acid;
Polyisocyanate plus hesperidin;
Polyisocyanate plus cinnamaldehyde;
Polyisocyanate plus vanillin;
Polyisocyanate plus carvacrol; or
Polyisocyanate plus a mixture of two or more of the further crosslinking agents described above.

The content of further crosslinking agent in the internal non-aqueous phase is in a range from 0.05 to 5 wt.-%, preferably in a range from 0.1 to 2 wt.-%, based on the total weight of the non-aqueous phase. Most preferably, the further crosslinking agent is used in the internal non-aqueous phase in a range of 0.15 to 1 wt.-%, based on the total weight of the non-aqueous phase.

The further crosslinking agent is added to the internal non-aqueous phase either as such, for example as a solid, or in the form of an aqueous solution.

The further crosslinking agent is present in the aqueous solution in a concentration of 0.01 to 2 mol/l, preferably in a concentration of 0.1 to 1.5 mol/l, most preferably in a concentration of 0.5 to 1.0 mol/l. The solution has a pH-value of 7 to 14, preferably a pH-value of 12.

The combined use of at least one first crosslinking agent and at least one further crosslinking agent, which are different from each other, results in microcapsules with a significantly improved stability and thus in a lower proportion of leaking perfume oil.

Due to the low proportion of the polyisocyanate component, it is possible according to the present invention to prepare protein- and/or polysaccharide-based microcapsules in which the absolute polyisocyanate proportion is only 1/50th of the total capsule comprising at least one lipophilic active ingredient to be encapsulated. Thus, protein- and/or polysaccharide-based microcapsules having a polyisocyanate content of only 0.6 wt.-%, based on the total weight of the capsule, can be prepared via the process according to the invention. Preferably, the polyisocyanate content is about 1.8 wt.-% of the capsule. Despite the low polyisocyanate content, the microcapsules according to the invention are nevertheless characterized by a high stability.

In step (i) of the process according to the invention, first the at least one crosslinking agent is substantially dissolved together with the at least one or more active ingredient(s) to be encapsulated, optionally in an inert non-aqueous solvent or a solvent mixture of inert non-aqueous solvents. By the term "substantially dissolved" it is understood that at least 90 wt.-%, preferably at least 98 wt.-%, more preferably 99.9 wt.-% of the aforementioned constituent is dissolved in the solvent or in the solvent mixture to be able to use it in the present process. Preferably, the at least one polyisocyanate and the at least one active ingredient to be encapsulated are completely dissolved in the solvent or in the solvent mixture. If a solvent does not provide sufficient solubility of the isocyanates, it is possible to overcome this drawback by using suitable solubility promoters.

Preferred solvents for the internal non-aqueous phase are immiscible with water and do not react with the isocyanate component(s) or the active ingredient component(s) and have little or no odor in the amounts used.

The term "solvent" in the context of the present invention comprises all types of oil bodies or oil components, in particular vegetable oils such as canola oil, sunflower oil, soybean oil, olive oil and the like, modified vegetable oils, e.g., alkoxylated sunflower or soybean oil, synthetic (tri) glycerides such as e.g., technical mixtures of mono-, di- and triglycerides of C6- to C22-fatty acids, fatty acid alkyl esters, e.g., methyl or ethyl esters of vegetable oils (Agnique® ME 18 RD-F, Agnique® ME 18 SD-F, Agnique® ME 12C-F, Agnique® ME1270), fatty acid alkyl esters based on these C6- to C22-fatty acids, mineral oils and mixtures thereof. Examples of suitable and preferred lipophilic solvents are: Guerbet alcohols based on fatty alcohols with 6 to 18, preferably 8 to 10, carbon atoms, esters of linear C6- to C22-fatty acids with linear or branched C6- to C22-fatty alcohols or -esters of branched C6- to C13-carboxylic acids with linear or branched C6- to C22-fatty alcohols, such as myristyl myristate, myristyl palmitate, myristyl stearate, myristyl isostearate, myristyl oleate, myristyl behenate, myristylerucate, cetyl myristate, cetyl palmitate, cetyl stearate, cetyl isostearate, cetyl oleate, cetyl behenate, cetylerucate, stearyl myristate, stearyl palmitate, stearyl stearate, stearyl isostearate, stearyl oleate, stearyl stearate, stearylerucate, isostearyl myristate, isostearyl palmitate, isostearyl stearate, isostearyl isostearate, isostearyl oleate, isostearyl behenate, oleyl myristate, oleyl palmitate, oleyl stearate, oleyl isostearate, oleyl oleate, oleyl behenate, oleylerucate, behenyl myristate, behenyl palmitate, behenyl stearate, behenyl isostearate, behenyl oleate, behenyl behenate, behenylerucate, erucyl myristate, erucyl palmitate, erucyl stearate, erucyl isostearate, erucyl oleate, erucyl behenate, and erucylerucate.

Also suitable are esters of linear C6- to C22-fatty acids with branched alcohols, in particular 2-ethylhexanol, esters of C18- to C38-alkyl hydroxycarboxylic acids with linear or branched C6- to C22-fatty acids, in particular dioctylalate, esters of linear or branched fatty acids with polyhydric alcohols (such as propylene glycol, dimer diol or trimer triol) and/or Guerbet alcohols, triglycerides based on C6- to C10-fatty acids, liquid mono-/di-/triglyceride mixtures of C6- to C18-fatty acids, esters of C6- to C22-fatty alcohols and/or Guerbet alcohols with aromatic carboxylic acids, in particular benzoic acid, esters of C2- to C12-dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear or branched C6- to C22-fatty alcohol carbonates, such as dicaprylyl carbonate (Cetiol® CC); Guerbet carbonates based on fatty alcohols with 6 to 18, preferably 8 to 10 carbon atoms, benzoic acid esters with linear or branched C6- to C22-alcohols, linear or branched, symmetrical or asymmetrical dialkyl ethers with 6 to 22 carbon atoms per alkyl group, such as dicaprylyl ethers, ring opening products of epoxidized fatty acid esters with polyols, silicone oils (cyclomethicones, silicone methicone grades, etc.), aliphatic or naphthenic hydrocarbons, such as squalane, squalene or dialkylcyclohexanes, and/or mineral oils.

Preferred solvents also include, in particular, esters of linear C6- to C22-fatty acids with branched alcohols, esters of C18- to C38-alkyl hydroxycarboxylic acids with linear or branched C6- to C22-fatty alcohols, linear or branched C6- to C22-fatty alcohols, in particular dioctyl malates, esters of linear or branched fatty acids with polyhydric alcohols, such as e.g., propylene glycol, dimer diol or trimer triol, and/or Guerbet alcohols, triglycerides based on C6- to C10-fatty acids, liquid mono-/di-/triglyceride mixtures based on C6- to C18-fatty acids, esters of C6- to C22-fatty alcohols and/or Guerbet alcohols with aromatic carboxylic acids, in particular benzoic acid, esters of C2- to C12-dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear and branched C6- to C22-fatty alcohol carbonates, such as e.g., dicaprylyl carbonate (Cetiol TM CC), Guerbet carbonates based on fatty alcohols with 6 to 18, preferably 8 to 10, carbon atoms, esters of benzoic acid with linear or branched C6- to C22-alcohols, linear or branched, symmetrical or asymmetrical dialkyl ethers with 6 to 22 carbon atoms per alkyl group, such as. e.g., dicaprylyl ethers (Cetiol TM OE), ring opening products of epoxidized fatty acid esters with polyols, silicone oils (cyclomethicones, silicon methicone types, etc.) and/or aliphatic or naphthenic hydrocarbons, such as squalane, squalene or dialkylcyclohexanes.

Furthermore, liquid linear and/or branched and/or saturated or unsaturated hydrocarbons or any desired mixtures thereof may be used as solvents within the scope of the present invention. These may be, for example, alkanes having 4 to 22, preferably 6 to 18 carbon atoms, or any mixtures thereof.

Particularly advantageously suitable as inert solvents for the internal non-aqueous phase are alkylaromatic hydrocarbons such as, for example, diisopropylnaphthalene or substituted biphenyls, chlorinated diphenyl, paraffins, chlorinated paraffins, natural vegetable oils such as, for example, cottonseed oil, peanut oil, palm oil, tricresyl phosphate, silicone oil, dialkyl phthalates, dialkyl adipates, partially hydrogenated terphenyl, alkylated biphenyl, alkylated naphthalene, diaryl ether, aryl alkyl ether, and higher alkylated benzene, benzyl benzoate, isopropyl myristate, and any mixtures of these hydrophobic solvents and mixtures of single or multiple of these hydrophobic solvents with kerosene, paraffins, and/or isoparaffins.

Preferably, vegetable oils triglycerides, benzyl benzoate or isopropyl myristate are used as solvents for providing the internal non-aqueous phase. Most preferred are vegetable oils selected from the group consisting of palm oil, soybean oil, canola oil, sunflower oil, palm seed oil, cottonseed oil, peanut oil, corn germ oil, coconut oil, olive oil, sesame oil, linseed oil, safflower oil, modified vegetable oils, as well as mixtures thereof.

The aforementioned solvents are used in the process according to the invention either individually or as a mixture of two or more solvents.

In an alternative and preferred variant of the process according to the invention, the at least one polyisocyanate is dissolved directly in a solution of the at least one active ingredient, preferably one or more fragrance or aroma substance/substances or a perfume oil, so that essentially no solvent, as described above, is present in the core of the microcapsule according to the invention. The avoidance of a solvent in the microcapsule core is advantageous in that it reduces manufacturing costs and addresses environmental concerns.

In particular, the fragrance or aroma substances are dissolved in solvents that are commonly used in the perfume or aroma industry. The solvent is preferably not an alcohol, since alcohols react with the isocyanates. Examples of suitable solvents include diethyl phthaloate, isopropyl myristate, Abalyn® (colophony resins, available from Eastman), benzyl benzoate, ethyl citrate, limonene or other terpenes or isoparaffins. Preferably, the solvent is highly hydrophobic. Preferably, the fragrance or aroma substance solution comprises less than 30% solvent. More preferably, the fragrance or aroma substance solution comprises less than 20% and even more preferably less than 10% solvent, all of these percentages being defined by weight relative to the total weight of the fragrance or aroma substance solution. Most preferably, the fragrance or aroma is substantially free of solvent.

When the at least one hydrophobic active ingredient is already in admixture with a solvent or solvent mixture, the use of an inert solvent or solvent mixture is not required. In such a case, the at least one first crosslinking agent can be mixed directly with the hydrophobic active agent to obtain an internal non-aqueous phase.

As active ingredient to be encapsulated or as core material for the preparation of the microcapsules according to the invention, basically any material suitable for inclusion in microcapsules can be considered in the process according to the invention. Preferably, hydrophobic, i.e., water-insoluble, or water-immiscible liquids or solids as well as suspensions can be considered as active ingredients to be encapsulated. These are predominantly non-polar substances. Such hydrophobic substances are almost always lipophilic, i.e., they dissolve well in fat and oil.

In the context of the present description, the core material is a hydrophobic active substance, i.e., a substance that has a specific effect or causes a specific reaction, for example, a drug, a pesticide, a cosmetic active ingredient, a food active ingredient, etc.

The at least one active ingredient to be encapsulated, which is used in the process according to the invention, is a hydrophobic or lipophilic active ingredient. This ensures that the active ingredient to be encapsulated is in the internal non-aqueous phase during the preparation of the microcapsule according to the invention and does not mix with the external aqueous phase, since otherwise no emulsion can form, and no precipitation of the capsule wall material can take place on the droplet surface. This results in the lipophilic drug being completely enclosed inside the microcapsule as core material during the subsequent emulsification and crosslinking of the capsule wall components. The internal non-aqueous phase thus formed is characterized by its organically hydrophobic, oily character.

In a particularly preferred variant of the present invention, the at least one lipophilic or hydrophobic active

EP 4 772 603 A2

ingredient is, in particular, a lipophilic or hydrophobic fragrance or aroma substance or a lipophilic or hydrophobic perfume oil or aroma (fragrance or aroma mixture), a cooling agent, a TRPV1 or a TRPV3 modulator, a substance that produces a pungent taste or a warmth or heat sensation on the skin or mucous membranes or a tingling sensation in the mouth or throat, or active ingredients with astringent effect, a pesticide, a biocide, an insecticide, a substance from the group of repellents, a food additive, a cosmetic active ingredient, a pharmaceutical active ingredient, a dye, a dye precursor; an agrochemical, a dye, a luminous paint, an optical brightener, a solvent, a wax, a silicone oil, a lubricant, a print coating for paper, or a mixture of two or more of the aforementioned active ingredients.

In a preferred variant of the present invention, hydrophobic or lipophilic active ingredients include, in particular, hydrophobic fragrant substances or fragrance mixtures of two or more fragrant substances (perfume oils) or hydrophobic aroma substances or aroma substance mixtures of two or more aroma substances (flavors) or also biogenic principles.

In a preferred embodiment according to the first and/or second aspect of the present invention, the microcapsules have a core material in the form of a hydrophobic single fragrant substance or single odorant substance, wherein the core material comprises at least one single fragrant substance or single odorant substance or mixtures thereof, selected from one or more of the following groups: Extracts of natural raw materials and also fractions thereof or components isolated therefrom; single fragrance substances from a group of hydrocarbons; aliphatic alcohols; aliphatic aldehydes and acetals; aliphatic ketones and oximes; aliphatic sulfur-containing compounds; aliphatic nitriles; esters of aliphatic carboxylic acids; formates, acetates, propionates, isobutyrates, butyrates, isovalerates, pentanoates, hexanoates, crotonates, tiglinates and 3-methyl-2-butenoates of acyclic terpene alcohols; acyclic terpene aldehydes and ketones as well as their dimethyl and diethyl acetals; formates, acetates, propionates, isobutyrates, butyrates, isovalerates, pentanoates, hexanoates, crotonates, tiglinates and 3-methyl-2-butenoates of cyclic terpene alcohols; cyclic terpene aldehydes and ketones; cyclic alcohols; cyclic and cycloaliphatic ethers; cyclic and macrocyclic ketones; cycloaliphatic aldehydes; cycloaliphatic ketones; esters of cyclic alcohols; esters of cycloaliphatic carboxylic acids; aromatic hydrocarbons; araliphatic alcohols; esters of araliphatic alcohols and aliphatic carboxylic acids; araliphatic ethers; aromatic and araliphatic aldehydes; aromatic and araliphatic ketones; aromatic and araliphatic carboxylic acids and their esters; nitrogen-containing aromatic compounds; phenyl ethers and phenyl esters; heterocyclic compounds; lactones; and mixtures of the above active ingredients.

Suitable fragrances and flavors for the preparation of the capsules according to the invention are described in the literature.

Preferably, the microcapsules according to the invention have a core material in the form of a hydrophobic single fragrance or single aroma, respectively, wherein the core material comprises at least one single fragrance or single aroma selected from one or more of the following groups:

- Hydrocarbons, such as e.g., 3-carene; a-pinene; beta-pinene; alpha-terpinene; gamma-terpinene; p-cymene; bisabolene; camphene; caryophyllene; cedrene; farnesene; limonene; longifolene; myrcene; ocimene; valencene; (E,Z)-1,3,5-undecatriene;

- Aliphatic alcohols, such as e.g., hexanol; octanol; 3-octanol; 2,6-dimethylheptanol; 2-methylheptanol, 2-methyloctanol; (E)-2-hexenol; (E)- and (Z)-3-hexenol; 1-octen-3-ol; mixture of 3,4,5,6,6-pentamethyl-3,4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;

- Aliphatic aldehydes and their acetals, such as e.g., hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-5,9-undecadienal; heptanaldiethyl acetal; 1,1-dimethoxy-2,2,5-trimethyl-4-hexene; citronellyl-oxyacetaldehyde;

- Aliphatic ketones and their oximes, such as e.g., 2-heptanone; 2-octanone; 3-octanone; 2-nonanone; 5-methyl-3-heptanone; 5-methyl-3-heptanone oxime; 2,4,4,7-tetramethyl-6-octen-3-one;

- Aliphatic sulfur-containing compounds, such as e.g., 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthen-8-thiol;

- Aliphatic nitriles, such as e.g., 2-nonenoic acid nitrile; 2-tridecenoic acid nitrile; 2,12-tridecenoic acid nitrile; 3,7-dimethyl-2,6-octadienoic acid nitrile; 3,7-dimethyl-6-octenoic acid nitrile;

- Aliphatic carboxylic acids and their esters, such as e.g., (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl-2-methyl pentanoate; ethyl hexanoate; allyl hexanoate; ethyl heptanoate; allyl heptanoate; ethyl octanoate; ethyl-(E,Z)-2,4-decadienoate; methyl-2-octinate; methyl-2-noninate; allyl-2-isoamyloxyacetate; methyl-3,7-dimethyl-2,6-octadienoate;

- Acyclic terpene alcohols, such as e.g., citronellol; geraniol; nerol; linalool; lavadulol; nerolidol; farnesol; tetrahydrolinalool; tetrahydrogeraniol; 2,6-dimethyl-7-octen-2-ol; 2,6-dimethyloctan-2-ol; 2-methyl-6-methylene-7-octen-2-ol;

2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-I-ol; and their formates, acetates, propionates, isobutyrates, butyrates, isovalerianates, pentanoates, hexanoates, crotonates, tiglinates, 3-methyl-2-butenoates;

- Acyclic terpene aldehydes and ketones, such as e.g., geranial; neral; citronellal; 7-hydroxy-3,7-dimethyloctanal; 7-methoxy-3,7-dimethyloctanal; 2,6,10-trimethyl-9-undecenal; geranyl acetone; and the dimethyl and diethylacetals of geranial, neral, 7-hydroxy-3,7-dimethyloctanal;

- Cyclic terpene alcohols, such as e.g., menthol; isopulegol; a-terpineol; terpinenol-4; menthan-8-ol; menthan-1-ol; menthan-7-ol; borneol; isoborneol; linalool oxide; nopol; cedrol; ambrinol; vetiverol; guaiaol; and their formates, acetates, propionates, isobutyrates, butyrates, isovalerianates, pentanoates, hexanoates, crotonates, tiglinates, 3-methyl-2-butenoates;

- Cyclic terpene aldehydes and ketones, such as e.g., menthone; isomenthone; 8-mercaptomenthan-3-one; carvone; camphor; fenchone; $\alpha$-ionone; beta-ionone; $\alpha$-n-methylionone; beta-n-methylionone; $\alpha$-isomethylionone; beta-iso-methylionone; $\alpha$-irone; $\beta$-irone; $\alpha$-damascenone; beta-damascenone; gamma-damascenone; d-damascenone; 1-(2,4,4-trimethyl-2-cyclohexen-1-yl)-2-buten-1-one; 1,3,4,6,7,8a-hexahydro-1,1,5,5-tetramethyl-2H-2,4a-methanonaphthalen-8(5H)-one; nootkatone; dihydronootkatone; $\alpha$-sinensal; beta-sinensal; acetylated cedarwood oil (methylcedryl ketone);

- Cyclic alcohols, such as e.g., 4-tert-butylcyclohexanol; 3,3,5-trimethylcyclohexanol; 3-isocamphylcyclohexanol; 2,6,9-trimethyl-(Z2,Z5,E9)-cyclododecatrien-1-ol; 2-isobutyl-4-methyltetrahydro-2H-pyran-4-ol; from the group of cycloaliphatic alcohols such as e.g., 3,3,3-trimethyl-cyclohexylmethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)-pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclo-hexyl)pentan-3-ol; 1-(2,2,6-trimethylcyclo-hexyl)hexan-3-ol;

- Cyclic and cycloaliphatic ethers, such as e.g., cineol; cedryl methyl ether; cyclododecyl methyl ether; (ethoxymethoxy)cyclododecane; a-cedrene epoxide; 3a,6,6,9a-tetramethyldodecahydronaphtho[2,1-b]furan; 3a-ethyl-6,6,9a-trimethyl-dodecahydronaphtho[2,1-b]furan; 1,5,9-trimethyl-13-oxabicyclo[10.1.0]trideca-4,8-diene; rose oxide; 2-(2,4-dimethyl-3-cyclohexen-1-yl)-5-methyl-5-(1-methylpropyl)-1,3-dioxane;

- Cyclic ketones, such as e.g., 4-tert-butylcyclohexanone; 2,2,5-trimethyl-5-pentylcyclopentanone; 2-heptylcyclopentanone; 2-pentylcyclopentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclo-hexadecen-1-one; 6,7-dihydro-1,1,2,3,3-pentamethyl-4(5H)-indanone; 9-cycloheptadecen-1-one; cyclopentadecanone; cyclohexadecanone;

- Cycloaliphatic aldehydes, such as e.g., 2,4-dimethyl-3-cyclohexenecarbaldehyde; 2-methyl-4-(2,2,6-trimethyl-cyclohexen-1-yl)-2-butenal; 4-(4-hydroxy-4-methylpentyl)-3-cyclohexenecarbaldehyde; 4-(4-methyl-3-penten-1-yl)-3-cyclohexene carbaldehyde;

- Cycloaliphatic ketones, such as e.g., 1-(3,3-dimethylcyclohexyl)-4-penten-1-one; 1-(5,5-dimethyl-2-cyclohexen-1-yl)-4-penten-1-one; 2,3,8,8-tetramethyl-1,2,3,4,5,6,7,8-octahydro-2-naphthalenyl methyl ketone; methyl-2,6,10-trimethyl-2,5,9-cyclododecatrienyl ketone; tert-butyl-(2,4-dimethyl-3-cyclohexen-1-yl) ketone;

- Esters of cyclic alcohols, such as e.g., 2-tert-butylcyclohexyl acetate; 4-tert-butylcyclohexyl acetate; 2-tert-pentylcyclohexyl acetate; 4-tert-pentylcyclohexyl acetate; decahydro-2-naphthyl acetate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5- or -6-indenyl acetate; 4,7-methano-3a,4,5,6,7,7a-hexahydro-5- or - 6-indenyl propionate; 4,7-methano-3a,4,5,6,7,7a-hexahydro-5- or -6-indenyl isobutyrate; 4,7-methanooctahydro-5- or -6-indenyl acetate;

- Esters of cycloaliphatic carboxylic acids, such as e.g., allyl-3-cyclohexyl propionate; allyl cyclohexyloxy acetate; methyl dihydrojasmonate; methyl jasmonate; methyl-2-hexyl-3-oxocyclopentanecarboxylate; ethyl-2-ethyl-6,6-dimethyl-2-cyclohexene carboxylate; ethyl-2,3,6,6-tetramethyl-2-cyclohexene carboxylate; ethyl-2-methyl-1,3-dioxolane-2-acetate;

- Aromatic hydrocarbons, such as e.g., styrene and diphenylmethane;

- Araliphatic alcohols, such as e.g., benzyl alcohol; 1-phenylethyl alcohol; 2-phenylethyl alcohol; 3-phenyl propanol; 2-phenyl propanol; 2-phenoxyethanol; 2,2-dimethyl-3-phenyl propanol; 2,2-dimethyl-3-(3-methylphenyl)propanol; 1,1-dimethyl-2-phenyl ethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;

- Esters of araliphatic alcohols and aliphatic carboxylic acids, such as e.g., benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; $\alpha$-trichloromethyl benzyl acetate; a,a-dimethylphenylethyl acetate; a,a-dimethylphenylethyl butyrate; cinnamyl acetate; 2-phenoxyethyl isobutyrate; 4-methoxybenzyl acetate;

**EP 4 772 603 A2**

- Araliphatic ethers, such as e.g., 2-phenylethyl methyl ether; 2-phenylethyl isoamyl ether; 2-phenylethyl-1-ethoxyethyl ether; phenylacetaldehyde dimethylacetal; phenylacetaldehyde diethylacetal; hydratropaaldehyde dimethylacetal; phenylacetaldehyde glycerol acetal; 2,4,6-trimethyl-4-phenyl-1,3-dioxanes; 4,4a,5,9b-tetrahydroindeno[1,2-d]-m-dioxin; 4,4a,5,9b-tetrahydro-2,4-dimethyl-indeno[1,2-d]-m-dioxin;

- Aromatic and araliphatic aldehydes, such as e.g., benzaldehyde; phenylacetaldehyde; 3-phenylpropanal; hydratropaaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-tert-butylphenyl)propanal; 3-(4-tert-butylphenyl)propanal; cinnamaldehyde; a-butyl-cinnamaldehyde; a-amylcinnamaldehyde; a-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxy benzaldehyde; 2-methyl-3-(4-methoxyphenyl)propanal; 2-methyl-3-(4-methylenedioxyphenyl)propanal;

- Aromatic and araliphatic ketones, such as e.g., acetophenone; 4-methylacetophenone; 4-methoxyacetophenone; 4-tert-butyl-2,6-dimethyl-acetophenone; 4-phenyl-2-butanone; 4-(4-hydroxyphenyl)-2-butanone; 1-(2-naphthalenyl) ethanone; benzophenone; 1,1,2,3,6-hexamethyl-5-indanyl methyl ketone; 6-tert-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;

- Aromatic and araliphatic carboxylic acids and their esters, such as e.g., benzoic acid; phenylacetic acid; methyl benzoate; ethyl benzoate; hexyl benzoate; benzyl benzoate; methyl phenyl acetate; ethyl phenyl acetate; geranyl phenyl acetate; phenyl ethyl phenyl acetate; methyl cinnamate; ethyl cinnamate; benzyl cinnamate; phenyl ethyl cinnamate; cinnamyl cinnamate; allylphenoxy acetate; methyl salicylate; isoamyl salicylate; hexyl salicylate; cyclohexyl salicylate; cis-3-hexenyl salicylate; benzyl salicylate; phenyl ethyl salicylate; methyl-2,4-dihydroxy-3,6-dimethyl benzoate; ethyl-3-phenyl glycidate; ethyl 3-methyl-3-phenyl glycidate;

- Nitrogen-containing aromatic compounds, such as e.g., 2,4,6-trinitro-1,3-dimethyl-5-tert-butylbenzene; 3,5-dinitro-2,6-dimethyl-4-tert-butylacetophenone; cinnamic acid nitrile; 5-phenyl-3-methyl-2-pentenoic acid nitrile; 5-phenyl-3-methylpentanoic acid nitrile; methyl anthranilate; methyl-N-methyl anthranilate; Schiff bases of methylanthranilate with 7-hydroxy-3,7-dimethyloctanal, 2-methyl-3-(4-tert-butylphenyl)propanal or 2,4-dimethyl-3-cyclohexene-carbaldehyde; 6-isopropyl-quinoline; 6-isobutylquinoline; 6-sec-butylquinoline; indole; scatole; 2-methoxy-3-isopropylpyrazine; 2-isobutyl-3-methoxypyrazine; 4-(4,8-dimethyl-3,7-nonadienyl)-pyridine;

- Phenols, phenyl ethers and phenyl esters, such as e.g., estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresylphenyl acetate; from the group of heterocyclic compounds such as e.g., 2,5-Dimethyl-4-hydroxy-2H-furan-3-on; 2-ethyl-4-hydroxy-5-methyl-2H-furan-3-on; 3-hydroxy-2-methyl-4H-pyran-4-on; 2-ethyl-3-hydroxy-4H-pyran-4-on;

- Lactones, such as e.g., 1,4-octanolide; 3-methyl-1,4-octanolide; 1,4-nonanolide; 1,4-decanolide; 8-decene-1,4-olide; 1,4-undecanolide; 1,4-dodecanolide; 1,5-decanolide; 1,5-dodecanolide; 1,15-pentadecanolide; cis- and trans-11-pentadecene-1,15-olide; cis- and trans-12-pentadecene-1,15-olide; 1,16-hexadecanolide; 9-hexadecene-1,16-olide; 10-oxa-1,16-hexadecanolide; 11-oxa-1,16-hexadecanolide; 12-oxa-1,16-hexadecanolide; ethylene-1,12-dodecanedioate; ethylene-1,13-tridecanedioate; coumarin; 2,3-dihydrocoumarin; octahydrocoumarin;

as well as the stereoisomers, enantiomers, positional isomers, diastereomers, cis/trans isomers and epimers, respectively, of the aforementioned substances.

Of the aforementioned single fragrant substances or single odorant substances that can be encapsulated within the spirit of the present invention, fragrances or odorants that have an aldehyde, carboxylic acid or ester functionality are particularly preferred for use.

Aldehydic fragrances or odorants, which also include the corresponding acetals as well as esters and lactones, can be divided into the following groups, namely

(i) aliphatic aldehydes and their acetals;
(ii) cycloaliphatic aldehydes;
(iii) aromatic or araliphatic aldehydes;
(iv) aliphatic, aromatic or araliphatic esters; and
(v) lactones;

and mixtures thereof, respectively.

The aforementioned fragrant and odorant substances having aldehyde, carboxylic acid or ester functionality, as well as mixtures thereof, are selected from one or more of the following groups:

- Aliphatic aldehydes and their acetals, such as e.g., hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-5,9-undecadienal; heptanal diethyl acetal; 1,1-dimethoxy-2,2,5-trimethyl-4-hexene; citronellyl-oxyacetaldehyde;
- Cycloaliphatic aldehydes, such as e.g., 2,4-dimethyl-3-cyclohexenecarbaldehyde; 2-methyl-4-(2,2,6-trimethyl-cyclohexene-1-yl)-2-butenal; 4-(4-hydroxy-4-methylpentyl)-3-cyclohexenecarbaldehyde; 4-(4-methyl-3-penten-1-yl)-3-cyclohexene-carbaldehyde;
- Aromatic and araliphatic aldehydes, such as e.g., benzaldehyde; phenylacetaldehyde; 3-phenylpropanal; hydratropaaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-tert-butylphenyl)-propanal; 3-(4-tert-butylphenyl)propanal; cinnamaldehyde; $\alpha$-butyl-cinnamaldehyde; $\alpha$-amylcinnamaldehyde; $\alpha$-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
- Aliphatic carboxylic acid esters, such as e.g., (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl-2-methyl pentanoate; ethyl hexanoate; allyl hexanoate; ethyl heptanoate; allyl heptanoate; ethyl octanoate; ethyl-(E,Z)-2,4-decadienoate; methyl-2-octinate; methyl-2-noninate; allyl-2-isoamyloxyacetate; methyl-3,7- dimethyl-2,6-octadienoate;
- Esters of cyclic alcohols, such as e.g., 2-tert-butylcyclohexyl acetate; 4-tert-butylcyclohexyl acetate; 2-tert-pentylcyclohexyl acetate; 4-tert-pentylcyclohexyl acetate; decahydro-2-naphthyl acetate; 3-pentyl tetrahydro-2/-/-pyran-4-yl acetate; decahydro-2,5,5,8a-tetramethyl-2-naphthyl acetate; 4,7-methano-3a,4,5,6,7,7a-hexahydro-5- or -6-indenyl acetate; 4,7-methano-3a,4,5,6,7,7a-hexahydro-5- or -6-indenyl propionate; 4,7-methano-3a,4,5,6,7,7a-hexahydro-5- or -6-indenyl isobutyrate; 4,7-methanooctahydro-5- or -6-indenyl acetate;
- Esters of araliphatic alcohols and aliphatic carboxylic acids, such as e.g., benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; a-trichloromethyl benzyl acetate; $\alpha,\alpha$-dimethylphenylethyl acetate; $\alpha,\alpha$-dimethylphenylethyl butyrate; cinnamyl acetate; 2-phenoxyethyl isobutyrate; 4-methoxybenzyl acetate;
- Esters of cycloaliphatic carboxylic acids, such as e.g., allyl-3-cyclohexyl propionate; allyl cyclohexyloxy acetate; methyl dihydrojasmonate; methyl jasmonate; methyl-2-hexyl-3-oxocyclopentanecarboxylate; ethyl-2-ethyl-6,6-dimethyl-2-cyclohexene-carboxylate; ethyl-2,3,6,6-tetramethyl-2-cyclohexenecarboxylate; ethyl-2-methyl-1,3-dioxolane-2-acetate;
- Aromatic and araliphatic carboxylic acid esters, such as e.g., methyl benzoate; ethyl benzoate; hexyl benzoate; benzyl benzoate; methyl phenyl acetate; ethyl phenyl acetate; geranyl phenyl acetate; phenyl ethyl phenyl acetate; methyl cinnamate; ethyl cinnamate; benzyl cinnamate; phenyl ethyl cinnamate; cinnamyl cinnamate; allyl phenoxy acetate; methyl salicylate; Isoamyl salicylate; hexyl salicylate; cyclohexyl salicylate; cis-3-hexenyl salicylate; benzyl salicylate; phenylethyl salicylate; methyl-2,4-dihydroxy-3,6-dimethylbenzoate; ethyl-3-phenyl glycidate; ethyl-3-methyl-3-phenyl glycidate.

Listed below are aldehydes, acetals, esters and lactones with their commercial designations, which are particularly preferred as representatives of groups (i) to (v) in the sense of the process according to the invention:
Aldehydes: 2-methylpentanal; Aldehyde C12 MNA HM; Aldehyde C 4; Aldehyde C 5; Aldehyde C 6; Aldehyde C 7; Aldehyde C 8; Aldehyde C 9; Aldehyde C 10; Aldehyde C 11 ISO; Aldehyde C 11 MOA PURE; Aldehyde C 11 UNDECANAL; Aldehyde C 11 UNDEYLENIC; Aldehyde C 12; Aldehyde C 12 MNA; Aldehyde C 13; ALDEHYDE MADARINE; AMYL CINNAMIC ALDEHYDE ALPHA; ANISALALDEHYDE-O; ANISYL ALDEHYDE; BENZALDEHYDE NAT.; BERGAMAL; BORONAL; BOURGENOAL; CAMPHONELIC ALDEHYDE; CITRAL; CITRONELLAL HM; CITRONELLYL OXYACET ALDEHYDE; CITRYLAL; CITROYLAL E HM; CORTEX ALDEHYDE; CORTEX ALDEHYDE 50 PCT PEMOSA; CROTONIC ALDEHYDE; CUMINAL ALDEHYDE; CYCLAMEN ALDEHYDE; DECADIENAL TRANS,TRANS-2,4, DECANAL CIS-4; DECANAL TRANS-2; DECANAL TRANS-2 NAT; DECANAL TRANS-4; DECANAL-9,1; DODECANIENAL 2,6; DODECANAL TRANS-2; DUPICAL; EPOXYDECENAL-4,5-2 10% TRI; ETHYL HEXANAL; FARENAL®; FLORHYDRAL; GERALDEHYDE; HELIONAL; HELIOPAN; HELIOTROPIN; HEPTADIENAL TRANS,TRANS,2-4; HEPTENAL CIS-4; HEPTENAL TRANS-2; HEXENAL TRANS-2; HEXYL CINNAMIC ALDEHYDE ALPHA; HYDRATROPIC ALDEHYDE; HYDROXY CITRONELLAL; INTRELEVEN ALDEHYDE SPEC.; ISONONYL ALDEHYDE; ISOVALERIC ALDEHYDE; LEMON ALDEHYDE H&R JS I; LILIAL; LINOLAL; LYRAL; MAJANTAL; MANDRINAL; MANDRAINE ALDEHYDE 10% IN TEC BHT; MEFRANAL; MELONAL®; METHODY CITRONELLAL; METHYL BUTYRALDEHYDE; METHYL CINNAMIC ALDEHYDE ALPHA; METHYL PHENYLPENTENAL-4,2,2; METHYL THIO PROPANAL-3; METHYL TRIDECANAL-12 10% VT; METHYL-3-BUTEN-2-AL; METHYL-5-PHENYL-2-HEXENE-2-AL; MUGENAL 50 DPG; NEOCYCLO CITRAL; NONADIENAL; TRANS,CIS-2,6; NONENAL CIS-6; NONENAL TRANS-2; ONCIDAL® 3/060251; PENTENAL TRANS-2; PERILLA ALDEHYDE; PHENYLACET ALDEHYDE; PHENYLBUTENAL TRANS-2,2; PHENYLPROPYL ALDEHYDE; PINOACET ALDEHYDE; PROFRANESAL; PROPIONALALDEHYDE 2-(P-TOLYL); PROPIONIC ALDEHYDE; PS-IRALDEIN X NEU; SAFRANAL; SALICYLIC ALDEHYDE FG; SILVIAL; TETRAHYDRO CITRAL; TIGLIC ALDEHYDE-2,2; TOLYL ALDEHYDE PARA FG; TRIDECENAL TRANS-2; TRIFERNAL; UNDECADIENAL-2,4; UNDECENAL TRANS-2; VERNALALDEHYDE; VERTOCITRAL; VERTOMUGAL; VERTIPRENAL; VETRAL CRUDE; CINNAMALDEHYDE NAT. HM; Acetals: FLOROPAL; HEPTANAL DIETHYL ACETAL; NONANDIENAL DIETHYL ACETAL; OKOUMAL; PHENYLACET ALD. GLYCERIN ACETAL; PHENYLACETALDEYHDEDIMETHYLACETAL; Esters: JASMAL; JESSEMAL; KHARISMAL; TIRAMISONE®.

In a further variant of the process according to the invention, aroma substances can also be encapsulated as a core material in the form of a single aroma, wherein the core material comprises at least one single aroma substance or mixtures thereof as active ingredient.

Typical examples of aroma substances or flavors that can be encapsulated in accordance with the invention are selected from the group consisting of: Acetophenone; allyl capronate; alpha-ionone; beta-ionone; anisaldehyde; anisyl acetate; anisyl formate; benzaldehyde; benzothiazole; benzyl acetate; benzyl alcohol; benzyl benzoate; beta-ionone; butyl butyrate; butyl capronate; butylidene phthalide; carvone; camphene; caryophyllene; cineole; cinnamyl acetate; citral; citronellol; citronellal; citronellyl acetate; cyclohexyl acetate; cymene; damascone; decalactone; dihydrocoumarin; dimethylanthranilate; dimethyl anthranilate; dodecalactone; ethoxyethyl acetate; ethyl butyric acid; ethyl butyrate; ethyl caprinate; ethyl capronate; ethyl crotonate; ethyl furaneol; ethyl guaiacol; ethyl isobutyrate; ethyl isovalerate; ethyl lactate; ethyl methyl butyrate; ethyl propionate; eucalyptol; eugenol; ethyl heptylate; 4-(p-hydroxyphenyl)-2-butanone; gamma-decalactone; geraniol; geranyl acetate; geranyl acetate; grapefruit aldehyde; methyl dihydrojasmonate (e.g., Hedion®); heliotropin; 2-heptanone; 3-heptanone; 4-heptanone; trans-2-heptenal; cis-4-heptenal; trans-2-hexenal; cis-3-hexenol; trans-2-hexenoic acid; trans-3-hexenoic acid; cis-2-hexenyl acetate; cis-3-hexenyl acetate; cis-3-hexenyl capronate; trans-2-hexenyl capronate; cis-3-hexenyl formate; cis-2-hexyl acetate; cis-3-hexyl acetate; trans-2-hexyl acetate; cis-3-hexyl formate; para-hydroxybenzylacetone; isoamyl alcohol; isoamyl isovalerate; isobutyl butyrate; isobutyraldehyde; isoeugenol methyl ether; isopropyl methyl thiazole; lauric acid; leavulinic acid; linalool; linalool oxide; linalyl acetate; menthol; menthofuran; methyl anthranilate; methyl butanol; methyl butyric acid; 2-methyl butyl acetate; methyl capronate; methyl cinnamate; 5-methyl furfural; 3,2,2-methylcyclopentenolone; 6,5,2-methylheptenone; methyl dihydrojasmonate; methyl jasmonate; 2-methyl methyl butyrate; 2-methyl-2-pentenolic acid; methyl thiobutyrate; 3,1-methylthiohexanol; 3-methylthiohexyl acetate; nerol; neryl acetate; trans,trans-2,4-nonadienal; 2,4-nonadienol; 2,6-nonadienol; 2,4-nonadienol; nootkatone; delta-octalactone; gamma-octalactone; 2-octanol; 3-octanol; 1,3-octenol; 1-octyl acetate; 3-octyl acetate; palmitic acid; paraldehyde; phellandrene; pentanedione; phenylethyl acetate; phenylethyl alcohol; phenylethyl alcohol; phenylethyl isovalerate; piperonal; propionaldehyde; propyl butyrate; pulegone; pulegol; sinensal; sulfurol; terpinene; terpineol; terpinolene; 8,3-thiomenthanone; 4,4,2-thiomethylpentanone; thymol; delta-undecalactone; gamma-undecalactone; valencene; valeric acid; vanillin; acetoin; ethylvanillin; ethylvanillin isobutyrate (3-ethoxy-4-isobutyryloxybenzaldehyde); 2,5-dimethyl-4-hydroxy-3(2H)-furanone and its derivatives (preferably homofuraneol (2-ethyl-4-hydroxy-5-methyl-3(2H)-furanone), homofuronol (2-ethyl-5-methyl-4-hydroxy-3(2H)-furanone and 5-ethyl-2-methyl-4-hydroxy-3(2H)-furanone); maltol and maltol derivatives (preferably ethylmaltol); coumarin and coumarin derivatives; gamma-lactones (preferably gamma-undecalactone, gamma-nonalactone, gamma-decalactone); delta-lactones (preferably 4-methyldeltadecalactone, massoilactone, deltadecalactone, tuberolactone); methyl sorbate; divanillin; 4-hydroxy-2(or 5)-ethyl-5(or 2)-methyl-3(2H)furanone; 2-hydroxy-3-methyl-2-cyclopentenone; 3-hydroxy-4,5-dimethyl-2(5H)-furanone; acetic acid isoamyl ester; butyric acid ethyl ester; butyric acid-n-butyl ester; butyric acid isoamyl ester; 3-methyl butyric acid ethyl ester; n-hexanoic acid ethyl ester; n-hexanoic acid allyl ester; n-hexanoic acid-n-butyl ester; n-octanoic acid ethyl ester; ethyl-3-methyl-3-phenyl glycidate; ethyl-2-trans-4-cis-decadienoate; 4-(p-hydroxyphenyl)-2-butanone; 1,1-dimethoxy-2,2,5-trimethyl-4-hexane; 2,6-dimethyl-5-heptene-1-al; phenyl-acetaldehyde; 2-methyl-3-(methylthio)furan; 2-methyl-3-furanthiol; bis(2-methyl-3-furyl)disulfide; furfuryl mercaptan; methional; 2-acetyl-2-thiazoline; 3-mercapto-2-pentanone; 2,5-dimethyl-3-furanthiol; 2,4,5-trimethylthiazole; 2-acetylthiazole; 2,4-dimethyl-5-ethylthiazole; 2-acetyl-1-pyrroline; 2-methyl-3-ethylpyrazine; 2-ethyl-3,5-dimethylpyrazine; 2-ethyl-3,6-dimethylpyrazine; 2,3-diethyl-5-methylpyrazine; 3-isopropyl-2-methoxypyrazine; 3-isobutyl-2-methoxypyrazine; 2-acetylpyrazine; 2-pentylpyridine; (E,E)-2,4-decadienal; (E,E)-2,4-nonadienal; (E)-2-octenal; (E)-2-nonenal; 2-undecenal; 12-methyltridecanal; 1-penten-3-one; 4-hydroxy-2,5-dimethyl-3(2H)-furanone; guaiacol; 3-hydroxy-4,5-dimethyl-2(5H)-furanone; 3-hydroxy-4-methyl-5-ethyl-2(5H)-furanone; cinnamaldehyde; cinnamalcohol; methyl salicylate; isopulegol as well as the stereoisomers, enantiomers, positional isomers, diastereomers, cis/trans isomers or epimers of these substances not explicitly mentioned herein; and mixtures of the aforementioned substances.

In an alternative embodiment according to the present invention, the microcapsules according to the invention use a fragrance substance mixture or a perfume oil, respectively, or an aroma mixture or an aroma as the active ingredient to be encapsulated or as the core material. These are compositions containing at least one fragrance substance or one aroma substance. Such compositions, in particular fragrance mixtures or perfume oils, preferably comprise two, three, four, five, six, seven, eight, nine, ten or more fragrance substances. The fragrance mixtures or perfume oils, respectively, are preferably selected from the group of extracts from natural raw materials, such as essential oils, concretes, absolutes, resins, resinoids, balsams, tinctures such as e.g., Ambergris oil; Amyris oil; Angelica seed oil; Angelica root oil; Anise oil; Valerian oil; Basil oil; Tree moss absolute; Bay oil; Mugwort oil; Benzoin resin; Bergamot oil; Beeswax absolute; Birch tar oil; Bitter almond oil; Savory oil; Bucco leaf oil; Cabreuva oil; Cade oil; Calmus oil; Camphor oil; Cananga oil; Cardamom oil; Cascarilla oil; Cassia oil; Cassie absolute; Castoreum absolute; Cedar leaf oil; Cedarwood oil; Cistus oil; Citronella oil; Citron oil; Copaiva balsam; Copaiva balsam oil; Coriander oil; Costus root oil; Cumin oil; Cypress oil; Davana oil; Dill herb oil; Dill seed oil; Eau de brouts absolute; Oak moss absolute; Elemi oil; Tarragon oil; Eucalyptus citriodora oil; Eucalyptus oil; Fennel oil; Spruce needle oil; Galbanum oil; Galbanum resin; Geranium oil; Grapefruit oil; Guaiac wood oil; Gurjun balsam; Gurjun balsam oil, Helichrysum absolute; Helichrysum oil; Ginger oil; Iris root absolute; Iris root oil; Jasmine absolute; Calamus oil; Chamomile oil blue; Chamomile oil Roman; Carrot seed oil; Cascarilla oil; Pine needle oil; Curly mint oil; Caraway seed oil; Labdanum oil; Labdanum absolute; Labdanum resin; Lavandin absolute; Lavandin oil; Lavender absolute; Lavender oil; Lemongrass oil; Lovage oil; Lime oil distilled; Lime oil pressed; Linal oil; Litsea cubeba oil; Bay leaf oil; Macis oil; Marjoram oil; Mandarin oil; Masso bark oil; Mimosa absolute; Musk grain oil; Musk tincture; Muscat sage oil; Muscat oil; Myrrh absolute; Myrrh oil; Myrtle oil; Clove leaf oil; Clove flower oil; Neroli oil; Olibanum absolute; Olibanum oil; Opopanax oil; Orange flower absolute; Orange oil; Origanum oil; Palmarosa oil; Patchouli oil; Perilla oil; Perubalsam oil; Parsley leaf oil; Parsley seed oil; Petitgrain oil; Peppermint oil; Pepper oil; Pimento oil; Pine oil; Poley oil; Rose absolute; Rosewood oil; Rose oil; Rosemary oil; Sage oil Dalmatian; Sage oil Spanish; Sandalwood oil; Celery seed oil; Spicy lavender oil; Star anise oil; Styrax oil; Tagetes oil; Fir needle oil; Tea tree oil; Turpentine oil; Thyme oil; Tolu balsam; Tonka absolute; Tuberose absolute; Vanilla extract; Violet leaf absolute; Verbena oil; Vetiver oil; Juniper berry oil; Wine yeast oil; Wormwood oil; Wintergreen oil; Ylang oil; Hyssop oil; Civet absolute; Cinnamon leaf oil; Cinnamon bark oil; and fractions thereof or ingredients isolated therefrom.

Most preferably used in the process according to the invention are fragrance substances or aroma substances selected from the group consisting of: AGRUMEX LC; AGRUNITRIL; ALDEHYD C11 UNDECYLENIC; ALDEHYD C12 LAURIN; ALDEHYD C12 MNA; ALDEHYD C14 SOG; ALDEHYD C16 SOG.; ALLYLAMYLGLYCOLATE; ALLYLCAPRONATE; ALLYLCYCLOHEXYL PROPIONATE; ALLYLHEPTYLATE; AMBROCENIDE® 10 TEC; AMBROCENIDE® Crist. 10% IPM; AMBROXIDE; ANETHOLE NAT. EX STERNANIS; ANISALDEHYDE PURE; APRIFLOREN®; BENZYL ACETONE; BENZYL SALICYLATE; BORNEOL L/ISOBORNEOL 65/35; BUCHU LEAF OIL; ÜCITRONELLOL 950; CLONAL; CYCLOHEXYL SALICYLATE; CYMOL PARA SUPRA; DAMASCONE DELTA; DIHYDROMYRCENOL; DIMETHYLBENZYLCARBINYL BUTYRATE; DYNASCONE; ETHYLENE BRASSYLATE; ETHYL METHYL BUTYRATE-2; ETHYL SAFFRONATE; EUCALYPTOL NAT.; EUCALYPTUS OIL GLOBULUS 80/85%; EUGENOL NAT.; FARENAL®; FENNEL OIL AROMA TYPE SWEET NAT.; FILBERTONE 10% IPM; FILBERTONE; FLOROPAL; GALBASCONE; GERANIOL 60; GLOBANONE®; HEDIONE; HERBAFLORATE; HERBANATE; HERBYL PROPIONATE; HEXENYL ACETATE CIS-3; HEXENYL SALICYLATE CIS-3; HEXYLACETATE; HEXYLACETATE S; HEXYL ISOBUTYRATE; HEXYL SALICYLATE; ISOAMYL BUTYRATE; ISOBORNYL ACETATE; ISOPROPYL METHYL BUTYRATE-2; ISORALDEIN 70; JAVANOL; CAMPHOR DL; CRESOL METHYL ETHER P(CR< 10 PPM); LEMONILE; LIGUSTRAL; LILIAL; LINALOOL; MANZANATE; MELONAL; METHYLHEPTIN CARBONATE; METHYLOCTIN CARBONATE; MUSCENONE; NEOCYCLOCITRAL; NEROLINE BROMELIA; NEROLINE YARA YARA CRYST.; NEROLIONE; NORLIMBANOL; ORANGEOIL; ORIVONE; OZONIL; PATCHOULI OIL; VEGETABLE OIL TRIGLYCERIDE; PHELLANDRENE FRACTION EX EUCALYPTUS OIL; PHENIRAT®; PHENYLETHYL ACETATE; ROSE OXIDE HIGH CIS; SANDRANOL®; STYRENE ACETATE; SULTANENE®; TERPINEN GAMMA; TETRAHYDROLINALOOL; TIMBERSILK; TRIETHYLCITRATE; UNDECAVERTOL; VERTOCITRAL; VERTOFIX; YSAMBER® K and mixtures of the foregoing active agents.

Exemplary cooling agents used as hydrophobic active ingredients in the manufacture of the microcapsules according to the invention include one or more of menthol and menthol derivatives (for example, L-menthol, D-menthol, racemic menthol, isomenthol, neoisomenthol, neomenthol), menthyl ether (for example, (1-menthoxy)-2-propanediol, (1-menthoxy)-2-methyl-1,2-propanediol, 1-menthyl methyl ether), menthyl ester (for example, menthyl formate, menthyl acetate, menthyl isobutyrate, menthyl lactate, L-menthyl-L-lactate, L-menthyl-D-lactate, menthyl-(2-methoxy)-acetate, menthyl-(2-methoxyethoxy)-acetate, menthyl pyroglutamate), menthyl carbonates (for example, menthyl propylene glycol carbonate, menthyl ethylene glycol carbonate, menthyl glycerol carbonate or mixtures thereof), the semiesters of menthol with a dicarboxylic acid or derivatives thereof (for example, monomenthyl succinate, monomenthyl glutarate, monomenthyl malonate, O-menthyl succinate-N,N-(dimethyl) amide, O-menthyl succinamide), menthane carboxamides (for example, menthane carboxylic acid-N-ethylamide [WS3], N-alpha-(methane carbonyl) glycine ethyl ester [WS5], menthane carboxylic acid-N-(4-cyanophenyl)-amide, menthanecarboxylic acid-N-(alkoxyalkyl)amide), menthone and menthone derivatives (for example, L-menthone glycerol ketal), 2,3-dimethyl-2-(2-propyl)-butanoic acid derivatives (for example, 2,3-dimethyl-2-(2-propyl)-butanoic acid-N-methylamide [WS23]), isopulegol or esters thereof (1-(-)-isopulegol, 1-(-)-isopulegol acetate), menthane derivatives (for example, p-menthane-3,8-diol), cubebol or synthetic or natural mixtures containing cubebol, pyrrolidone derivatives of cycloalkyldione derivatives (for example, 3-(methyl)-2-(1-pyrrolidinyl)-2-cyclopenten-1-one) or tetrahydropyrimidin-2-ones (for example, icilin or related compounds as described in WO 2004/026840). Other cooling agents include menthol (L-menthol, D-menthol, racemic menthol, isomenthol, neoisomenthol, neomenthol), L-menthyl methyl ether, menthyl formate, menthyl acetate), menthone, isopulegol, L-(-)-isopulegol acetate) and cubebol, which have a cooling taste effect. Suitable cooling agents are well known in the art and are described, for example, in US 2017/216802 (A1), US 2010/273887 (A1), EP 2 033 688 (A2) and EP 1 958 627 (A2).

In an alternative embodiment, a TRPV1 or a TRPV3 modulator is used as the active ingredient to be encapsulated or as the core material in the polyurea/polyurethane microcapsules according to the invention. TRPV1 and TRPV3 modulators are known in the prior art and refer to TRP channels (Transient Receptor Potential channels) of the vanilloid (TRPV)-subfamily. TRPV1 modulators impart a spicy taste and hot sensation associated with capsaicin and piperine. The TRPV3 protein belongs to the family of nonselective cation channels that function in a variety of processes, including temperature sensation and vasoregulation. The TRPV3 channel is directly activated by several natural compounds such as carvacrol, thymol, and eugenol. Some other monoterpenoids that either cause a sensation of warmth or which are skin sensitizers can also open the channel. Monoterpenoids also induce agonist-specific desensitization of TRPV3 channels in a calcium-independent manner.

In another variant, the polyurea/polyurethane microcapsules according to the invention use, as the active ingredient to be encapsulated or as the core material, an active ingredient selected from the group consisting of substances that cause a pungent taste or a heat or heat sensation on the skin or mucous membranes or a tingling sensation in the mouth or throat are, or active ingredients with a pungent or acrid or astringent effect.

The heat inducing or pungent active ingredients are preferably selected from the group consisting of: Paprika powder, chili pepper powder, extracts of paprika, extracts of bell pepper, extracts of chili pepper, extracts of ginger roots, extracts of grains of paradise (Aframomum melegueta), extracts of paracress (Jambu oleoresin; Spilanthes acmella, resp. Spilanthes oleracea), extracts from Japanese pepper (Zanthoxylum piperitum), extracts from Kaempferia galanga, extracts from Alpinia galanga, extracts from water pepper (Polygonium hydropiper), capsaicinoids, in particular capsaicin, dihydrocapsaicin or nonivamide; gingerols, in particular gingerol-[6], gingerol-[8], or gingerol-[10]; shogaols, in particular shogaol-[6], shogaol-[8], shogaol-[10]; gingerdiones, in particular gingerdione-[6], gingerdione-[8], or gingerdione-[10]; paradols, in particular paradol-[6], paradol-[8], or paradol-[10]; dehydrogingerdiones, in particular dehydrogingerdione-[6], dehydrogingerdione-[8] or dehydrogingerdione-[10]; piperine; piperine derivatives; ethyl-2-(4-hydroxy-3-methoxy-phenyl)acetate and 3-phenylpropyl-2-(4-hydroxy-3-methoxy-phenyl)acetate and mixtures thereof.

The active ingredients perceived as pungent or acrid are preferably selected from the group consisting of: Aromatic isothiocyanates, in particular phenyl ethyl isothiocyanate, allyl isothiocyanate, cyclopropyl isothiocyanate, butyl isothiocyanate, 3-methylthiopropyl isothiocyanate, 4-hydroxybenzyl isothiocyanate, 4-methoxybenzyl isothiocyanate and mixtures thereof.

The active ingredients causing a tingling sensation (tingling) are preferably selected from the group consisting of 2E,4E-decadienoic acid-N-isobutylamide (trans-pellitorin), in particular those as described in WO 2004/043906; 2E,4Z-decadienoic acid-N-isobutylamide (cis-pellitorin), in particular those as described in WO 2004/000787; 2Z,4Z-decadienoic acid-N-isobutylamide; 2Z,4E-decadienoic acid-N-isobutylamide; 2E,4E-decadienoic acid-N-([2S]-2-methylbutyl)amide; 2E,4E-decadienoic acid-N-([2S]-2-methylbutyl)amide; 2E,4E-decadienoic acid-N-([2R]-2-methylbutylamide); 2E,4Z-decadienoic acid-N-(2-methylbutyl)amide; 2E,4E-decadienoic acid-N-piperide (achilleamide); 2E,4E-decadienoic acid-N-piperide (sarmentin); 2E-decenoic acid-N-isobutylamide; 3E-decenoic acid-N-isobutylamide; 3E-nonenoic acid-N-isobutylamide; 2E,6Z,8E-decatrienoic acid-N-isobutylamide (spilanthol); 2E,6Z,8E-decatrienoic acid-N-([2S]-2-methylbutyl)amide (homospilanthol); 2E,6Z,8E-decatrienoic acid-N-([2R]-2-methylbutyl)amide; 2E-decen-4-ynoic acid-N-isobutylamide; 2Z-decen-4-ynoic acid-N-isobutylamide; 2E,6Z,8E,10E-dodecatetraenoic acid-N-(2-methylpropyl)amide (alpha-sanshool); 2E,6Z,8E,10E-dodecatetraenoic acid-N-(2-hydroxy-2-methylpropyl)amide (alpha-hydroxysanshool); 2E,6E,8E,10E-dodecatetraenoic acid-N-(2-hydroxy-2-methylpropyl)amide (gamma-hydroxysanshool); 2E,4E,8Z,10E,12E-tetradecapentaenoic acid-N-(2-hydroxy-2-methylpropyl)amide (gamma-hydroxysanshool); 2E,4E,8E,10E,12E-tetradecapentaenoic acid-N-(2-hydroxy-2-methylpropyl)amide (gamma-hydroxyisosanshool); 2E,4E,8Z,10E,12E-tetradecapentaenoic acid-N-(2-methyl-2-propenyl)amide (gamma-dehydrosanshool); 2E,4E,8Z,10E,12E-tetradecapentaenoic acid-N-(2-methylpropyl)amide (gamma-sanshool); 2E,4E,8Z,11Z-tetradecatetraenoic acid-N-(2-hydroxy-2-methylpropyl)amide (bungeanool); 2E,4E,8Z,11E-Tetradecatetraenoic acid-N-(2-hydroxy-2-methylpropyl)amide (isobungeanool); 2E,4E,8Z-tetradecatrienoic acid-N-(2-hydroxy-2-methylpropyl)amide (dihydrobungeanool) and 2E,4E-tetradecadienoic acid-N-(2-hydroxy-2-methylpropyl)amide (tetrahydrobungeanool) and mixtures thereof.

Active ingredients with astringent activity are preferably selected from the group consisting of: Catechins, in particular epicatechins, gallocatechins, epigallocatechins as well as their respective gallic acid esters, in particular epigallocatechin gallate or epicatechin gallate, their oligomers (procyanidins, proanthocyanidins, prodelphinidins, procyanirins, thearubigenins, theogallins) as well as their C- and O-glycosides; dihydroflavonoids such as dihydromyricetin, taxifolin, as well as their C- and O-glycosides, flavonols such as myricetin, quercetin as well as their C- and O-glycosides such as quercetrin, rutin, gallic acid esters of carbohydrates such as tannin, pentagalloylglucose or their reaction products such as elligatannin, aluminum salts, e.g., alum, and mixtures thereof.

In another variant according to the first and/or second aspect of the present invention, biogenic principles can also be encapsulated as core material, wherein the core material comprises at least one biogenic principle or mixtures thereof.

By biogenic principles are meant active ingredients with biological activity, for example tocopherol, tocopherol acetate, tocopherol palmitate, ascorbic acid, carnotine, carnosine, caffeine, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, as well as vitamin complexes.

In a further variant of the process according to the invention, substances for print coatings for paper are also used as the active ingredient to be encapsulated or as the core material, respectively, as described in US 2800457 A, the disclosure of which in this respect is incorporated by reference in its entirety into the present description.

The internal non-aqueous phase may contain, for example, from 20 to 80 wt.-%, preferably from 25 to 75 wt.-% and even more preferably from 33 to 50 wt.-% of the hydrophobic active ingredient to be encapsulated, from 0.1 to 5 wt.-%, preferably from 0.15 to 3.5 wt.-% and even more preferably from 0.5 to 2.5 wt.-% of first crosslinking agent and, complementarily to 100 wt.-% of a hydrophobic solvent, based on the total weight of the internal non-aqueous phase.

Thus, it is possible to achieve a high loading of active ingredient in the microcapsules according to the invention using the process according to the invention.

In a further step (ii) of the process according to the invention, an external aqueous phase is provided comprising at least one protein and/or at least one polysaccharide and optionally at least one protective colloid.

Suitable solvents for preparing the external aqueous phase are water or mixtures of water with at least one water-miscible organic solvent. Suitable organic solvents include glycerol, 1,2-propanediol, 1,3-propanediol, ethanediol, diethylene glycol, triethylene glycol, and other analogs. Preferably, however, the solvent is water.

According to the present invention, the at least one protein is selected from the group consisting of proteinogenic L-amino acids, animal or plant proteins, in particular protein isolates, animal or plant proteins in the form of fractions, partial or complete hydrolysates or intermediates produced by physicochemical processes or fermentative or enzymatic treatment of the proteins, in particular proteins of the group consisting of: Meat (mammals, birds, reptiles, amphibians, fish), crabs, crustaceans, mussels, mollusks, insects, eggs, milk, especially casein and whey, rennet casein, whey protein concentrate 80%, gelatin, algae, cereals, especially wheat, barley, rye, spelt, gluten, especially wheat gluten, rapeseed, sunflower, rice, potato, corn, soybean, bean, pea, chickpea, lentil, lupin, peanut, alfalfa, hemp, other proteins from edible plants, chitosan, as well as mixtures thereof.

Among the aforementioned proteins, gelatin, milk proteins, whey proteins, chickpea proteins, and pea proteins are particularly preferred.

The amino acids are proteinogenic L-amino acids. The L-amino acids are selected from the group consisting of L-alanine, L-arginine, L-asparagine, L-aspartic acid, L-cysteine, L-glutamine, L-glutamic acid, L-glycine, L-histidine, L-isoleucine, L-leucine, L-lysine, L-methionine, L-phenylalanine, L-proline, L-serine, L-threonine, L-tryptophan, L-tyrosine and L-valine.

Of the abovementioned amino acids, L-glutamine and L-lysine are particularly preferred.

In a preferred variant, one of the abovementioned proteins is used in combination with one or more free L-amino acid(s). Preferred combinations are: Milk protein with L-glutamine and/or L-lysine. With such a combination, both large and small molecules are brought into the network, thus increasing the stability. In addition, the amino acids L-glutamine and/or L-lysine can be crosslinked particularly easily by an enzymatic crosslinker such as transglutaminase.

The abovementioned proteins have the advantageous effect of exhibiting an emulsifying effect. Due to their emulsifying effect, they contribute to the stabilization of the emulsion. Proteins are amphiphilic and thus interfacially active due to their more or less flexible structure and their differently charged regions within the molecule. By modifying the proteins, for example by physical or chemical modification, the secondary and/or tertiary structure of the molecule can be changed. Thus, an influence on the emulsification properties can be achieved by changing the spatial availability of charged regions of the molecule or exposing amino acid side chains. Due to these advantageous properties, an additional emulsifier or a protective colloid can be dispensed with in the process according to the invention.

The proportion of the at least one protein in the external aqueous phase is in a range from 0.25 to 5.0 wt.-%, preferably in a range from 0.5 to 3.0 wt.-%, based on the total weight of the external aqueous phase. Most preferably, the at least one protein is used in a range of 1.0 to 1.5 wt.-%, based on the total weight of the external aqueous phase.

According to a preferred variant of the present invention, chickpea protein is used as the protein component.

According to the first and second aspects of the present invention, the at least one polysaccharide is selected from the group consisting of

- indigestible fibers and dietary fibers, in particular insoluble dietary fibers, in particular cellulose, cellulose derivatives such as hydroxyethyl cellulose, in particular quaternized hydroxyethyl cellulose, carboxymethylcellulose (CMC) and microcrystalline cellulose (MCC), hemicelluloses, lichenin, chitin, chitosan, lignin, xanthan, plant fibers, in particular cereal fibers, potato fibers, apple fibers, citrus fibers, bamboo fibers, extracted sugar beet fibers; oat fibers and soluble dietary fibers, in particular inulin, especially native inulin, highly soluble inulin, granulated inulin, high performance inulin, pectins, alginates, agar, carrageenan, gum arabic (Senegal type, Seyal type), konjac gum, gellan gum, curdlan (paramylon), guar gum, locust bean gum, xanthan gum, raffinose, xylose, polydextrose and lactulose;
- starch, in particular starch from wheat, potatoes, corn, rice, tapioca and oats, modified starch, and starch derivatives, e.g., dextrins or maltodextrins, in particular dextrins and maltodextrins from wheat, potatoes, corn, rice, pea, chickpea and oats, in particular maltodextrins DE8-10, DE17-20, DE18-20, cyclodextrins, oligosaccharides, in particular oligofructose; and
- sugar alcohols, in particular sorbitol, mannitol, isomalt, maltitol, maltilol syrup, lactitol, xylitol, erythritol;
- glucose,

as well as mixtures of two or more of the aforementioned polysaccharides.

Among the aforementioned polysaccharides, gum arabic and maltodextrins are particularly preferred. Most preferred are maltodextrins DE8-10 potato; DE17-20 corn; DE17-20 potato and DE18-20 wheat.

Moreover, dextrins are also preferred, and in particular dextrins from pea starch, especially for the preparation of chickpea-based microcapsules. However, any polysaccharide can be used for the preparation of chickpea-based microcapsules according to the present invention.

The proportion of the at least one polysaccharide in the external aqueous phase is in a range from 0.5 to 7.0 wt.-%, preferably in a range from 1.5 to 6.0 wt.-%, based on the total weight of the external aqueous phase. Most preferably, the at least one polysaccharide is used in a range from 3.0 to 5.0 wt.-%, based on the total weight of the external aqueous phase.

Preferably, the external aqueous phase is provided with both main components of the capsule shell, i.e., at least one protein and at least one polysaccharide. When protein and polysaccharide are used in combination, soluble or insoluble protein-polysaccharide complexes are formed. The emulsion thus formed is less prone to aggregate formation, so that the addition of a protective colloid or an additional emulsifier is not necessary in the process according to the invention.

According to an alternative variant, the external aqueous phase comprises at least one protective colloid and the protein-building block(s) and/or the polysaccharide-building(s) is/are added after emulsification.

For building the capsule wall or capsule shell, the following combinations of protein and polysaccharide are particularly preferred: chickpea protein and dextrin (preferably from pea starch); whey protein and maltodextrin; milk protein and maltodextrin; whey protein and gum arabic; gelatin and maltodextrin; milk protein, L-glutamine, L-lysine and maltodextrin; and gelatin, milk protein and maltodextrin.

Most preferred for building the capsule shell are a combination of gelatin and maltodextrin DE8-10 potato or a combination of milk protein, L-glutamine and/or L-lysine and maltodextrin. Microcapsules prepared with such capsule shell materials result in microcapsules that yield a free oil content in isopropanol of ≤ 1%.

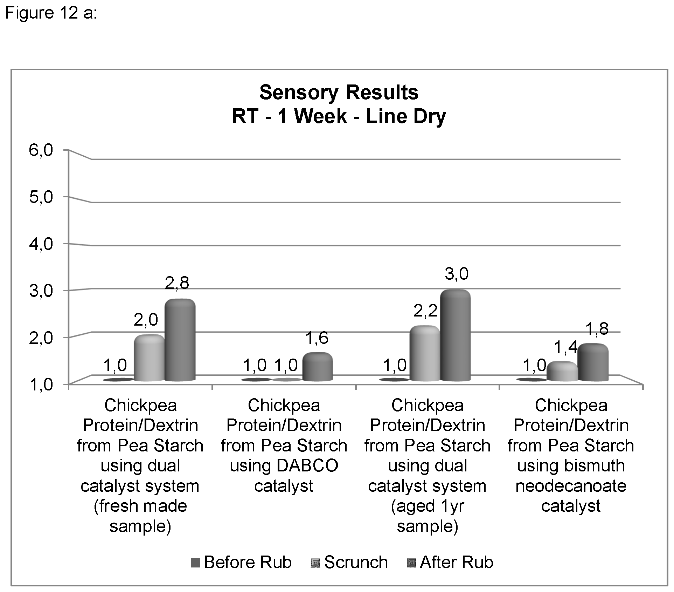

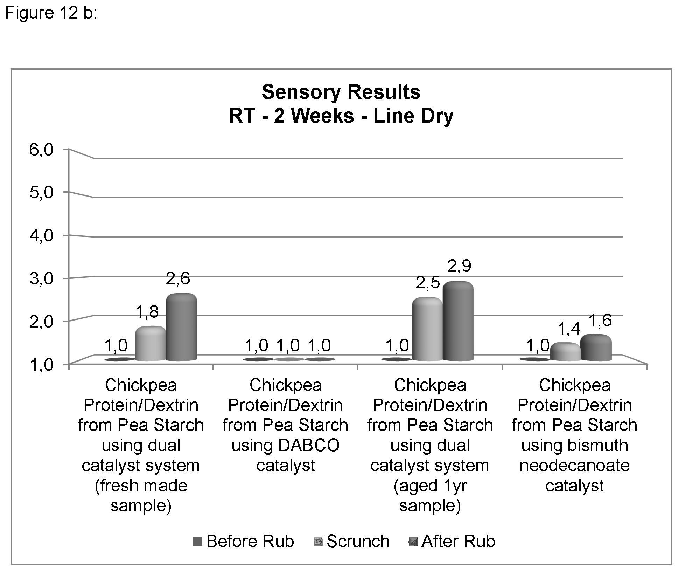

According to another embodiment the capsule wall comprises chickpea proteins as the protein-building blocks and dextrins (preferably from pea starch) as the polysaccharide-building blocks. It was surprisingly found that such microcapsules show excellent encapsulation as well as release properties allowing for the efficient encapsulation and targeted release of the active ingredient(s). In addition, such microcapsules show excellent storage properties with increased shelf life of more than one year without showing any deterioration in product properties such as efficiency of the encapsulation or release properties. The chickpea-dextrin-based microcapsules are able to efficiently encapsulate the active ingredient(s) for a long time and efficiently suppress the leakage/evaporation of volatile active ingredients such as for example fragrance substance (see Figures 12 a and b).

In an alternative variant thereof, maltodextrin is used as the polysaccharide component for the preparation of the microcapsules.

Preferably, for the above mentioned chickpea-based microcapsules a combination of aliphatic and aromatic polyisocyanates is used as the crosslinking agent, even more preferred a combination of aliphatic and aromatic polyisocyanates in a molar ratio of approximately 80 : 20.

Furthermore, in the case of the latter microcapsules, it is preferred that no further crosslinking agents or protein- and/or polysaccharide-components are added.

The previously described and exemplified components for building the capsule wall, proteins and polysaccharides, are readily available from biological sources. Moreover, they are readily biodegradable as such.

In an alternative variant of the process according to the invention, the external aqueous phase is provided with only one of the main components of the capsule shell, protein or polysaccharide. In this variant, the addition of the other main ingredient polysaccharide or protein optionally takes place in process step (iv) after emulsification/dispersion and before or with the addition of the catalyst in process step (v).

By using at least one protein and at least one polysaccharide, the content of the crosslinking agent polyisocyanate in the capsule shell can be reduced, preferably to a content of isocyanate of up to 50 wt.-%, even more preferably to a content of isocyanate of up to 20 wt.-%, based on the capsule shell, compared to the microcapsules of the state of the art, which have a high polyisocyanate content. By replacing polyisocyanate with protein and/or polysaccharide as components for building the capsule wall or capsule shell, the polyisocyanate content in the microcapsule is reduced, resulting in a lower degree of crosslinking.

Surprisingly, however, this lower degree of crosslinking results in stable microcapsules on the one hand and microcapsules with better biodegradability on the other hand, as illustrated in the following embodiments.

A protective colloid may optionally be added to the external aqueous phase.

A protective colloid is a polymer system that prevents clumping (agglomeration, coagulation, flocculation) of the emulsified, suspended, or dispersed components in suspension or dispersion. During solvation, protective colloids bind large amounts of water and generate high viscosities in aqueous solutions, depending on the concentration. In the preparation of oil-in-water emulsions, the protective colloid attaches itself to the primary particles with its hydrophobic part and turns its polar, i.e., hydrophilic, molecular part towards the aqueous phase. By this attachment to the interface, it lowers the interfacial tension and prevents agglomeration of the primary particles. In addition, it stabilizes the emulsion and favors the formation of comparatively smaller droplets and thus also corresponding microcapsules.

In the process according to the invention, the protective colloid also exhibits emulsifying properties in addition to the abovementioned properties. If the emulsifying properties of the protective colloid are sufficient, such as, for example, carboxymethyl cellulose, acid-modified starch, polyvinyl alcohol, ammonium derivatives of polyvinyl alcohol, polystyrene sulfonates, polyvinyl pyrrolidones, polyvinyl acrylates, it is thereby advantageously even possible to dispense with the use of an emulsifier in the downstream emulsifying/dispersing step (iii) in the process according to the invention. For example, in the case of chickpea-based microcapsules preferably polyvinyl alcohol is preferably the only protective colloid which is preferably added to the aqueous phase.

The protective colloid used in the process according to the invention is selected from the group consisting of

- Diols, in particular ethanediol, 1,2-propanediol, 1,3-propanediol, 1,2-butanediol, isomeric butanediol, 1,2-pentanediol, 1,2-hexanediol, 1,2-octanediol, 1,2-decanediol, 1,2-dodecanediol, and
- Polyols, preferably triols, in particular glycerin as well as its ethoxylation and propoxylation products, trimethylolpropane as well as its ethoxylation and propoxylation products, polyvinyl alcohol (PVOH) and its derivatives, in particular ammonium- or sulfonate-functionalized polyvinyl alcohols, polyphenols, preferably 1,3,5-trihydroxybenzene, polysaccharides, in particular glucose, starches or chemically, mechanically and/or enzymatically modified starches, cellulose derivatives such as hydroxyethyl cellulose, in particular quaternized hydroxyethyl cellulose, and carboxymethylcellulose,
- Polyvinylpyrrolidone, maleic acid vinyl copolymers, sodium lignosulfonates, maleic acid anhydride/styrene copolymers, ethylene/maleic acid anhydride copolymers, copolymers of ethylene oxide, propylene oxide and acid esters of polyethoxylated sorbitol, sodium dodecyl sulfate,
- Animal and plant polymers, in particular gum arabic (Senegal type and Seyal type), proteins, gelatin, olibanum resin, shellac, lignin, chitosan, saponin

as well as mixtures of the aforementioned compounds.

Starches, in particular modified starches, or animal or plant polymers are naturally occurring substances which are biodegradable. In combination with the polyisocyanates described herein, the present process can thus provide biobased and biodegradable capsule shells. In the process according to the invention, the starch and the animal and plant polymers therefore also function as so-called bio-crosslinkers.

The starch used in the process according to the invention is selected from the group consisting of corn starch, potato starch, rye starch, wheat starch, barley starch, oat starch, rice starch, pea starch, chickpea starch, tapioca starch, and mixtures thereof.

Preferably, the chemically modified starches are acid-modified starches, alkali-modified starches, oxidized starches, acetylated starches, succinated starches, or ocentylsuccinated starches.

Preferably, the external aqueous phase comprises at least one protective colloid selected from polyvinylpyrrolidones, polyvinyl alcohols, and mixtures thereof. Polyvinylpyrrolidones are particularly preferred. Commercial standard polyvinylpyrrolidones have molecular weights in the range of about 2 500 to 750 000 g/mol.

Even more preferred, polyols, polyphenols or starch, in particular modified starch, is used as the protective colloid. Particularly preferred, polyvinyl alcohol or its ammonium derivatives, 1,3,5-trihydroxybenzene, modified starch or carboxymethyl cellulose is used as protective colloid for the preparation of the microcapsules according to the present invention.

In accordance with the present invention, combinations of two or more different protective colloids may also be used in the preparation of the microcapsule according to the invention.

In the process according to the invention, it has been found to be particularly advantageous if a combination of one of the abovementioned protective colloids with starch as a further protective colloid is used in the external aqueous phase. Such a combination stabilizes the emulsion due to the high number of functional hydroxyl groups and, on the other hand, favors a reaction between the protective colloid and the polyisocyanate(s), whereby the reaction equilibrium of the reaction of the protective colloids with the polyisocyanate(s) is shifted to the side of the products, i.e., the polyurethanes. Furthermore, the large number of functional hydroxyl groups in starch enables the formation of spatially particularly pronounced crosslinks.

Depending on the number of functional groups and/or size of the protective colloid, the abovementioned protective colloids exhibit different reaction rates with the isocyanate groups of the at least one polyisocyanate. For example, glycerin reacts faster with the isocyanate groups than, for example, starch due to its size. Therefore, the crosslinking of the protective colloid with the isocyanate groups of the polyisocyanate can be controlled by the selection of the protective colloid.

A particularly advantageous combination has proven to be glycerin with starch or with modified starch or the combination of glycerin with quaternized hydroxyethyl cellulose or gum arabic type Seyal; with such a combination, one takes advantage of the previously described properties of both protective colloids: high reaction rate of the glycerin on the one hand and number of polymerizable functional groups of the other protective colloid on the other hand.

The protective colloids used in the process according to the invention have a dual function in that, on the one hand, they act as a protective colloid and thus prevent the agglomeration of the emulsified, suspended or dispersed components, stabilize the emulsion subsequently formed, promote the formation of small droplets and stabilize the microcapsule dispersion ultimately formed.

The amount of protective colloid used or the amount of a combination of protective colloids used is thus in a range from 1 to 6 wt.-%, preferably in a range from 2 to 4 wt.-%, even more preferred in a range from 2 to 3 wt.-%, based on the total weight of the external aqueous phase.

The external aqueous phase is preferably prepared under stirring by successively adding the polysaccharide and/or the protein and optionally the protective colloid or vice versa to the external aqueous phase, or by adding the ingredients simultaneously to the external aqueous phase.

To improve the solubility of the protein, the pH-value of the external aqueous phase is optionally adjusted to a pH-value below the isoelectric point of the protein, i.e., to a pH-value which is lower than the isoelectric point of the protein used.

The isoelectric point is the pH-value at which the isoelectric state is reached, i.e., at which the positive and negative charges balance in ampholytes or zwitterions (e.g., amino acids and proteins). This value is a constant characteristic of each amino acid and depends on the pKs-value of the functional group. In addition to amino acids, peptides and proteins also have an isoelectric point. At the isoelectric point, amino acids, and thus also proteins, exhibit the lowest water solubility.

Preferably, the pH-value of the aqueous phase is adjusted to a pH-value in the range of 2.0 to 7.0, even more preferred to a pH-value in the range of 2.0 to 6.0, and most preferred to a slightly acidic pH-value in the range of 3.0 to 5.0, as a function of the isoelectric point of the protein used. Adjusting the pH-value to a pH-value below the isoelectric point, i.e., to a pH-value lower than the isoelectric point, of the protein has the advantage that at such a pH-value the emulsifying properties and the solubility of the protein are highest.

The pH-value of the external aqueous phase is adjusted by adding an organic acid. For this purpose, an organic acid, for example formic acid or acetic acid, is added to the external aqueous phase prior to the emulsification step and a pH-value in the above ranges is adjusted.

The internal non-aqueous phase, which comprises at least one first crosslinking agent and at least one hydrophobic active ingredient, is emulsified or dispersed in the external aqueous phase in a further process step (iii) to form an oil-in-water emulsion/dispersion.

The oil-in-water emulsion is prepared by mixing the internal non-aqueous phase and the external aqueous phase. The weight ratio of internal non-aqueous phase to external aqueous phase is preferably in a range from 70 : 30 to 60 : 40, preferably in a range from 30 : 70 to 60 : 40.

In order to facilitate the formation of an emulsion or dispersion from internal non-aqueous phase and external aqueous phase, to stabilize the formed emulsion or dispersion, respectively, and to prevent segregation of the internal non-aqueous (oily/organic/hydrophobic) phase and the external aqueous (hydrophilic) phase, a stabilizer and/or an emulsifier or emulsification aid is optionally added to the emulsion or dispersion in the process according to the invention.

Preferably, a stabilizer is added to the external aqueous phase which stabilizes the emulsion/dispersion to prevent segregation of the internal non-aqueous (oily) phase and the external aqueous phase.

The preferred stabilizers for preparing the polysaccharide- and protein-based microcapsules according to the present invention are mainly acrylic copolymers that have sulfonate groups. Also suitable are copolymers of acrylamides and acrylic acid, copolymers of alkyl acrylates and N-vinylpyrrolidone, such as LUVISKOL® K15, K30 or K90 (BASF); sodium polycarboxylates, sodium polystyrene sulfonates, vinyl and methyl vinyl ether-maleic acid anhydride copolymers as well as ethylene, isobutylene or styrene-maleic acid anhydride copolymers, microcrystalline cellulose, which is commercially available, for example, under the name VIVAPUR®, diutan gum, xanthan gum or carboxymethyl celluloses.

The amount of stabilizers used may be in the range from 0.01 to 10 wt.-% and preferably in the range from 0.1 to 3 wt.-%, in each case based on the external aqueous phase.

Optionally, an emulsifier, preferably an O/W-emulsifier, is used in the process according to the invention which allows a homogeneous distribution of the oil droplets of the internal non-aqueous phase in the external aqueous phase and stabilizes the emulsion. The similar applies to the mixing of solid, non-soluble active ingredients in the external aqueous phase in order to stabilize the dispersion thus obtained.

The addition of an emulsifier is optionally carried out, in particular when the protein or the protective colloid have no or only low, i.e., insufficient, emulsifying properties. If an emulsifying protein and/or protective colloid is used, the use of an emulsifier can advantageously be dispensed with in the process according to the invention.

Suitable emulsifiers include, for example, non-ionic surfactants from at least one of the following groups:

- Adhesion products of 2 to 30 moles of ethylene oxide and/or 0 to 5 moles of propylene oxide to linear fatty alcohols having 8 to 22 C-atoms, to fatty acids having 12 to 22 C-atoms, to alkylphenols having 8 to 15 C-atoms in the alkyl group as well as alkylamines having 8 to 22-carbon atoms in the alkyl radical;
- Alkyl and/or alkenyl oligoglycosides with 8 to 22 carbon atoms in the alk(en)yl radical and their ethoxylated analogs;
- Addition products of 1 to 15 mol ethylene oxide to castor oil and/or hardened castor oil;
- Addition products of 15 to 60 moles of ethylene oxide to castor oil and/or hardened castor oil;
- Partial esters of glycerin and/or sorbitan with unsaturated, linear or saturated, branched fatty acids having 12 to 22 carbon atoms and/or hydroxycarboxylic acids having 3 to 18 carbon atoms, and adducts thereof with 1 to 30 mol of ethylene oxide;
- Partial esters of polyglycerin (average degree of self-condensation 2 to 8), polyethylene glycol (molecular weight 400 to 5 000), trimethylolpropane, pentaerythritol, sugar alcohols (e.g., sorbitol), alkyl glucosides (e.g., methyl glucoside, butyl glucoside, lauryl glucoside) as well as polyglucosides (e.g., cellulose) with saturated and/or unsaturated, linear or branched fatty acids with 12 to 22 carbon atoms and/or hydroxycarboxylic acids with 3 to 18 carbon atoms as well as their adducts with 1 to 30 mol ethylene oxide, preferably Cremophor®;
- Mixed esters of pentaerythritol, fatty acids, citric acid and fatty alcohol and/or mixed esters of fatty acids containing 6 to 22 carbon atoms, methylglucose and polyols, preferably glycerin or polyglycerin;
- Mono-, di- and trialkyl phosphates as well as mono-, di- and/or tri-PEG-alkyl phosphates and their salts;
- Wool wax alcohols;
- Polysiloxane-polyalkyl-polyether copolymers or corresponding derivatives;
- Block copolymers e.g., polyethylene glycol-30 dipolyhydroxystearates; Polymer emulsifiers, e.g., pemulene grades (TR-1, TR-2) from Goodrich or Cosmedia® SP from Cognis;
- Polyalkylene glycols as well as glycerin carbonate.

Typical anionic emulsifiers that can be used in the process according to the invention for the preparation of the isocyanate-based microcapsules are aliphatic fatty acids with 12 to 22 carbon atoms, such as palmitic acid, stearic acid or behenic acid, as well as dicarboxylic acids with 12 to 22 carbon atoms, such as azelaic acid or sebacic acid.

Furthermore, zwitterionic surfactants can be used as emulsifiers in the process according to the invention for the preparation of the polysaccharide- and protein-based microcapsules. Zwitterionic surfactants are surface-active compounds that bear at least one quaternary ammonium group and at least one carboxylate and one sulfonate group in the molecule. Particularly suitable zwitterionic surfactants are the so-called betaines such as the N-alkyl-N,N-dimethylammonium glycinates, for example the cocoalkyl dimethylammonium glycinate, N-acylaminopropyl-N,N-dimethylammonium glycinate, for example, the cocoacylaminopropyldimethylammonium glycinate, and 2-alkyl-3-carboxylmethyl-3-hydroxyethylimidazolines each having 8 to 18 C-atoms in the alkyl or acyl group, as well as the cocoacylaminoethylhydroxyethyl carboxymethylglycinate. Particularly preferred is the fatty acid amide derivative known under the CTFA name cocamidopropyl betaine.

Also suitable emulsifiers are ampholytic surfactants. By ampholytic surfactants are meant those surface-active compounds which, in addition to a C8/18 alkyl or acyl group in the molecule, contain at least one free amino group and at least one -COOH or -SO$_3$H group and are capable of forming internal salts. Examples of suitable ampholytic surfactants are N-alkylglycines, N-alkylpropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids each having about 8 to 18 C-atoms in the alkyl group. Particularly preferred ampholytic surfactants are N-cocosalkylaminopropionate, cocosacylaminoethyl aminopropionate and C12/18 acyl sarcosine.

Finally, cationic surfactants may also be employed as emulsifiers, with those of the esterquat type, preferably methyl-quaternized difatty acid triethanolamine ester salts, quaternized hydroxyethylcellulose, modified chitosan with propylene glycol and quaternized with epichlorohydrin, distearyldimethylammonium chloride (DSDMAC), benzalkonium chloride, benzethonium chloride, cetylalkonium chloride, cetylpyridinium chloride, cetyltrimethylammonium bromide (cetrimonium bromide), dequalinium chloride being particularly preferred.

The emulsifiers may be added to the external aqueous phase in an amount of from about 0.5 to about 10 wt.-%, and preferably from about 1 to about 5 wt.-%, each based on the total weight of the external aqueous phase.

The emulsion formation (in the case of liquid active ingredients) or dispersion formation (in the case of solid active ingredients), i.e., the emulsification or dispersion of the internal non-aqueous or oily phase with the external aqueous or hydrophilic phase, is carried out under high turbulence or high shear, whereby the strength of the turbulence or shear determines the diameter of the microcapsules obtained. The preparation of the microcapsules can be carried out continuously or discontinuously. As the viscosity of the aqueous phase increases or the viscosity of the oily phase decreases, the size of the capsules generally decreases.

The process according to the invention for the preparation of the polysaccharide- and protein-based microcapsules can be carried out, for example, according to the "inline"-technique. Thereby, by means of a forced metering pump, the internal non-aqueous phase and the external aqueous phase are first fed separately to an emulsification turbine, combined shortly before entering the emulsification turbine, or combined in the emulsification turbine, at a throughput volume of 1 200 to 1 500 l/h. In addition, the process according to the invention for the preparation of the polysaccharide- and protein-based microcapsules can also be carried out in conventional dispersion apparatus or emulsification apparatus.

The emulsification or dispersion of the external aqueous phase and the internal non-aqueous phase is carried out for the preparation of the microcapsules according to the invention, for example, by means of an emulsification turbine (IKA Eurostar 20 high-speed stirrer).

The process of emulsification or dispersion in the process according to the invention is advantageously carried out for a time of from 30 seconds to 20 minutes, preferably from 1 to 4 minutes and most preferred from 1 to 2.5 minutes, at a stirring speed of from 1 000 rpm to 5 000 rpm, preferably at 3 000 rpm to 4 000 rpm until a capsule size of from 10 to 50 $\mu$m $\pm$ 5 $\mu$m has been obtained.

After completion of the emulsification or dispersion step (iii), an oil-in-water emulsion or dispersion is present in which the internal oily phase with the active ingredients to be encapsulated is finely emulsified or dispersed in the external aqueous phase in the form of droplets.

In an alternative variant of the process according to the invention, after the emulsification or dispersion step (iii), the addition of at least one polysaccharide or at least one protein is optionally carried out in a process step (iv), as described above. If the external aqueous phase is provided in process step (ii) with only at least one protein main component, the addition of at least one polysaccharide takes place in process step (iv). If, on the other hand, the external aqueous phase in process step (ii) is provided only with at least one polysaccharide main component, the addition of at least one protein takes place in process step (iv). The separate addition results in the formation of multiple layers ("layer-by-layer"), the individual layers of which are crosslinked with each other in the subsequent process step (v). Thereby, for example, the charge of the emulsion and thus the aggregation stability can be controlled.

Alternatively thereto, a further protein and/or polysaccharide can optionally be added in process step (iv), which is the same as or different from the at least one protein and/or at least one polysaccharide from process step (ii) or has a different charge or changes the charge when the pH-value changes. By adding another protein and/or polysaccharide, further layers ("layer-by-layer") are built up, the individual layers of which are crosslinked with each other in the subsequent process step (v). This leads to a denser and more stable network of the capsule wall components and consequently to more stable capsule shells, hereby increasing the stability of the microcapsule.

The further protein and/or further polysaccharide is selected from the group protein and/or polysaccharide as already defined in detail above for process step (ii). The same also applies with respect to the preferred variants or preferred combinations of the protein and/or polysaccharide described therein.

In a subsequent process step (v) of the process according to the invention, likewise under stirring, a first crosslinking of the material of the capsule shell or capsule wall is carried out.

The first crosslinking takes place after emulsification or dispersion by adding a catalyst in order to crosslink the layers ("layer-by-layer) described above of capsule wall components and to stabilize the resulting capsule shell. Thereby, catalyzed polymerization reactions between the carboxyl group and/or sulfo group and/or hydroxyl group of polysaccharide and the amino group of protein on the one hand, and the isocyanate group of the first crosslinking agent on the other hand, take place by interfacial polymerization at the interface between the outer aqueous phase and the disperse inner phase, i.e., at the interface of the emulsified or dispersed oil droplets enclosing the active ingredient to be encapsulated. Comparably, if the catalyst is already added to the internal non-aqueous phase, the crosslinking likewise takes place after emulsification or dispersion of the non-aqueous and aqueous phases.

Due to the catalyzed crosslinking between the functional groups of the at least one polysaccharide and/or the at least one protein of the previously described layers and the functional groups of the first crosslinking agent, first crosslinking units or a first crosslinking matrix for the construction of a capsule shell or capsule wall are formed.

The formation of the first crosslinking units in the process according to the invention is based on the polyaddition reaction between polysaccharide and first crosslinking agent and/or protein and first crosslinking agent. Thereby, the hydroxyl groups of the polysaccharide react with the isocyanate groups of the first crosslinking agent to form polyurethane, and the amino groups of the protein react with the isocyanate groups of the first crosslinking agent to form polyurea. In addition to polyurethane and polyurea, soluble or insoluble complexes of protein and polysaccharide are also formed during the first crosslinking step (v) to constitute the capsule wall matrix or capsule shell.

The higher the number of crosslinking functional groups of the capsule wall building blocks, the greater the spatial crosslinking and the denser and more stable the resulting capsule shell or capsule wall of the microcapsule. In addition to the number of functional groups, the chain length of the individual capsule wall building blocks also has a significant influence on the mechanical properties, i.e., the stability, of the microcapsules: for example, the large number of hydroxyl groups in starch enables the formation of particularly pronounced spatial crosslinking; longer-chained capsule wall building blocks, for example polyisocyanates, cause to the formation of more stable capsule walls.

Due to the formation of the first crosslinking matrix or first crosslinking units, the emulsified or dispersed oil droplets with the core material, i.e., the encapsulated active ingredients, are enclosed at the interface by the crosslinking matrix or the crosslinking units on the outside, thus generating a capsule wall, which makes diffusion of the encapsulated active ingredient more difficult.

The addition of the at least one catalyst to the emulsion or dispersion accelerates the crosslinking reaction between the polysaccharide and/or protein and the crosslinking agent, and catalyzes the reaction in favor of the formation of a first crosslinking matrix or first crosslinking units.

The catalyst added in the process according to the invention is preferably diazabicyclo[2.2.2]octane (DABCO), also known as triethylenediamine (TEDA), a bicyclic tertiary amine. DABCO is generally used as a catalyst for the preparation of polyurethane plastics. The tertiary amine with free electron pairs promotes the reaction between the isocyanate groups of the first crosslinking agent and the hydroxyl groups of the polysaccharide.

In addition to DABCO, for example, catalysts based on bismuth or tin are also used to catalyze the first crosslinking, such as for example, catalysts based on bismuth(II) salts or bismuth(III) salts, as described in K. C. Frisch & L.P. Rumao, Catalysis in Isocyanate Reactions, Polymer Reviews, 1970, 5:1, pages 103 - 149, DOI: 10.1080/15583727008085365, the disclosure of which in this respect is incorporated in its entirety in the present description.

According to the invention, a combination of DABCO and one of the abovementioned catalysts is preferred. Such a mixture results in a multiplication of reactivity as described in K.C. Frisch & L.P. Rumao, Catalysis in Isocyanate Reactions, Polymer Reviews, 1970, 5:1, pages 103 - 149, DOI: 10.1080/15583727008085365, the disclosure of which in this respect is incorporated in its entirety in the present description.

It was surprisingly found that, in particular, the combination of DABCO and a bismuth catalyst such as bismuth neodecanoate is preferred and results in highly stable and excellently performing microcapsules. For this combination of catalysts, it is preferred that the bismuth catalyst is added to the internal non-aqueous phase and that the DABCO catalyst is added to the aqueous phase before emulsification or alternatively to the mixture after emulsification of the non-aqueous phase and the aqueous phase.

DABCO and the aforementioned catalysts preferably catalyze the polyurethane reaction between the at least one polymerizable polyisocyanate having two or more isocyanate groups and the diols or polyols in the process according to the invention.

The amount in which the catalyst is added to the external aqueous phase is in a range from 0.001 to 1 wt.-%, preferably in a range from 0.02 to 0.75 wt.-%, and particularly preferred in a range from 0.05 to 0.5 wt.-%, based on the total weight of the external aqueous phase. However, the catalyst can also be increased in the case of sluggish polymerization.

The catalyst is added to the emulsion or dispersion either as such, for example as a solid, or in the form of an aqueous solution, preferably in water, under stirring. The catalyst is present in the aqueous solution in a concentration of 0.5 to 2 mol/l, preferably of 1 mol/l.

The catalyst is added at a stirring speed of from 500 rpm to 2 000 rpm, preferably from 1 000 rpm to 1 500 rpm, and at a temperature in the range of from 20 °C to 30 °C., preferably at temperatures of from 22 °C to 26 °C.

Even more preferred, the process step (v) of catalyzed first crosslinking is carried out by gradually heating the emulsion or dispersion to a temperature in the range of 60 °C to 90 °C, preferably to a temperature in the range of 65 to 85 °C, most preferably to a temperature in the range of 70 to 80 °C. The first crosslinking in the process according to the invention is carried out for a duration of from about 30 minutes to 90 minutes, preferably for a duration of from 40 minutes to 70 minutes and most preferably for a duration of 60 minutes.

After the initial crosslinking and formation of the capsule shell or capsule wall, the capsules prepared according to the process according to the invention are present as crude microcapsules in the form of an aqueous dispersion or slurry.

After crosslinking, the microcapsules in the slurry still have a flexible shell, which does not have any particular stability and therefore breaks open easily. For this purpose, the shell is cured. The curing in process step (vi) is preferably carried out by gradually raising the microcapsule slurry to a temperature of at least 60 °C, preferably to a temperature in the range of 60 °C to 65 °C, up to maximally the boiling point of the microcapsule slurry. The curing is typically carried out for a duration of at least 3 hours, preferably for a duration of 4 hours, and most preferably for a duration of 5 hours. Alternatively, e.g., in the case of chickpea protein-based microcapsules it is beneficial to cure the microcapsules at a temperature of 70 °C for one hour, followed by an increase of the temperature to preferably 80 °C (within approximately half an hour) and preferably curing for another hour at 80 °C.

It is additionally advantageous to add substances to the microcapsule slurry for hardening. For this purpose, natural plant-based tanning agents of the tannin type are used, which, from a chemical point of view, are proanthocyanidins as found in dicotyledonous perennials, shrubs, and leaves, especially in the tropics and subtropics. The terpenes generally have molecular weights in the range of 500 to 3 000 KDa. A preferred example of a suitable tannin is corigallin. For curing, an aqueous preparation of the tannins is added to the aqueous dispersion containing the crude microcapsules. Typically, the tannins are added in amounts of from about 0.1 to about 2 wt.-% and preferably from about 0.5 to about 1.5 wt.-%, based on the microcapsules.

In order to optimize the crosslinking of the capsule wall matrix, in an alternative variant of the process according to the invention, optionally a further protein and/or further polysaccharide can be added to the microcapsule slurry in process step (vi).

The further protein and/or further polysaccharide is selected from the group protein and/or polysaccharide as already defined in detail above for process step (ii). The same definitions and preferred embodiments and/or preferred combinations as for the protein and/or polysaccharide are also fully valid for the further protein and/or polysaccharide.

The further protein and/or the further polysaccharide may be the same as or different from the protein and/or polysaccharide of process step (ii). Preferably, the further protein and/or the further polysaccharide is different from the protein and/or polysaccharide of process step (ii).

The addition of a further protein and/or further polysaccharide leads to a further crosslinking with the first crosslinking agent and contributes to the formation of a particularly dense and stable network of the capsule wall building blocks.

The curing step (vi) of the process according to the invention is followed by a step of cooling the microcapsule slurry to room temperature and optionally a second crosslinking step of the capsule wall building blocks by adding a second crosslinking agent.

As a second crosslinking agent for the second crosslinking step, the process according to the invention uses at least one crosslinking agent selected from the group consisting of transglutaminase, peroxidase, secondary plant compounds selected from the group, which consists of polyphenols, in particular tannin, gallic acid, ferulic acid, hesperidin, cinnamaldehyde, vanillin, carvacrol, and mixtures of two or more of the aforementioned crosslinking agents, as already described above in the context of the first and further crosslinking agents. The same definitions and preferred embodiments as for the first and further crosslinking agents are also fully valid for the second crosslinking agent.

Particularly preferred of the foregoing further crosslinking agents are cinnamaldehyde, tannin and gallic acid.

In a preferred variant of the process according to the invention, the second crosslinking agent is different from the first and further crosslinking agents in process step (i).

The content of the second crosslinking agent is in a range from 0.1 to 5 wt.-%, preferably in a range from 0.15 to 2.5 wt.-%, based on the total weight of the non-aqueous phase. Most preferably, the second crosslinking agent is used in the internal non-aqueous phase in a range of 0.5 to 1 wt.-%, based on the total weight of the non-aqueous phase.

The second crosslinking agent is added to the emulsion or dispersion either as such, for example as a solid, or in the form of an aqueous solution.

The second crosslinking agent is present in the aqueous solution in a concentration of 0.01 to 2 mol/l, preferably in a concentration of 0.1 to 1.5 mol/l, most preferably in a concentration of 0.5 to 1.0 mol/l. The solution has a pH-value of 7 to 14, preferably a pH-value of 12.

Even more preferably, the second crosslinking in process step (vii) is carried out by gradually heating the emulsion or dispersion to a temperature in the range of 20 °C to 50 °C, preferably to a temperature in the range of 30 to 40 °C. The second crosslinking in the process according to the invention is carried out for a duration of about 20 minutes to 10 hours, preferably for a duration of 30 minutes to 8 hours.

To optimize the first crosslinking in process step (v) and/or the second crosslinking in process step (vii) of the process according to the invention, optionally the pH-value of the emulsion or dispersion is adjusted to a pH-value above or below the isoelectric point of the protein used. At a pH-value below the isoelectric point, the net electrostatic charge of a protein is positive; above the isoelectric point, the net charge of a protein is negative.

Preferably, the pH-value is adjusted to a pH-value in the range of pH 2.0 to pH 4.0, more preferably to a pH-value in the range of pH 2.5 to pH 3.5, and most preferably to a pH-value of pH 3.0 to obtain a positive charge of the protein. To obtain a negative charge of the protein, the pH-value is preferably adjusted to a pH-value in the range of pH 8.0 to pH 12.0, even more preferably to a pH-value in the range of pH 9.0 to pH 10.0 and most preferably to a pH-value of 9.5.

For this purpose, an organic acid, for example formic acid or acetic acid, or a base, for example sodium hydroxide solution, are added to the emulsion or dispersion and a pH-value in the abovementioned ranges is adjusted.

Carrying out the first crosslinking and/or the second crosslinking at a pH-value above or below the isoelectric point has the advantage that the electrical charge of the protein is modified, and thus electrostatic interactions can positively influence the capsule formation. In addition, such modification of the proteins positively influences their emulsifying ability.

During the first and second crosslinking steps, the stirring power is reduced, for example to a stirring speed of about 800 to 1200 rpm, in order not to break the forming microcapsules again immediately.

An important criterion for the usability of microcapsules is the weight ratio of core material to capsule wall material. While on the one hand the highest possible proportion of core material is desired to enable the capsules to have the highest possible utility value, on the other hand it is necessary for the capsule to still have a sufficient proportion of capsule wall material to ensure the stability of the capsules.

According to the invention, it was found to be particularly advantageous that the microcapsules are designed in such a way that the microcapsules have a weight ratio of core material to capsule wall material which is from 50 : 50 to 90 : 10, preferably from 70 : 30 to 80 : 20.

After complete curing, the microcapsules prepared according to the process of the invention are present as a dispersion in water, which is also referred to as a microcapsule dispersion or microcapsule slurry. In this form, the microcapsules are in principle already ready for sale.

In order to prevent segregation or creaming of such a suspension and thus to achieve high storage stability, it has proven advantageous for the suspension to have a viscosity of 12 to 1 500 mPas. In order to obtain the desired viscosity of the suspension, a thickening agent is preferably used.

As thickening agents preferably xanthan gum, diutan gum; carboxymethyl cellulose (CMC), microcrystalline cellulose (MCC) or guar gum are used.

To improve the shelf life, optionally one or more preservatives are added to the microcapsule slurry, or the microcapsule slurry is dried.

As preservatives preferably 1,2-hexanediol, 1,2-octanediol, phenoxyethanol-based products, products from mixtures of 1,2-benzisothiazolin-3-one (2.5%) and 2-methyl-4-isothiazolin-3-one (2.5%), or the like are used.

Alternatively, and for preservation purposes, the microcapsule slurry is preferably dried.

For drying of the microcapsule slurry, processes such as lyophilization com into consideration, however, referred is spray drying, for example in a fluidized bed. Thereby, it has proven advantageous to add further polysaccharides, preferably dextrins and in particular maltodextrins, to the suspension at temperatures of about 20 to about 50 °C and preferably about 40 °C, which support the drying process and protect the capsules during this process. Thereby, the amount of polysaccharides used can be about 50 to about 150 wt.-% and preferably about 80 to about 120 wt.-% based on the capsule mass in the dispersion.

The spray drying itself can be carried out continuously or batchwise in conventional spray plants, wherein the inlet temperature is about 170 to about 200 °C and preferably about 180 to 185 °C and an outlet temperature is about 70 to about 80 °C and preferably about 72 to 78 °C.

Due to the catalyzed crosslinking of polysaccharide and/or protein with the first crosslinking agent and optionally second crosslinking agent, large molecules are introduced into the network of the capsule shell, which increase the amount of natural components in the capsule shell or microcapsule slurry and thus increase the biodegradability of the capsule shell, as illustrated in the following embodiments.

The process according to the invention is further characterized in that, as main components, protein and polysaccharide and polyisocyanates are polymerized and/or crosslinked via specifically catalyzed mechanisms, thus enabling the preparation of biobased and biodegradable microcapsules based on biocompatible polymers. Unlike state-of-the-art microcapsules, where polyisocyanates make up a large portion of the capsule shell material, the exact opposite is the case here: The polyisocyanates no longer function as the main material in the microcapsules according to the invention, but serve exclusively as crosslinking agents for the amino acids and the other components mentioned above.

The process according to the invention thus allows to replace part of the polyisocyanate by biodegradable wall materials such as proteins and/or polysaccharides and thus to lower the polyisocyanate content without causing any loss or degradation in the functionality of the microcapsules, such as olfactory properties and positive secondary properties such as high stability, namely the ability to retain the active ingredient. Thus, microcapsules can be prepared by the process according to the invention that, on the one hand, have excellent functionality and, at the same time, are readily biodegradable.

Surprisingly, it has been found that with the process according to the invention, microcapsules can be prepared with a reduced amount of starting substance isocyanate by up to 25%, preferably by up to 50%, even more preferably by up to 75%, or other microcapsule starting substances of the state of the art, while maintaining the same amount of active ingredient to be encapsulated, without causing any loss or degradation of the stability of the microcapsules obtained, as shown in the following embodiments.

In a second aspect, the present invention relates to a microcapsule or microcapsule slurry prepared according to the process of the invention.

The biodegradable protein- and/or polysaccharide-based microcapsules are characterized in that they are composed of or comprise:

(a) a core comprising or consisting of at least one hydrophobic active ingredient;
(b) a capsule shell comprising or consisting of a crosslinking matrix or crosslinking units of at least one polysaccharide and/or at least one protein and at least one first crosslinking agent; and optionally a protective colloid and/or optionally a second crosslinking agent.

The microcapsule according to the invention comprises a core surrounded or encased by the capsule shell or capsule wall. As a core material for the preparation of the microcapsules according to the invention, any material suitable for encapsulation in microcapsules may be used. Preferably, hydrophobic, water-insoluble or water-immiscible liquids or solids as well as suspensions are considered as materials to be encapsulated.

In the context of the present description, the core material is a hydrophobic active ingredient, i.e., a substance that has a specific effect or causes a specific reaction, for example, a drug, a pesticide, a cosmetic active ingredient, a food active ingredient, etc., as described above. The term "hydrophobic active ingredient" means that the active ingredient to be encapsulated is in the internal non-aqueous phase during the preparation of the microcapsule and does not mix with the external aqueous phase.

Polymerization and/or crosslinking of functional groups of protein and/or polysaccharide with polyisocyanate results in a stable capsule wall of alternating and dense and thus stable crosslinking matrices or crosslinking units based on polyurea and polyurethane, as well as soluble or insoluble complexes of protein and polysaccharide.

In a preferred embodiment, the capsule shell comprises or consists of: a crosslinking matrix or crosslinking units from a polymerization and/or crosslinking of at least one protein with the first and optionally the second crosslinking agent and/or a crosslinking matrix or crosslinking units from a polymerization and/or crosslinking of at least one polysaccharide, with the first and optionally the second crosslinking agent.

The crosslinking matrix or crosslinking units from a polymerization and/or crosslinking of at least one protein with the first and optionally the second crosslinking agent is predominantly a polyurea-based network, and the crosslinking matrix or crosslinking units from a polymerization and/or crosslinking of at least one polysaccharide with the first and optionally the second crosslinking agent is predominantly a polyurethane-based network as well as soluble or insoluble complexes of protein and polysaccharide.

In addition to the polyurea formation and/or polyurethane formation described above, by-products are formed during the previously described crosslinking steps due to the reactivity of the polyisocyanates, for example, urea, allophanate, biuret, uretidione, carbodiimide, uretonimine, etc., as described in M.F. Sonnenschein, Introduction to Polyurethane Chemistry, Polyurethanes: Science, Technology, Markets, and Trends, First Edition, 2015, John Wiley & Sons, pages 105 to 126, the respective disclosure of which is incorporated herein in its entirety. These by-products are part of the capsule shell or capsule wall, respectively.

By building up the capsule wall based on several individual defined and alternating crosslinking matrices or crosslinking units, it is possible to prepare particularly stable microcapsules with excellent sensory performance, while at the same time significantly reducing the shell constituents.

In addition to the main ingredients listed above, the capsule shell may optionally comprise a protective colloid and/or optionally a further crosslinking agent.

In a preferred variant according to the second aspect, the microcapsules according to the invention are in the form of a dispersion or slurry in which the microcapsules are dispersed in the external aqueous phase. The weight percentage of the microcapsules in the dispersion or slurry is about 20 to 60 wt.-%, in particular about 25 to 50 wt.-%, more preferably about 30 to 35 wt.-%.

The microcapsules prepared according to the process according to the invention can be characterized by the d(0.5)-value of their size distribution: 50% of the capsules are larger, 50% of the capsules are smaller than this value.

To determine the particle size distribution, the microcapsules according to the invention with different compositions were dispersed in water as part of a dynamic process and then the particle size was determined by laser diffraction. Depending on the size of the capsule, the laser beam is refracted differently and can thus be converted to a size. Mie theory was used for this purpose. A MALVERN Mastersizer 3000 was used for the particle measurement. The corresponding calculation is based on the Mie theory.

The microcapsules according to the invention are characterized by having a particle size distribution at a d(0.5)-value of 18 to 50 $\mu$m, preferably a d(0.5)-value of 22 to 30 $\mu$m.

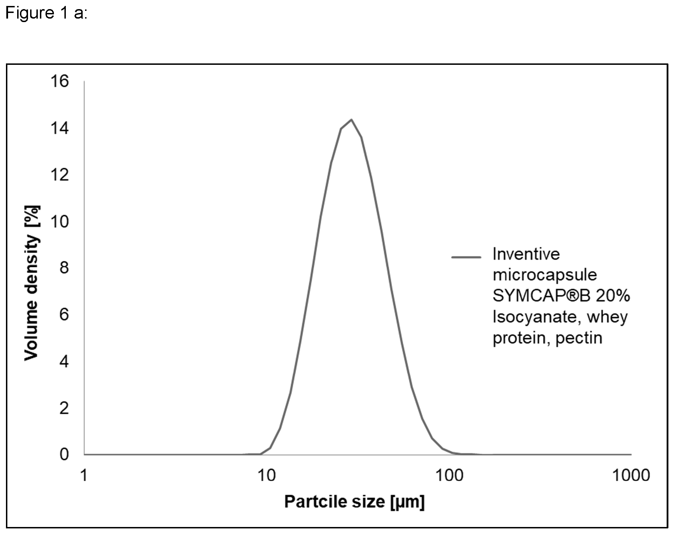

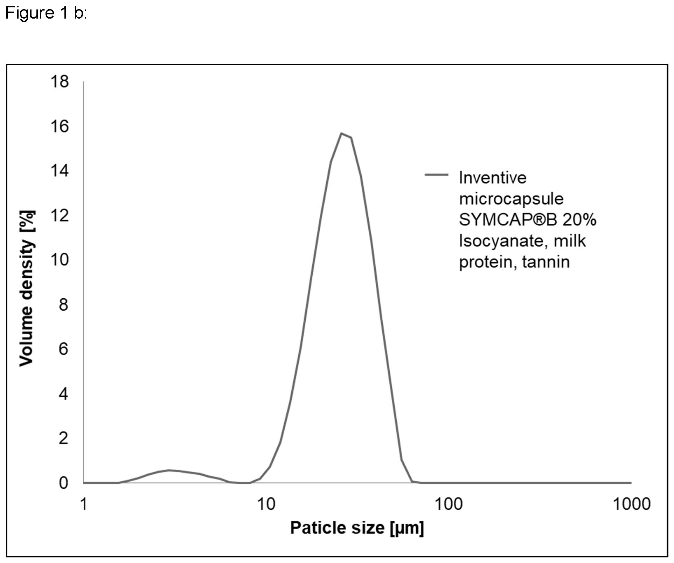

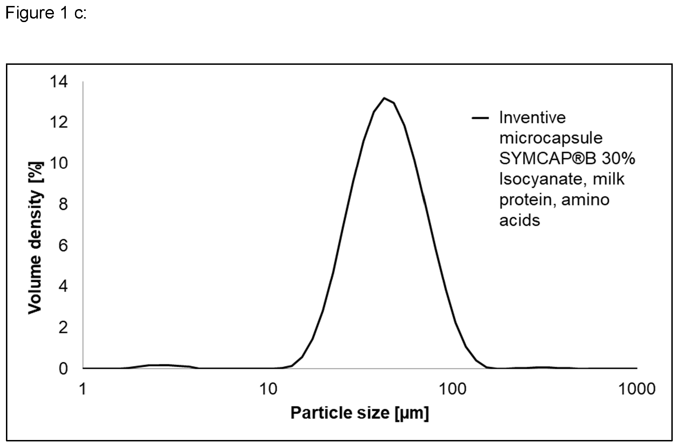

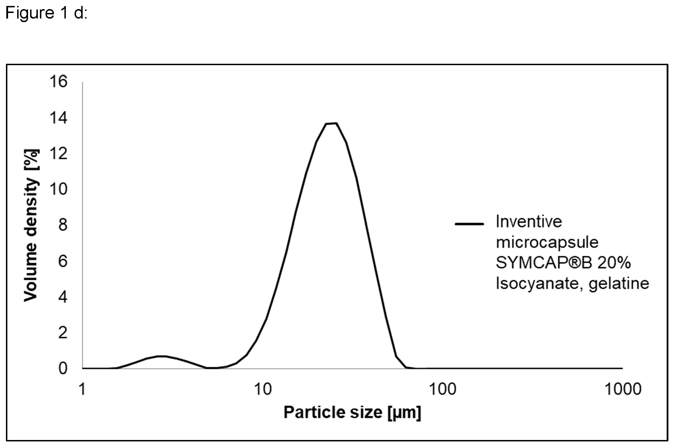

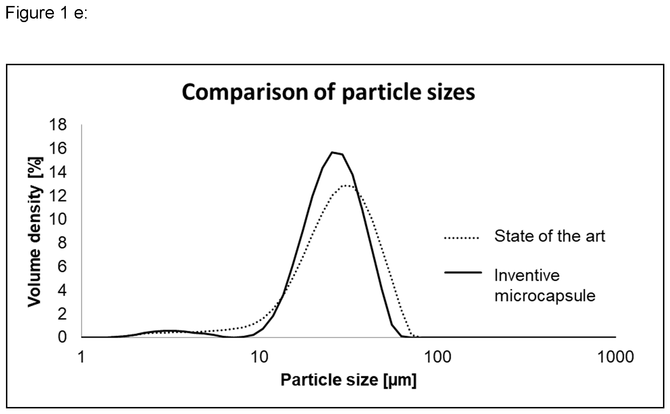

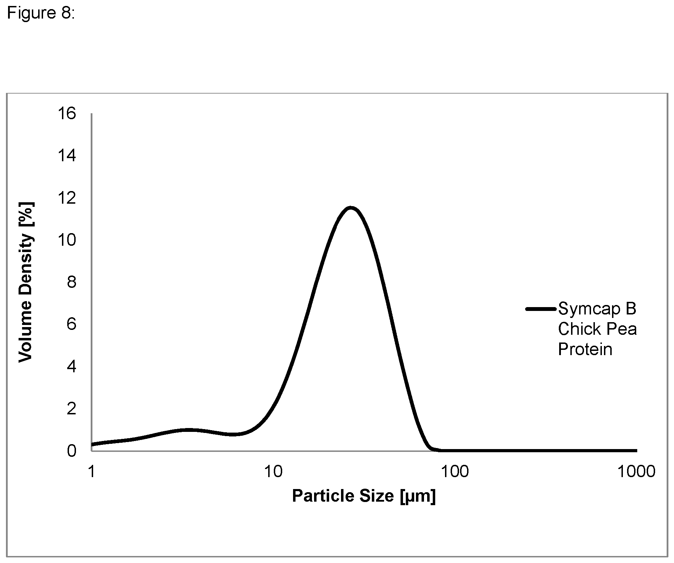

The corresponding particle size distributions of the microcapsules according to the invention are illustrated in Figures 1 a to 1 d, as well as Figure 8:

Figure 1 a: Symcap B: 20% isocyanate content; whey protein/pectin and maltodextrin; microcapsule according to the present invention;

Figure 1 b: Symcap B: 20% isocyanate content; milk protein and maltodextrin, additional crosslinker tannin; microcapsule according to the present invention;

Figure 1 c: Symcap B: 30% isocyanate content; milk protein, L-glutamine, L-lysine and maltodextrin; microcapsule according to the present invention;

Figure 1 d: Symcap B: 20% isocyanate content; gelatin and maltodextrin; microcapsule according to the present invention.

Figure 1 e: Comparison of particle size distribution: microcapsule according to the state of the art, Symcap G 2.1: 100% isocyanates, polyvinyl alcohol and guanidinium carbonate; microcapsule according to the present invention, Symcap B: 20% isocyanates, milk protein, tannin.

Figure 8: Symcap B: 75% isocyanate content; chickpea protein and dextrin from pea starch; microcapsule according to the present invention.

The direct comparison of the microcapsules shows that the process according to the invention can achieve microcapsules with the same particle size distribution as microcapsules of the state of the art.

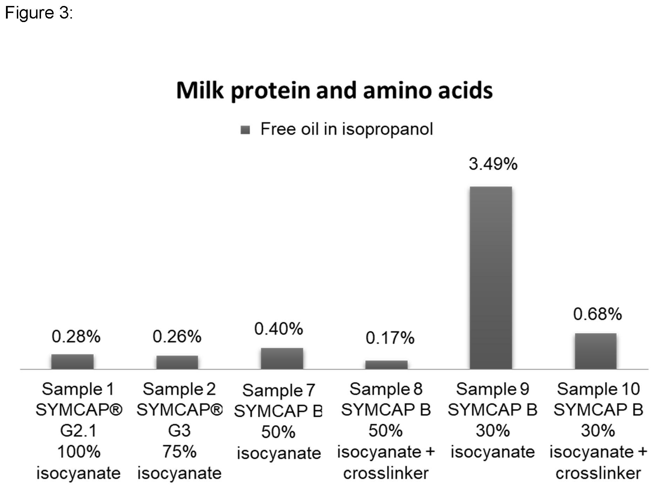

Surprisingly, despite a reduction in the polyisocyanate content in the microcapsule wall, the protein- and/or polysaccharide-based microcapsule prepared according to the process of the invention exhibits comparable stability and content of unintentionally leaking perfume oil as microcapsules according to the state of the art, as shown in Figures 2 and 3 as well as Figure 9. Microcapsules according to the invention prepared with an additional second crosslinking agent exhibit comparable, if not better, stability and thus a lower content of free oil compared to microcapsules of the state of the art, as shown in Figures 2 and 3, which can be attributed in particular to a more efficient encapsulation of the odorant substances.

In particular, the use of a further crosslinking agent, in particular with a reduction in the isocyanate content, leads to a significant improvement in stability and thus to a low proportion of leaking perfume oil (see Samples 7 and 8 and Samples 3 and 4, 5 and 6 in Figures 2 and 3).

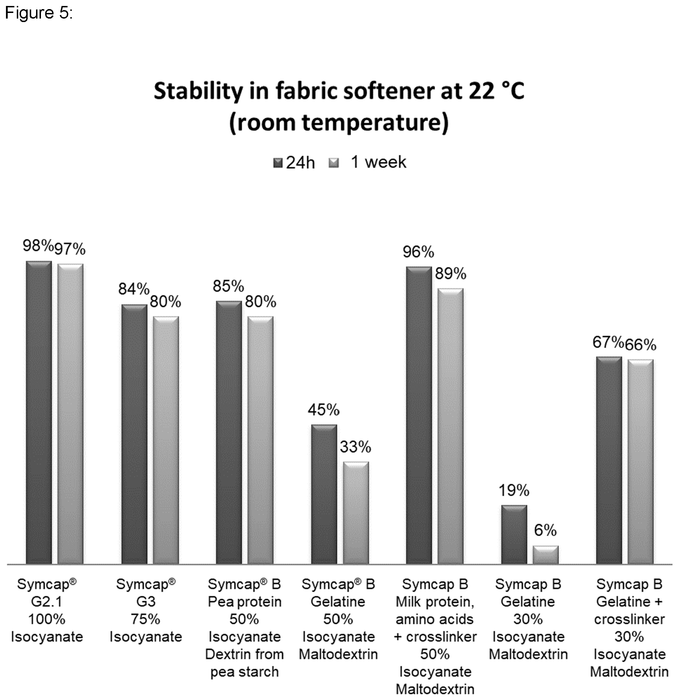

The use of the microcapsules, in particular microcapsules prepared with a further crosslinking agent, in the target application also shows comparable stability values compared to microcapsules of the state of the art, despite reduced polyisocyanate content (see Figure 5).

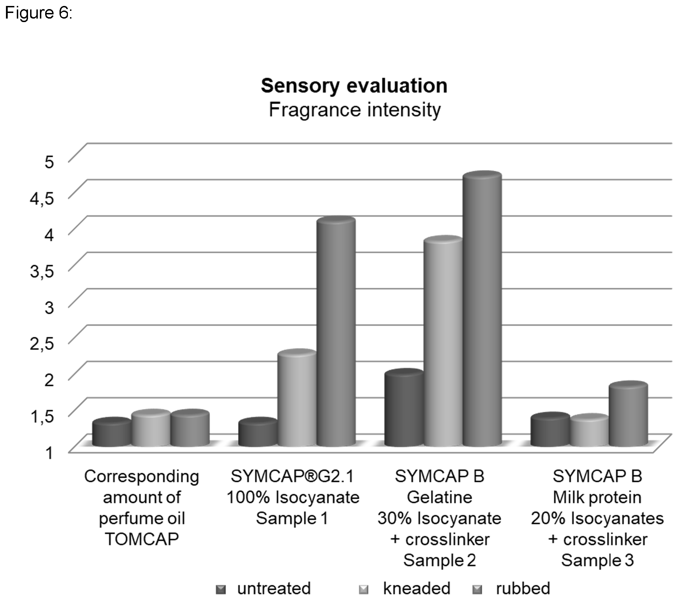

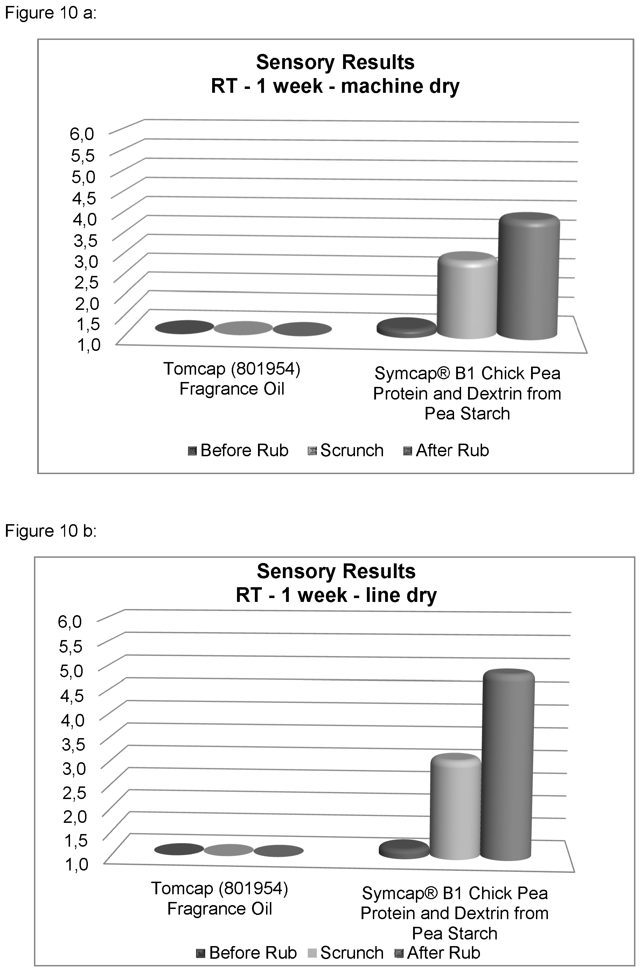

The protein- and/or polysaccharide-based microcapsules according to the invention also show a significant improvement in sensory performance (fragrance release) compared to state-of-the-art capsules, which can be attributed to the stable active ingredient encapsulation and the associated low active ingredient losses. The microcapsules according to the invention show a significantly higher sensory intensity when fragrance is released by opening the capsules by means of mechanical friction or by pressure, as illustrated in Figure 6 and Figures 10 a and 10 b.

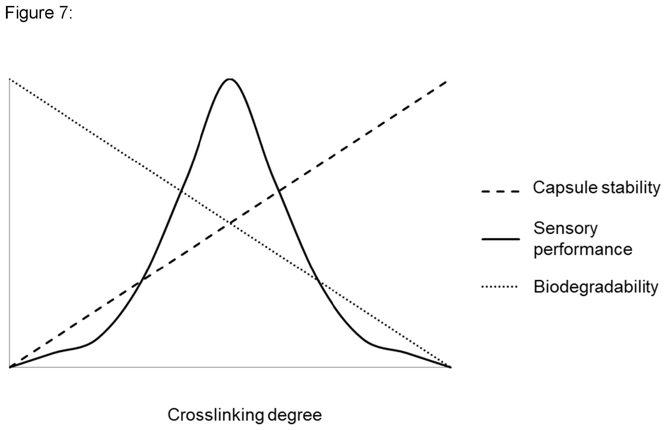

As the degree of crosslinking increases, which is dependent on the crosslinking agent and its concentration, the stability of the microcapsules also increases, but at the same time the ability to biodegrade the capsule shell decreases. Figure 7 shows in general the correlation between microcapsule stability, performance, and biodegradability as a function of the degree of crosslinking. For example, if the microcapsules are very stable, the performance, e.g., sensory performance, is lower because the number of microcapsules that break open through rubbing, pressure, etc. and release the active ingredients is reduced. If the microcapsules are too unstable, they are already destroyed during use or during storage and also do not perform.

The protein- and/or polysaccharide-based microcapsules according to the invention have a reduced content of polyisocyanate by up to 75% compared to polyurea/polyurethane microcapsules of the state of the art, without causing any loss or decrease in stability or in loading with active ingredient of the microcapsules to be encapsulated, as shown in the following embodiments. In contrast to the microcapsules of the state of the art, the isocyanates no longer function as the main material of the capsule shell or capsule wall, but serve exclusively as crosslinkers of the proteins and/or polysaccharides of the capsule shell. The absolute polyisocyanate content of the microcapsules described herein thus corresponds to only 1.5% of the total microcapsule.

Due to the lower polyisocyanate content in the capsule shell or capsule wall, on the one hand, and the use of a protein and/or polysaccharide as capsule wall building block, on the other hand, the microcapsules according to the invention are more biodegradable than state-of-the-art capsules. Microcapsules according to the invention exhibit significantly better biodegradability, as shown in the following embodiment examples.

Biodegradability is the ability of organic material to be degraded to water, carbon dioxide ($CO_2$), and biomass after a prescribed time under defined conditions of temperature, oxygen, and moisture in the presence of microorganisms or fungi.

According to OECD 301F, a microcapsule is considered readily biodegradable if more than 60% of the wall material has degraded after 28 days.

The microcapsules according to the invention have a biodegradability according to OECD 301F after 28 days of $\geq$ 10%, preferably a biodegradability of $\geq$ 50%, even more preferably a biodegradability of $\geq$ 70% and most preferably a biodegradability of $\geq$ 90%.

The combination of starting ingredients enables microcapsules according to the invention with sufficient stability (mechanical as well as diffusion stability in use), high sensory performance and at the same time excellent biodegradability. At the same time, the composition of the starting ingredients allows the degree of crosslinking to be kept low, which greatly increases the biodegradability of the microcapsule. Thus, the previously valid correlation between sensory performance, high crosslinking and biodegradability can be broken.

Due to the biodegradability, the excellent stability and an outstanding release capability of the microcapsules and the possibility of encapsulating a wide range of hydrophobic active ingredients with the microcapsules according to the invention, the protein- and polysaccharide-based microcapsules according to the present invention can be used for a wide range of applications for fragrancing and flavoring.

Moreover, the microcapsule according to the present invention is a universal capsule that can be used to encapsulate a wide range of fragrance substances or aroma substances according to the present state of the art, even fragrance or aroma substances that have aldehyde, carboxylic acid or ester functionality, so that there are no restrictions against individual active ingredients.

Due to their advantageous properties, in particular their stability and targeted release of the active ingredients and their biodegradability, the microcapsules according to the invention are suitable for a wide range of applications and in particular for use in household products, textile care products, laundry detergents, fabric softeners, cleaning agents, scent boosters, scent lotion and scent enhancers, cosmetics, personal care products, agricultural products, pharmaceutical products, or print coatings for paper and the like.

Thus, in another aspect, the present invention relates to the use of the biodegradable protein- and/or polysaccharide-based microcapsules according to the invention or a dispersion of the biodegradable protein- and/or polysaccharide-based microcapsules according to the invention for the preparation of household products, textile care products, laundry detergents, fabric softeners, cleaning agents, scent boosters, scent lotion and fragrance enhancers in liquid or solid form, cosmetics, personal care products, agricultural products, pharmaceutical products or print coatings for paper. The microcapsules according to the invention are particularly suitable for the encapsulation of hydrophobic fragrance substances or aroma substances, which can be used in various household and textile care products.

Finally, the present invention relates to household products, textile care products, laundry detergents, fabric softeners, cleaning agents, scent boosters, scent lotion and fragrance enhancers, cosmetics, personal care products, agricultural products, pharmaceutical products or print coating for paper and the like comprising the biodegradable protein- and/or polysaccharide-based microcapsules according to the invention or a dispersion of the biodegradable protein- and/or polysaccharide-based microcapsules according to the invention.

The proportion of microcapsules in the aforementioned products is from 0.05 to 15 wt.-%, based on the total weight of the product, preferably from 0.2 to 5 wt.-%.

**Examples of embodiments**

The biodegradable protein- and/or polysaccharide-based microcapsules according to the invention and their advantageous properties are described in more detail with reference to the following examples.

**Example 1: Free oil content of microcapsules according to the invention compared with microcapsules SYMCAP® G2.1 and SYMCAP® G3 according to the state of the art**

The following stability data refer to a test at 40 °C in a commercially available formulation, such as scent booster or fabric softener.

In the following examples, microcapsules according to the state of the art were chosen as those whose capsule walls are exclusively based on a polyurea network (Samples 1 and 2). The polyisocyanate used was a mixture consisting of hexamethylene diisocyanate and 4,4'-methyldiphenylene diisocyanate in a ratio of 80 : 20. Polyvinyl alcohol was used as protective colloid, and guanidinium carbonate was used for crosslinking. No catalyst was generally used in the preparation of these capsules and the synthesis was carried out at a pH-value of 9.

Microcapsules according to the invention were prepared with two different proteins (Samples 3 to 10). One was with gelatin, and the other with milk protein to which amino acids glutamine L and lysine L were additionally added. The polysaccharide used in both cases was maltodextrin DE 8-10. As polyisocyanate, a mixture of hexamethylene diisocyanate and 4,4'-methyldiphenylene diisocyanate was used in a ratio of 80 : 20. DABCO was used as catalyst. As further crosslinking agent cinnamaldehyde was used. Sample 11 was prepared using chickpea protein as the protein component and dextrin from pea starch as the polysaccharide component; no additional crosslinking agent was used. The latter sample was prepared using a combination of two different catalysts, DABCO and bismuth neodecanoate. Thereby the bismuth catalyst was added to the internal non-aqueous phase and the DABCO catalyst was added to the mixture after emulsification of the non-aqueous phase and the aqueous phase along with the chickpea protein solution and the dextrin solution. As polyisocyanate component a mixture of Desmodur® N-3400 and Mondur M flakes in a molar ratio of 80 : 20 was used. The chickpea-microcapsules were cured at a temperature of 70 °C for one hour, followed by an increase of the temperature to 80 °C (within approximately half an hour) and curing for another hour at 80 °C.

The content of perfume oil in all samples was 35% of the obtained microcapsule slurry, wherein the perfume oil was mixed with vegetable oil in a ratio of 1 : 1.

The amount of free oil is measured in isopropanol, i.e., a defined amount of the microcapsule slurry is mixed with isopropanol, stirred for 30 seconds and a sample is taken from it. The sample taken is measured by GC-MS. The result indicates how much of the encapsulated oil has passed into the isopropanol, or has not been fully encapsulated, respectively. The free oil content thus provides an indication of whether the process itself has worked, i.e., whether the perfume oil has been fully encapsulated and/or whether the capsule shell is stable enough to prevent the perfume oil from bleeding into isopropanol. In this context, values of less than 1% are considered to be indicative of a successful encapsulation and a stable capsule shell.

The content of free oil of microcapsules according to the invention was compared to the free oil content of microcapsules SYMCAP®G2.1 (100%) and SYMCAP®G3 (75%) according to the state of the art.

Microcapsules according to the state of the art:

Sample 1: SYMCAP® G2.1: isocyanate content: 100%; crosslinked with polyvinyl alcohol and guanidinium carbonate.
Sample 2: SYMCAP® G3: isocyanate content: 75%; crosslinked with polyvinyl alcohol and guanidinium carbonate.

Microcapsules according to the present invention:

Sample 3: SYMCAP B: isocyanate content 50%; gelatin and maltodextrin;
Sample 4: SYMCAP B: isocyanate content 50%; gelatin and maltodextrin; additional crosslinking agent gallic acid;
Sample 5: SYMCAP B: isocyanate content 30%; gelatin and maltodextrin;
Sample 6: SYMCAP B: isocyanate content 30%; gelatin and maltodextrin; additional crosslinking agent cinnamaldehyde;
Sample 7: SYMCAP B: isocyanate content 50%; milk protein + L-glutamine + L-lysine and maltodextrin;
Sample 8: SYMCAP B: isocyanate content 50%; milk protein + L-glutamine + L-lysine and maltodextrin; additional crosslinking agent cinnamaldehyde;
Sample 9: SYMCAP B: isocyanate content 30%; milk protein + L-glutamine + L-lysine and maltodextrin;
Sample 10: SYMCAP B: isocyanate content 30%; milk protein + L-glutamine + L-lysine and maltodextrin; additional crosslinking agent cinnamaldehyde;
Sample 11: SYMCAP B: isocyanate content 75%; chickpea protein + dextrin from pea starch

Since good results were already achieved with an isocyanate content of 50%, an attempt was made to further reduce the isocyanate content and to achieve comparable results to the microcapsules according to the state of the art by means of a second crosslinking.

Table 1:

| Capsule wall components | Free oil in isopropanol (%) |
|---|---|
| **Gelatin** | |
| Sample 1: SYMCAP® G2.1 - 100% isocyanate. (state of the art) | 0.28 |
| Sample 2: SYMCAP® G3 - 75% isocyanate (state of the art) | 0.26 |
| Sample 3: SYMCAP B - 50% isocyanate (according to the invention) | 0.76 |
| Sample 4: SYMCAP B - 50% isocyanate + crosslinker (according to the invention) | 0.07 |
| Sample 5: SYMCAP B - 30% isocyanate (according to the invention) | 2.62 |
| Sample 6: SYMCAP B - 30% isocyanate + crosslinker (according to the invention) | 0.25 |
| **Milk protein and amino acids** | |
| Sample 1: SYMCAP® G2.1 - 100% isocyanate (state of the art) | 0.28 |
| Sample 2: SYMCAP® G3 - 75% isocyanate (state of the art) | 0.26 |
| Sample 7: SYMCAP B - 50% isocyanate (according to the invention) | 0.40 |
| Sample 8: SYMCAP - 50% isocyanate + crosslinker (according to the invention) | 0.17 |
| Sample 9: SYMCAP - 30% isocyanate (according to the invention) | 3.49 |
| Sample 10: SYMCAP - 30% isocyanate + crosslinker (according to the invention) | 0.68 |

(continued)

| Chickpea protein | |
|---|---|
| Sample 11: SYMCAP B - 75% isocyanate (according to the invention) | 2.50 |

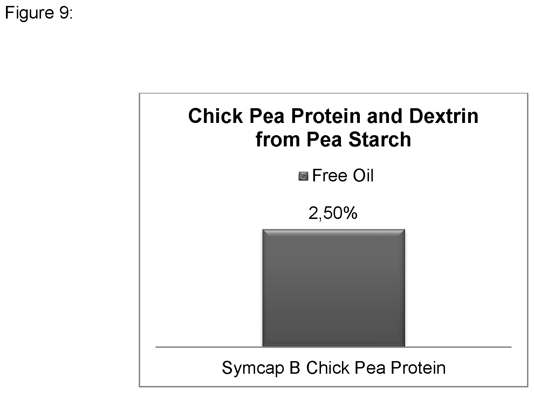

The results are shown in Figures 2 and 3 as well as in Figure 9 for Sample 11.

As can be seen from the above results, the microcapsules according to the invention with a reduced isocyanate content have an almost comparable free oil portion compared to the state-of-the-art microcapsules. By crosslinking with a further crosslinking agent, the stability of the microcapsule wall can be further increased, and the free oil content can be further reduced.

All three microcapsule samples according to the present invention have a free oil content of less than 1%, which is an indicator of a successful encapsulation and a stable capsule shell.

**Example 2: Free oil content of microcapsules according to the invention with and without additional crosslinking agent (transglutaminase (TG))**

Microcapsules were prepared with three different proteins. Firstly, with gelatin, secondly with milk protein and furthermore with milk protein which was additionally mixed with amino acids L-glutamine and L-lysine. The polysaccharide used in both cases was maltodextrin DE 8-10. As catalyst, DABCO was used. The microcapsule samples were prepared without and with another crosslinking agent, respectively; as further crosslinking agent transglutaminase (TG) was used.

The free oil content was determined as described in Example 1.

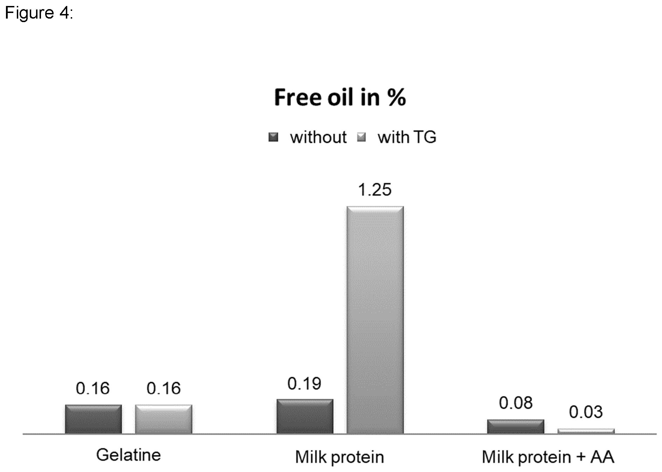

The results are shown in Figure 4.

**Example 3: Stability of microcapsules in application**

The stability of microcapsules according to the invention and microcapsules according to the state of the art, which were prepared as previously described or analogously, was measured in the target application. The stability test was performed using a representative softener (fabric softener) in which the microcapsule slurry was incorporated in an amount of 1 wt.-% and stored at room temperature and at 40 °C, respectively. After defined time intervals (24 h, 1 week, 2 weeks, 4 weeks of aging), samples were taken therefrom, and the stabilities were measured.

Microcapsules according to the state of the art:

    Sample 1: SYMCAP® G2.1: isocyanate content: 100%;
    Sample 2: SYMCAP® G3: isocyanate content: 75%;

Microcapsules according to the present invention:

| | |
|---|---|
| Sample 3: | Symcap B: isocyanate content 50%, pea protein, dextrin. |
| Sample 4: | Symcap B: isocyanate content 50%, gelatin, maltodextrin |
| Sample 5: | Symcap B: isocyanate content 50%, milk protein, amino acids, crosslinker transglutaminase, maltodextrin |
| Sample 6: | Symcap B: isocyanate content 30%, gelatin, maltodextrin |
| Sample 7: | Symcap B: isocyanate content 30%, gelatin, crosslinker tannin, maltodextrin |

Measurement: The capsule contents were analyzed by headspace GC/MS (SPME-fiber: PDMS-DVB 65 $\mu$m sheath).

Analysis:

Integration of the area of each perfume oil compound and calculation of the stability interval

$$\Sigma \text{GC areas of all encapsulated components of the sample} \cdot 100\%$$

Capsule stability = 100 % -

ΣGC areas of all perfume components of the quantification standard

The identification of fragrance constituents is based on an in-house database as well as commercially available analytical databases of fragrance formulas.

This results in a percentage of the perfume oil remaining in the capsule. For example, a result of 98% means that 2% of the original amount of perfume oil used is no longer in the capsule.

The results are shown in Figure 5.

As can be seen from Figure 5, the use of an additional crosslinking agent shows equivalent stability values in the application despite the reduced polyisocyanate content.

**Example 4: Sensory evaluation of the microcapsules according to the invention**

For the sensory evaluation, the microcapsules according to the invention were compared with microcapsules according to the state of the art, i.e., microcapsules prepared as described previously:

Microcapsules according to the state of the art:

Sample 1: SYMCAP® G2.1: Isocyanate content: 100%;

Microcapsules according to the present invention:

Sample 2: SYMCAP B: isocyanate content 30%; gelatin and maltodextrin; additional crosslinking agent tannin;
Sample 3: SYMCAP B: isocyanate content 20%; milk protein and maltodextrin; additional crosslinking agent tannin;
Sample 4: SYMCAP B: isocyanate content 75%; chickpea protein and dextrin from pea starch (corresponding to Sample 11 of Example 1);
Perfume oil: Tomcap

The sensory evaluation was performed as follows: The abovementioned microcapsules were each added to a fabric softener at a slurry concentration of 0.4 wt.-% (perfume Tomcap) and then washed. At a loading of the capsules with 17.5% perfume oil (+ 17.5% plant oil = total loading 35%) and a dosage of 0.4% capsule slurry, this results in a dosage of pure perfume oil of 0.07% perfume oil in the fabric softener as a comparison. 30 g of the fabric softener were used for 2 kg of wash load including the Terry Towels. The washing instructions were as follows: The wash load including the Terry Towels (cotton cloth) was placed in the washing machine and the fabric softener was placed in the fabric softener compartment. The washing program "Express 20; 900 rpm was started. Afterwards, the Terry Towels were allowed to dry overnight at room temperature.

16 test persons assessed the fragrance intensity of the Terry-Towels after washing by pairwise comparison tests against a corresponding level of free perfume oil in the fabric softener on a scale of 1 (no odor) to 9 (very strong odor).

For Sample 4 a corresponding scale was applied ranging from 1 (no odor) to 6 (very strong odor). The corresponding results are shown in Figure 6 (Samples 1 to 3) and Figures 1 a and b (Sample 4), respectively. The microcapsules were aged in the fabric softener formulation for one week or longer and preferably for one week, for two weeks and four weeks, respectively (marked as 1-week sample, 2-week sample etc. in the figures). After washing, the towels were divided into two groups and either air dried by hanging them out on a clothesline (indicated as "line-dried" in the figures) or machine dried (indicated as "machine dried" in the figures) in the dryer. Subsequently, the towels were labeled correspondingly and stored in large plastic bags until testing.

The fragrance release was carried out in three steps. The first step describes the smelling of an untreated cloth. The second step describes the smelling of a lightly kneaded cloth; for this purpose, the cloth was subjected to slight mechanical stress by moving it back and forth between the hands several times, causing the capsules to break. The third step describes the smelling after the cloths were rubbed strongly and thus the capsules broke. After each step, the fragrance intensity was evaluated.

The results of the sensory evaluation are shown in Figure 6 as well as Figures 10 a and 10 b for Sample 4.

The microcapsules according to the invention have factually the same, if not even more intense, odor as the microcapsules according to the state of the art. In particular, the use of a further crosslinking agent leads to a better sensory performance.

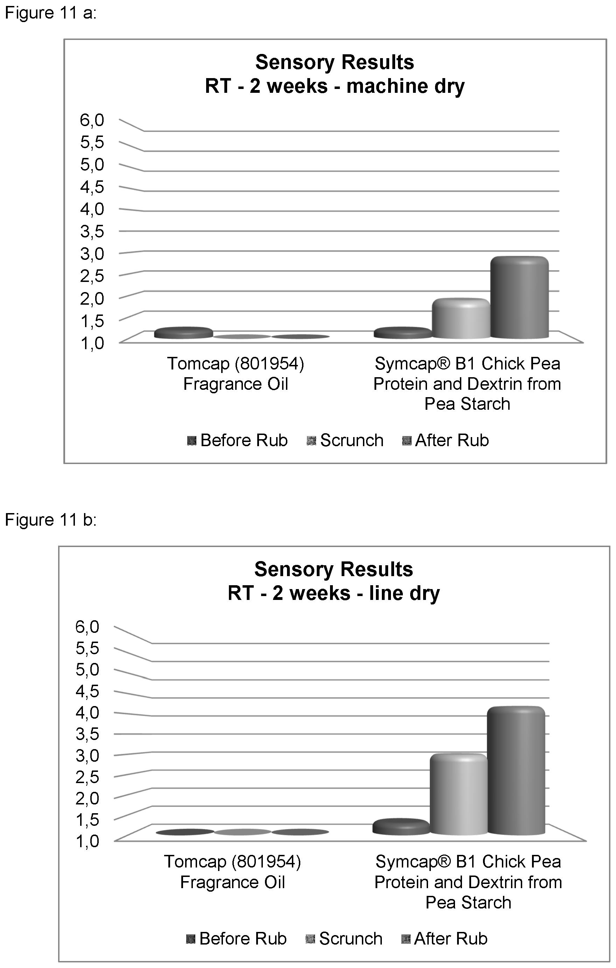

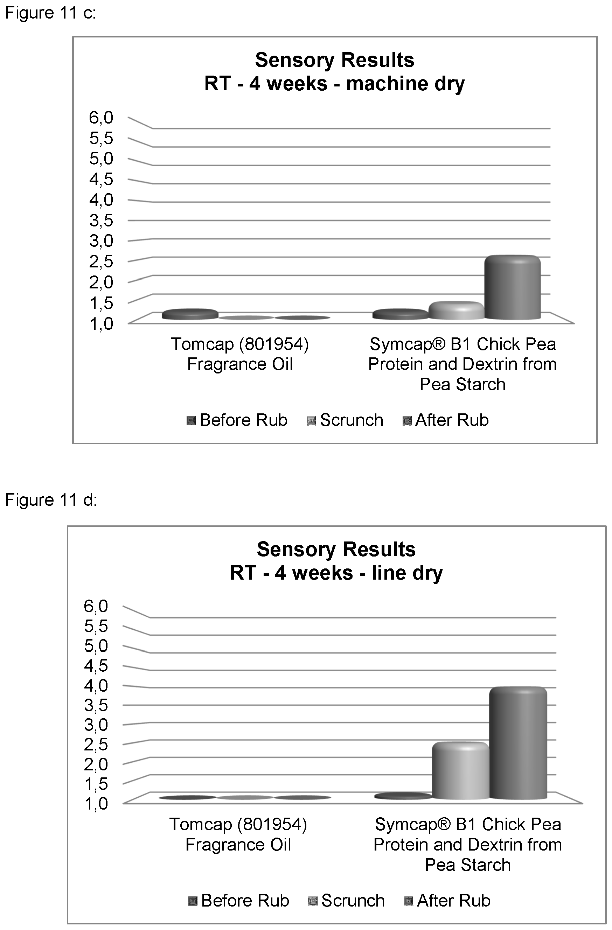

Chickpea protein-based microcapsules (Sample 4) seem to be highly stable and are able to suppress an evaporation of the volatile fragrance components efficiently. The high increase in intensity after scrunching and/or rubbing indicates an efficient encapsulation of the fragrance material. The experiments indicate a high mechanical and thermal stability of the inventive microcapsules (washing machine, drier) while still allowing for a targeted release of the active ingredient. Additionally, the inventive microcapsules show increased chemical stabilities, e.g., when incorporated and aged within consumer product formulations allowing for an efficient encapsulation of the active ingredient and effectively maintaining the long-term product quality. Therefore, the inventive capsules allow for an efficient encapsulation and an excellent and targeted release of the active ingredients. In addition, the inventive microcapsules are less affected by the drying process within the dryer than the fully synthetical state-of-the-art microcapsules. Consequently, the inventive biobased core-shell microcapsules are highly suitable for the incorporation of a variety of consumer product formulations. While the free non-encapsulated fragrance oil quickly evaporated and was not perceivable after the drying process and storage, the biobased microcapsules according to the invention enabled a stable encapsulation of the fragrance material and showed an excellent and targeted release behavior and, as a result, allowed for a highly perceptible fragrance intensity (i.e., high sensory performance). Even after four weeks of aging in the fabric softener an efficient encapsulation of the fragrance material could be observed based on the efficient fragrance release and its high intensities, indicating a high stability of the inventive core-shell microcapsules within the softer and thus within consumer product formulations (see Figures 11 a and b).

The advantage here is due to the stability of the microcapsules according to the invention. These exhibit comparable stability despite isocyanate reduction to 30% based on the capsule shell.

Due to the advantageous properties described above, a consistent quality and consequently also sensory stability can be expected in the long term with the microcapsules according to the invention compared to the microcapsules of the state of the art. The microcapsules according to the state of the art, on the other hand, are less degradable than the microcapsules according to the invention.

**Example 5: Biodegradability of the microcapsules according to the invention**

The biodegradability according to OECD 301F was determined as follows: Degradability of the wall material in a non-preadapted inocolum, measured by manometric respiration (oxygen consumption).

Table 2:

| Microcapsules with different isocyanate content | Biodegradability according to OECD 301F after 28 days [%]. |
|---|---|
| Symcap B: 50% isocyanate content; gelatin and maltodextrin | 2.7 |
| Symcap B: 30% isocyanate content; gelatin and maltodextrin; additional crosslinking agent cinnamaldehyde | 20 |
| Symcap B: 30% isocyanate content; gelatin and maltodextrin; additional crosslinking agent cinnamaldehyde; beeswax | 25 |
| Symcap B: 20% isocyanate content; milk protein and maltodextrin; additional crosslinking agent tannin | 63 |
| Symcap B: 10% isocyanate content; milk protein and maltodextrin; additional crosslinking agent ferulic acid, carboxymethylcellulose (CMC) | 87 |

The biodegradability increases as the amount of isocyanate (crosslinker) decreases.

**Example 6: Sensory evaluation of the microcapsules according to the invention in dependence of the catalytic system and shelf-life**

In another example the efficiency of the encapsulation was analyzed in dependance of the catalytic system used for the preparation of said microcapsules according to the invention. In addition, the shelf-life has been tested.

The analyzed samples were prepared using chickpea protein as the protein component and dextrin from pea starch as the polysaccharide component; no additional crosslinking agent was used. The samples were prepared using a combination of two different catalysts, DABCO and bismuth neodecanoate (dual catalyst system). Thereby the bismuth catalyst was added to the internal non-aqueous phase and the DABCO catalyst was added to the mixture after emulsification of the non-aqueous phase and the aqueous phase. As polyisocyanate component a mixture of Desmodur® N-3400 and Mondur M flakes in a molar ratio of 80 : 20 was used. The chickpea-microcapsules were cured at a temperature of 70 °C for one hour, followed by an increase of the temperature to 80 °C (within approximately half an hour) and curing for another hour at 80 °C. In addition, corresponding samples of the chickpea-dextrin-microcapsules were prepared, wherein only one catalyst was used for the preparation of the microcapsules, i.e., either the DABCO catalyst or the bismuth neodecanoate catalyst.

The sensory evaluation was performed according to Example 4 as specified above and based on a scale ranging from 1 (no odor) to 6 (very strong odor). The corresponding results are shown in Figures 12 a and b. The microcapsules were aged in the fabric softener formulation for one week (Figure 12 a) or for two weeks (Figure 12 b). After washing, the towels were air dried by hanging them out on a clothesline (indicated as "line-dried" in the figures).

A comparison of the above samples shows that microcapsules which were prepared using a dual catalyst system show better intensities compared to microcapsules being prepared by using only one catalyst instead of a dual catalyst system indicating a more efficient encapsulation and/or increased stability said microcapsules (see Figures 12 a and b).

In addition, freshly prepared microcapsules (using the dual catalyst system as specified above) were compared with one year old microcapsules (also using the dual catalyst system). The comparison show that the as-prepared microcapsules show long shelf-life. Even after one year of storage the microcapsules could efficiently be incorporated into the product formulation (fabric softener) and showed excellent stabilities, and encapsulation properties as well as release properties.

**Claims**

1. Process for the preparation of a biodegradable protein- and/or polysaccharide-based microcapsule comprising the following steps in this order:

   (i) Providing an internal non-aqueous phase comprising at least one first crosslinking agent and at least one hydrophobic active ingredient and optionally at least one further crosslinking agent;
   (ii) Providing an external aqueous phase comprising at least one protein and/or at least one polysaccharide and optionally at least one protective colloid, and optionally adjusting the pH-value of the aqueous phase to a pH-value below the isoelectric point of the protein;
   (iii) Emulsifying or dispersing the internal non-aqueous phase in the external aqueous phase, optionally in the presence of at least one stabilizer and/or at least one emulsifier, to obtain an oil-in-water emulsion or dispersion;
   (iv) Optionally adding at least one further polysaccharide and/or at least one further protein;
   (v) First crosslinking by addition of at least one catalyst to obtain a microcapsule slurry;
   (vi) Curing the microcapsule slurry at a temperature of at least 60 °C and optionally adding a further polysaccharide and/or a further protein;
   (vii) Cooling and optionally second crosslinking by adding at least one second crosslinking agent; and
   (viii) Optionally separating the microcapsules from the microcapsule slurry and optionally drying the microcapsules or adjusting the viscosity of the microcapsule slurry by adding at least one thickening agent.

2. The process according to claim 1, wherein the at least one first crosslinking agent is selected from the group consisting of polyisocyanate having two or more isocyanate groups, selected from the group consisting of aliphatic, cycloaliphatic, hydroaromatic, aromatic or heterocyclic polyisocyanates, their substitution products as well as mixtures of the aforementioned compounds, in particular wherein the at least one polyisocyanate comprises two aliphatic polyisocyanates or one aliphatic and one aromatic polyisocyanate, in particular wherein the at least one polyisocyanate comprises polyisocyanates in alternating monomeric, oligomeric or polymeric structure with different chain length; as well as mixtures of two or more of the aforementioned first crosslinking agents.

3. Process according to claim 1 or claim 2, wherein the at least one further or at least one second crosslinking agent is selected from the group consisting of transglutaminase, peroxidase, secondary plant substances selected from the group consisting of polyphenols, in particular tannin, gallic acid, ferulic acid, hesperidin, cinnamaldehyde, vanillin, carvacrol, as well as mixtures of two or more of the aforementioned crosslinking agents.

4. Process according to any one of claims 1 to 3, wherein the at least one hydrophobic active ingredient is selected from the group consisting of fragrance substances, aroma substances, cooling agents, TRPV1 and TRPV3 modulators, substances that cause a sharp taste or a warmth or heat sensation on skin or mucous membranes or a tingling sensation in the mouth or throat or active ingredients with a pungent or acrid or astringent effect, pesticides, biocides, insecticides, a substance from the group of repellents, food additives, cosmetic active ingredients, pharmaceutical active ingredients, agrochemicals, dyes, colorants, dye precursors; luminous paint, optical brighteners, solvents, waxes, silicone oils, lubricants, print coating for paper, as well as mixtures of two or more of the aforementioned active ingredients.

5. Process according to one or more of claims 1 to 4, wherein the at least one polysaccharide or the at least one further polysaccharide is selected from the group consisting of

- indigestible fibers and dietary fibers, in particular insoluble dietary fibers, in particular cellulose, cellulose derivatives such as hydroxyethyl cellulose, in particular quaternized hydroxyethyl cellulose, carboxymethylcellulose (CMC) and microcrystalline cellulose (MCC), hemicelluloses, lichenin, chitin, chitosan, lignin, xanthan, plant fibers, especially cereal fibers, potato fibers, apple fibers, citrus fibers, bamboo fibers, extracted sugar beet fibers; oat fibers and soluble dietary fibers, in particular inulin, especially native inulin, highly soluble inulin, granulated inulin, high performance inulin, pectins, alginates, agar, carrageenan, gum arabic, konjac gum, Curdlan (Paramylon) guar gum, locust bean gum, xanthan gum, raffinose, xylose, polydextrose and lactulose;
- starches, in particular starch from wheat, potatoes, corn, rice, tapioca and oats, chemically, mechanically and/or enzymatically modified starch; and starch derivatives, e.g., dextrins or maltodextrins, in particular dextrins and maltodextrins from wheat, potatoes, corn, rice and oats, in particular maltodextrins DE8-10, DE17-20, DE18-20, cyclodextrins, oligosaccharides, in particular oligofructose; and
- sugar alcohols, in particular sorbitol, mannitol, isomalt, maltitol, maltilol syrup, lactitol, xylitol, erythritol;
- gellan; glucose,

as well as mixtures of the aforementioned polysaccharides.

6. Process according to one or more of claims 1 to 5, wherein the at least one protein or the at least one further protein is selected from the group consisting of proteinogenic L-amino acids, animal or plant proteins, in particular protein isolates, animal or plant proteins in the form of fractions, partial or complete hydrolysates or intermediates prepared by physicochemical processes or fermentative or enzymatic treatment of the proteins, in particular proteins of the group consisting of: meat (mammals, birds, reptiles, amphibians, fish), crabs, crustaceans, mollusks, insects, eggs, milk, in particular casein and whey, rennet casein, whey protein concentrate 80%, gelatin, algae, cereals, especially wheat, barley, rye, spelt, gluten, in particular wheat gluten, rapeseed, sunflower, rice, potato, corn, soybean, bean, pea, chickpea, lentil, lupin, peanut, alfalfa, hemp, other proteins from edible plants, chitosan, as well as mixtures thereof.

7. Process according to one or more of claims 1 to 6, wherein the at least one protective colloid is selected from the group consisting of

- diols, in particular ethanediol, 1,2-propanediol, 1,3-propanediol, 1,2-butanediol, isomeric butanediol, 1,2-pentanediol, 1,2-hexanediol, 1,2-octanediol, 1,2-decanediol, 1,2-dodecanediol, and
- polyols, preferably triols, in particular glycerin as well as its ethoxylation and propoxylation products, trimethylolpropane as well as its ethoxylation and propoxylation products, polyvinyl alcohol (PVOH) and its derivatives, in particular ammonium- or sulfonate-functionalized polyvinyl alcohols, polyphenols, preferably 1,3,5-trihydroxybenzene, polysaccharides, in particular glucose, starches or chemically, mechanically and/or enzymatically modified starches, cellulose derivatives such as hydroxyethyl cellulose, in particular quaternized hydroxyethyl cellulose, and carboxymethylcellulose,
- polyvinylpyrrolidone, maleic acid vinyl copolymers, sodium lignosulfonates, maleic acid anhydride/styrene copolymers, ethylene/maleic acid anhydride copolymers, copolymers of ethylene oxide, propylene oxide and acid esters of polyethoxylated sorbitol, sodium dodecyl sulfate,
- animal and plant polymers, especially gum arabic (Senegal type and Seyal type), proteins, gelatin, olibanum resin, shellac, lignin, chitosan, saponin,

as well as mixtures of the aforementioned compounds; and/or the protective colloid is used in combination with starch.

8. Process according to one or more of claims 1 to 7, wherein the at least one catalyst is selected from the group consisting of diazobicyclo[2.2.2]octane (DABCO), bismuth-catalyst and tin-catalyst, as well as mixtures of two or more of the aforementioned catalysts.

9. Process according to one or more of claims 1 to 8, wherein the first crosslinking is carried out at a temperature of 60 °C to 90 °C.

10. Process according to one or more of claims 1 to 9, wherein curing of the microcapsules is carried out at a temperature of 60 °C to 90 °C and/or for a duration of at least 3 h.

11. Biodegradable microcapsule or microcapsule slurry obtainable by the process according to one or more of claims 1 to 10.

12. Biodegradable microcapsule comprising or consisting of

(a) a core comprising or consisting of at least one hydrophobic active ingredient;

(b) a capsule shell comprising or consisting of a crosslinking matrix or crosslinking units of at least one polysaccharide and/or at least one protein and at least one first crosslinking agent; and optionally at least one first protective colloid and/or optionally at least one further crosslinking agent.

13. Biodegradable microcapsule according to claim 12, wherein the capsule shell comprises or consists of: a crosslinking matrix or crosslinking units from a polymerization and/or crosslinking of at least one protein with the first and optionally the further crosslinking agent; and/or a crosslinking matrix or crosslinking units from a polymerization and/or crosslinking of at least one polysaccharide with the first and optionally the further crosslinking agent.

14. Microcapsule slurry comprising a microcapsule according to any one of claims 11 to 13, optionally in combination with a thickener and/or a preservative.

15. Use of the microcapsules according to one or more of claims 11 to 13 or a microcapsule slurry according to claim 11 or claim 14, for the preparation of household products, textile care products, detergents, fabric softeners, cleaning agents, scent boosters or fragrance enhancers in liquid or solid form, cosmetics, personal care products, perfume compositions, agricultural products, pharmaceutical products or print coating for paper.

16. Household products, fabric care products, detergents, fabric softeners, cleaning agents, scent boosters and fragrance enhancers, cosmetics, personal care products, perfume compositions, agricultural products, or pharmaceutical products, comprising a microcapsule according to any one of claims 11 to 13 or a microcapsule slurry according to claim 11 or claim 14.

Figure 1 a:

Figure 1 b:

Figure 1 c:

Figure 1 d:

Figure 1 e:

Figure 2:

Figure 3:

**Milk protein and amino acids**

■ Free oil in isopropanol

Figure 4:

**Free oil in %**

■ without ■ with TG

Figure 5:

Stability in fabric softener at 22 °C
(room temperature)

■ 24h  ■ 1 week

Figure 6:

**Sensory evaluation**
Fragrance intensity

Figure 7:

Crosslinking degree

Figure 8:

Figure 9:

**Chick Pea Protein and Dextrin from Pea Starch**

Free Oil

2,50%

Symcap B Chick Pea Protein

Figure 10 a:

**Sensory Results**
**RT - 1 week - machine dry**

Tomcap (801954)
Fragrance Oil

Symcap® B1 Chick Pea
Protein and Dextrin from
Pea Starch

■ Before Rub   ▤ Scrunch   ▨ After Rub

Figure 10 b:

**Sensory Results**
**RT - 1 week - line dry**

Tomcap (801954)
Fragrance Oil

Symcap® B1 Chick Pea
Protein and Dextrin from
Pea Starch

■ Before Rub   ▤ Scrunch   ▨ After Rub

Figure 11 a:

**Sensory Results**
**RT - 2 weeks - machine dry**

■ Before Rub   ▨ Scrunch   ▥ After Rub

Figure 11 b:

**Sensory Results**
**RT - 2 weeks - line dry**

■ Before Rub   ▨ Scrunch   ▥ After Rub

Figure 11 c:

**Sensory Results
RT - 4 weeks - machine dry**

Figure 11 d:

**Sensory Results
RT - 4 weeks - line dry**

Figure 12 a:

**Sensory Results**
**RT - 1 Week - Line Dry**

■ Before Rub   ▨ Scrunch   ▨ After Rub

Figure 12 b:

**Sensory Results**
**RT - 2 Weeks - Line Dry**

**EP 4 772 603 A2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011161229 A
- WO 2011160733 A
- WO 2012107323 A
- EP 0537467 A
- WO 2007096592 A
- US 4855490 A
- US 4144268 A
- WO 2004026840 A
- US 2017216802 A1
- US 2010273887 A1
- EP 2033688 A2
- EP 1958627 A2
- WO 2004043906 A
- WO 2004000787 A
- US 2800457 A

**Non-patent literature cited in the description**

- **K. C. FRISCH** ; **L.P. RUMAO**. Catalysis in Isocyanate Reactions. *Polymer Reviews*, 1970, vol. 5 (1), 103-149
- **K.C. FRISCH** ; **L.P. RUMAO**. Catalysis in Isocyanate Reactions. *Polymer Reviews*, 1970, vol. 5 (1), 103-149
- **M.F. SONNENSCHEIN**. Introduction to Polyurethane Chemistry, Polyurethanes: Science, Technology, Markets, and Trends. John Wiley & Sons, 2015, 105-126